# EUROPEAN PATENT APPLICATION

(11) **EP 1 704 868 A1**
(43) Date of publication of application: **27.09.2006**
(21) Application number: 06010437.9
(22) Date of filing: 06.08.1993
(51) Int. Cl.: A61K 39/00, C07K 7/06, C07K 14/705

(54) **HLA binding peptides and their uses**

(30) Priority: 07.08.1992 US 926666; 05.03.1993 US 27746
(62) Divisional of application: 93919916.2
(71) Applicant: Pharmexa Inc., San Diego, CA 92121 (US)
(72) Inventor: Kubo, Ralph T., San Diego, CA 92130 (US); Grey, Howard M., La Jolla, CA 92037 (US); Sette, Alessandro, La Jolla, CA 92037 (US); Celis, Esteban, San Diego, CA 92130 (US)
(74) Representative: Cripps, Joanna Elizabeth

(57) **Abstract**

The present invention provides peptide compositions capable of specifically binding selected MHC alleles and inducting T cell activation in T cells restricted by the MHC allele. The peptides are useful to elicit an immune response against a desired antigen.

SEQUENCE LISTING

<110>  Pharmexa Inc.

<120>  HLA Binding Peptides and Their Uses

<130>  JEC/FP6374920

<150>  EP 93919916.2
<151>  1993-08-06

<150>  PCT/US93/07421
<151>  1993-08-06

<150>  US 07/926,666
<151>  1992-08-07

<150>  US 08/027,746
<151>  1993-03-05

<160>  662

<170>  PatentIn version 3.3

<210>  1
<211>  10
<212>  PRT
<213>  Artificial sequence

<220>
<221> source
<223> /note = "Description of artificial sequence:   Synthetic sequence:
              Radiolabeled probe"

<400>  1

Lys Val Phe Pro Tyr Ala Leu Ile Asn Lys
1               5                   10

<210>  2
<211>  9
<212>  PRT
<213>  Artificial sequence

<220>
<221> source
<223> /note = "Description of artificial sequence:   Synthetic sequence:
               Radiolabeled probe"

<400>  2

Ala Val Asp Leu Tyr His Phe Leu Lys
1               5


<210>  3
<211>  9
<212>  PRT
<213>  Artificial sequence

<220>
<221> source
<223> /note = "Description of artificial sequence:   Synthetic sequence:
               Radiolabeled probe"

<400>  3

Tyr Leu Glu Pro Ala Ile Ala Lys Tyr
1               5


<210>  4
<211>  9
<212>  PRT
<213>  Artificial sequence

<220>
<221> source
<223> /note = "Description of artificial sequence:   Synthetic sequence:
               Consensus peptide whose sequence was based on the pool
               sequencing data of PCT/US93/07421"

<400>  4

Ala Tyr Ile Asp Asn Val Tyr Lys Phe
1               5

<210>  5
<211>  9
<212>  PRT
<213>  Artificial sequence

<220>
<221> source
<223> /note = "Description of artificial sequence:   Synthetic sequence:
              Consensus peptide whose sequence was based on the pool
              sequencing data of PCT/US93/07421"

<400>  5

Ala Tyr Ile Asp Asn Tyr Asn Lys Phe
1               5

<210>  6
<211>  9
<212>  PRT
<213>  Homo sapiens

<220>
<221>  MISC_FEATURE
<222>  1, 3..8
<223>  "Xaa" represents a wildcard character (any amino acid)

<400>  6

Xaa Ser Xaa Xaa Xaa Xaa Xaa Xaa Tyr
1               5

<210>  7
<211>  10
<212>  PRT
<213>  Homo sapiens

<220>
<221>  MISC_FEATURE
<222>  1, 3..9
<223>  "Xaa" represents a wildcard character (any amino acid)

<400>  7

Xaa Ser Xaa Xaa Xaa Xaa Xaa Xaa Xaa Tyr
1               5                   10

<210>  8
<211>  9
<212>  PRT
<213>  Homo sapiens

<220>
<221>  MISC_FEATURE
<222>  1, 3..8
<223>  "Xaa" represents a wildcard character (any amino acid)

<400>  8

Xaa Thr Xaa Xaa Xaa Xaa Xaa Xaa Tyr
1               5

<210>  9
<211>  10
<212>  PRT
<213>  Homo sapiens

<220>
<221>  MISC_FEATURE
<222>  1, 3..9
<223>  "Xaa" represents a wildcard character (any amino acid)

<400>  9

Xaa Thr Xaa Xaa Xaa Xaa Xaa Xaa Xaa Tyr
1               5                   10

<210>  10
<211>  9
<212>  PRT
<213>  Homo sapiens

<220>
<221> MISC_FEATURE
<222> 1, 2, 4..8
<223> "Xaa" represents a wildcard character (any amino acid)

<400> 10

Xaa Xaa Asp Xaa Xaa Xaa Xaa Xaa Tyr
1               5


<210> 11
<211> 10
<212> PRT
<213> Homo sapiens


<220>
<221> MISC_FEATURE
<222> 1, 2, 4..9
<223> "Xaa" represents a wildcard character (any amino acid)

<400> 11

Xaa Xaa Asp Xaa Xaa Xaa Xaa Xaa Xaa Tyr
1               5                   10


<210> 12
<211> 9
<212> PRT
<213> Homo sapiens


<220>
<221> MISC_FEATURE
<222> 1, 2, 4..8
<223> "Xaa" represents a wildcard character (any amino acid)

<400> 12

Xaa Xaa Glu Xaa Xaa Xaa Xaa Xaa Tyr
1               5

<210>  13
<211>  10
<212>  PRT
<213>  Homo sapiens

<220>
<221>  MISC_FEATURE
<222>  1, 2, 4..9
<223>  "Xaa" represents a wildcard character (any amino acid)

<400>  13

Xaa Xaa Glu Xaa Xaa Xaa Xaa Xaa Xaa Tyr
1               5                   10

<210>  14
<211>  9
<212>  PRT
<213>  Homo sapiens

<220>
<221>  MISC_FEATURE
<222>  1, 3..8
<223>  "Xaa" represents a wildcard character (any amino acid)

<400>  14

Xaa Val Xaa Xaa Xaa Xaa Xaa Xaa Lys
1               5

<210>  15
<211>  10
<212>  PRT
<213>  Homo sapiens

<220>
<221>  MISC_FEATURE
<222>  1, 3..9
<223>  "Xaa" represents a wildcard character (any amino acid)

<400>  15

Xaa Val Xaa Xaa Xaa Xaa Xaa Xaa Xaa Lys
1               5                   10


<210>  16
<211>  9
<212>  PRT
<213>  Homo sapiens


<220>
<221>  MISC_FEATURE
<222>  1, 3..8
<223>  "Xaa" represents a wildcard character (any amino acid)

<400>  16

Xaa Leu Xaa Xaa Xaa Xaa Xaa Xaa Lys
1               5


<210>  17
<211>  10
<212>  PRT
<213>  Homo sapiens


<220>
<221>  MISC_FEATURE
<222>  1, 3..9
<223>  "Xaa" represents a wildcard character (any amino acid)

<400>  17

Xaa Leu Xaa Xaa Xaa Xaa Xaa Xaa Xaa Lys
1               5                   10


<210>  18
<211>  9
<212>  PRT
<213>  Homo sapiens

<220>
<221> MISC_FEATURE
<222> 1, 3..8
<223> "Xaa" represents a wildcard character (any amino acid)

<400> 18

Xaa Met Xaa Xaa Xaa Xaa Xaa Xaa Lys
1               5

<210> 19
<211> 10
<212> PRT
<213> Homo sapiens

<220>
<221> MISC_FEATURE
<222> 1, 3..9
<223> "Xaa" represents a wildcard character (any amino acid)

<400> 19

Xaa Met Xaa Xaa Xaa Xaa Xaa Xaa Xaa Lys
1               5                   10

<210> 20
<211> 9
<212> PRT
<213> Homo sapiens

<220>
<221> MISC_FEATURE
<222> 1, 3..8
<223> "Xaa" represents a wildcard character (any amino acid)

<400> 20

Xaa Thr Xaa Xaa Xaa Xaa Xaa Xaa Lys
1               5

<210>  21
<211>  10
<212>  PRT
<213>  Homo sapiens

<220>
<221>  MISC_FEATURE
<222>  1, 3..9
<223>  "Xaa" represents a wildcard character (any amino acid)

<400>  21

Xaa Thr Xaa Xaa Xaa Xaa Xaa Xaa Xaa Lys
1               5                   10

<210>  22
<211>  9
<212>  PRT
<213>  Homo sapiens

<220>
<221>  MISC_FEATURE
<222>  1, 3..8
<223>  "Xaa" represents a wildcard character (any amino acid)

<400>  22

Xaa Val Xaa Xaa Xaa Xaa Xaa Xaa Lys
1               5

<210>  23
<211>  10
<212>  PRT
<213>  Homo sapiens

<220>
<221>  MISC_FEATURE
<222>  1, 3..9
<223>  "Xaa" represents a wildcard character (any amino acid)

<400>  23

Xaa Val Xaa Xaa Xaa Xaa Xaa Xaa Xaa Lys
1               5                   10

<210>  24
<211>  9
<212>  PRT
<213>  Homo sapiens

<220>
<221>  MISC_FEATURE
<222>  1, 3..8
<223>  "Xaa" represents a wildcard character (any amino acid)

<400>  24

Xaa Tyr Xaa Xaa Xaa Xaa Xaa Xaa Phe
1               5

<210>  25
<211>  10
<212>  PRT
<213>  Homo sapiens

<220>
<221>  MISC_FEATURE
<222>  1, 3..9
<223>  "Xaa" represents a wildcard character (any amino acid)

<400>  25

Xaa Tyr Xaa Xaa Xaa Xaa Xaa Xaa Xaa Phe
1               5                   10

<210>  26
<211>  9
<212>  PRT
<213>  Homo sapiens

<220>
<221>  MISC_FEATURE
<222>  1, 3..8
<223>  "Xaa" represents a wildcard character (any amino acid)

<400>  26

Xaa Tyr Xaa Xaa Xaa Xaa Xaa Xaa Leu
1               5

<210>  27
<211>  10
<212>  PRT
<213>  Homo sapiens

<220>
<221>  MISC_FEATURE
<222>  1, 3..9
<223>  "Xaa" represents a wildcard character (any amino acid)

<400>  27

Xaa Tyr Xaa Xaa Xaa Xaa Xaa Xaa Xaa Leu
1               5                   10

<210>  28
<211>  10
<212>  PRT
<213>  Human papillomavirus

<400>  28

Ile His Asp Ile Ile Leu Glu Cys Val Tyr
1               5                   10

<210>  29
<211>  9
<212>  PRT
<213>  Human papillomavirus

<400> 29

Val Cys Asp Lys Cys Leu Lys Phe Tyr
1               5

<210> 30
<211> 10
<212> PRT
<213> Human papillomavirus

<400> 30

Tyr Ser Lys Ile Ser Glu Tyr Arg His Tyr
1               5                   10

<210> 31
<211> 9
<212> PRT
<213> Human papillomavirus

<400> 31

Ile Ser Glu Tyr Arg His Tyr Cys Tyr
1               5

<210> 32
<211> 10
<212> PRT
<213> Human papillomavirus

<400> 32

Gly Thr Thr Leu Glu Gln Gln Tyr Asn Lys
1               5                   10

<210> 33
<211> 9
<212> PRT
<213> Human papillomavirus

<400>  33

Thr Thr Leu Glu Gln Gln Tyr Asn Lys
1               5

<210>  34
<211>  10
<212>  PRT
<213>  Human papillomavirus

<400>  34

Leu Leu Ile Arg Cys Ile Asn Cys Gln Lys
1               5                   10

<210>  35
<211>  10
<212>  PRT
<213>  Human papillomavirus

<400>  35

His Gly Asp Thr Pro Thr Leu His Glu Tyr
1               5                   10

<210>  36
<211>  10
<212>  PRT
<213>  Human papillomavirus

<400>  36

Gln Pro Glu Thr Thr Asp Leu Tyr Cys Tyr
1               5                   10

<210>  37
<211>  9
<212>  PRT
<213>  Human papillomavirus

<400>  37

Gln Ala Glu Pro Asp Arg Ala His Tyr
1               5

<210>  38
<211>  9
<212>  PRT
<213>  Human papillomavirus

<400>  38

Ile Val Cys Pro Ile Cys Ser Gln Lys
1               5

<210>  39
<211>  10
<212>  PRT
<213>  Human papillomavirus

<400>  39

Arg Phe Glu Asp Pro Thr Arg Arg Pro Tyr
1               5                   10

<210>  40
<211>  9
<212>  PRT
<213>  Human papillomavirus

<400>  40

Phe Glu Asp Pro Thr Arg Arg Pro Tyr
1               5

<210> 41
<211> 10
<212> PRT
<213> Human papillomavirus

<400> 41

Leu Gln Asp Ile Glu Ile Thr Cys Val Tyr
1               5                   10

<210> 42
<211> 10
<212> PRT
<213> Human papillomavirus

<400> 42

Leu Thr Glu Val Phe Glu Phe Ala Phe Lys
1               5                   10

<210> 43
<211> 10
<212> PRT
<213> Human papillomavirus

<400> 43

Tyr Ser Arg Ile Arg Glu Leu Arg His Tyr
1               5                   10

<210> 44
<211> 9
<212> PRT
<213> Human papillomavirus

<400> 44

Ser Val Tyr Gly Asp Thr Leu Glu Lys
1               5

<210> 45
<211> 10
<212> PRT
<213> Human papillomavirus

<400> 45

Leu Leu Ile Arg Cys Leu Arg Cys Gln Lys
1               5                   10

<210> 46
<211> 9
<212> PRT
<213> Human papillomavirus

<400> 46

His Thr Met Leu Cys Met Cys Cys Lys
1               5

<210> 47
<211> 10
<212> PRT
<213> Homo sapiens

<400> 47

Ser Leu Glu Gln Arg Ser Leu His Cys Lys
1               5                   10

<210> 48
<211> 9
<212> PRT
<213> Homo sapiens

<400> 48

Ser Leu Phe Arg Ala Val Ile Thr Lys
1               5

<210>  49
<211>  10
<212>  PRT
<213>  Homo sapiens

<400>  49

Ser Leu Phe Arg Ala Val Ile Thr Lys Lys
1               5                   10


<210>  50
<211>  9
<212>  PRT
<213>  Homo sapiens

<400>  50

Asp Leu Val Gly Phe Leu Leu Leu Lys
1               5


<210>  51
<211>  10
<212>  PRT
<213>  Homo sapiens

<400>  51

Met Leu Glu Ser Val Ile Lys Asn Tyr Lys
1               5                   10


<210>  52
<211>  9
<212>  PRT
<213>  Homo sapiens

<400>  52

Met Leu Glu Ser Val Ile Lys Asn Tyr
1               5

<210>  53
<211>  9
<212>  PRT
<213>  Homo sapiens

<400>  53

Gln Leu Val Phe Gly Ile Asp Val Lys
1               5

<210>  54
<211>  9
<212>  PRT
<213>  Homo sapiens

<400>  54

Glu Ala Asp Pro Thr Gly His Ser Tyr
1               5

<210>  55
<211>  10
<212>  PRT
<213>  Homo sapiens

<400>  55

Leu Leu Gly Asp Asn Gln Ile Met Pro Lys
1               5                   10

<210>  56
<211>  10
<212>  PRT
<213>  Homo sapiens

<400>  56

Trp Glu Glu Leu Ser Val Met Glu Val Tyr
1               5                   10

<210>  57
<211>  10
<212>  PRT
<213>  Homo sapiens

<400>  57

Val Tyr Asp Gly Arg Glu His Ser Ala Tyr
1               5                   10



<210>  58
<211>  10
<212>  PRT
<213>  Homo sapiens

<400>  58

Leu Leu Thr Gln Asp Leu Val Gln Glu Lys
1               5                   10



<210>  59
<211>  9
<212>  PRT
<213>  Homo sapiens

<400>  59

Leu Thr Gln Asp Leu Val Gln Glu Lys
1               5



<210>  60
<211>  10
<212>  PRT
<213>  Homo sapiens

<400>  60

Leu Thr Gln Asp Leu Val Gln Glu Lys Tyr
1               5                   10

<210>  61
<211>  9
<212>  PRT
<213>  Homo sapiens

<400>  61

Thr Gln Asp Leu Val Gln Glu Lys Tyr
1               5

<210>  62
<211>  9
<212>  PRT
<213>  Homo sapiens

<400>  62

Ile Val Gly Gly Trp Glu Cys Glu Lys
1               5

<210>  63
<211>  10
<212>  PRT
<213>  Homo sapiens

<400>  63

Leu Thr Ala Ala His Cys Ile Arg Asn Lys
1               5                   10

<210>  64
<211>  10
<212>  PRT
<213>  Homo sapiens

<400>  64

Val Ser His Ser Phe Pro His Pro Leu Tyr
1               5                   10

<210>  65
<211>  9
<212>  PRT
<213>  Homo sapiens

<400>  65

Pro Leu Tyr Asp Met Ser Leu Leu Lys
1               5

<210>  66
<211>  10
<212>  PRT
<213>  Homo sapiens

<400>  66

Asp Val Cys Ala Gln Val His Pro Gln Lys
1               5                   10

<210>  67
<211>  9
<212>  PRT
<213>  Homo sapiens

<400>  67

Gln Val His Pro Gln Lys Val Thr Lys
1               5

<210>  68
<211>  10
<212>  PRT
<213>  Homo sapiens

<400>  68

Pro Ser Leu Tyr Thr Lys Val Val His Tyr
1               5                   10

<210>  69
<211>  9
<212>  PRT
<213>  Homo sapiens

<400>  69

Tyr Thr Lys Val Val His Tyr Arg Lys
1               5

<210>  70
<211>  10
<212>  PRT
<213>  Homo sapiens

<400>  70

Lys Val Val His Tyr Arg Lys Trp Ile Lys
1               5                   10

<210>  71
<211>  9
<212>  PRT
<213>  Homo sapiens

<400>  71

Val Val His Tyr Arg Lys Trp Ile Lys
1               5

<210>  72
<211>  9
<212>  PRT
<213>  Hepatitis C virus

<400>  72

Ser Thr Asn Pro Lys Pro Gln Arg Lys
1               5

<210>  73
<211>  10
<212>  PRT
<213>  Hepatitis C virus

<400>  73

Asn Thr Asn Arg Arg Pro Gln Asp Val Lys
1               5                   10

<210>  74
<211>  9
<212>  PRT
<213>  Hepatitis C virus

<400>  74

Arg Leu Gly Val Arg Ala Thr Arg Lys
1               5

<210>  75
<211>  9
<212>  PRT
<213>  Hepatitis C virus

<400>  75

Val Gln Asp Cys Asn Cys Ser Ile Tyr
1               5

<210>  76
<211>  9
<212>  PRT
<213>  Hepatitis C virus

<400>  76

Trp Met Asn Ser Thr Gly Phe Thr Lys
1               5

<210>  77
<211>  9
<212>  PRT
<213>  Hepatitis C virus

<400>  77

Leu Thr Pro Arg Cys Met Val Asp Tyr
1               5

<210>  78
<211>  9
<212>  PRT
<213>  Hepatitis C virus

<400>  78

Phe Thr Ile Phe Lys Ile Arg Met Tyr
1               5

<210>  79
<211>  9
<212>  PRT
<213>  Artificial sequence

<220>
<221> source
<223> /note = "Description of artificial sequence:   Synthetic sequence"

<400>  79

Ala Ala Asp Lys Ala Ala Ala Ala Tyr
1               5

<210>  80
<211>  9
<212>  PRT
<213>  Artificial sequence

<220>
<221> source
<223> /note = "Description of artificial sequence:   Synthetic sequence"

<400>  80

Ala Thr Ala Lys Ala Ala Ala Ala Tyr
1               5


<210>  81
<211>  9
<212>  PRT
<213>  Artificial sequence

<220>
<221> source
<223> /note = "Description of artificial sequence:   Synthetic sequence"

<400>  81

Ala Thr Asp Lys Ala Ala Ala Ala Tyr
1               5


<210>  82
<211>  9
<212>  PRT
<213>  Artificial sequence

<220>
<221> source
<223> /note = "Description of artificial sequence:   Synthetic sequence"

<400>  82

Ala Leu Ala Lys Ala Ala Ala Ala Val
1               5


<210>  83
<211>  9
<212>  PRT
<213>  Artificial sequence

<220>
<221> source
<223> /note = "Description of artificial sequence:   Synthetic sequence"

<400>  83

Ala Met Ala Ala Ala Ala Ala Ala Lys
1               5



<210>  84
<211>  9
<212>  PRT
<213>  Artificial sequence

<220>
<221> source
<223> /note = "Description of artificial sequence:   Synthetic sequence"

<400>  84

Ala Thr Ala Ala Ala Ala Ala Ala Lys
1               5



<210>  85
<211>  9
<212>  PRT
<213>  Artificial sequence

<220>
<221> source
<223> /note = "Description of artificial sequence:   Synthetic sequence"

<400>  85

Ala Tyr Ala Lys Ala Ala Ala Ala Phe
1               5



<210>  86
<211>  10
<212>  PRT
<213>  Artificial sequence

<220>
<221> source
<223> /note = "Description of artificial sequence:   Synthetic sequence"

<400>  86

Ala Ala Asp Lys Ala Ala Ala Ala Ala Tyr
1               5                   10



<210>  87
<211>  10
<212>  PRT
<213>  Artificial sequence

<220>
<221> source
<223> /note = "Description of artificial sequence:   Synthetic sequence"

<400>  87

Ala Thr Ala Lys Ala Ala Ala Ala Ala Tyr
1               5                   10



<210>  88
<211>  10
<212>  PRT
<213>  Artificial sequence

<220>
<221> source
<223> /note = "Description of artificial sequence:   Synthetic sequence"

<400>  88

Ala Thr Asp Lys Ala Ala Ala Ala Ala Tyr
1               5                   10

<210>  89
<211>  10
<212>  PRT
<213>  Artificial sequence

<220>
<221> source
<223> /note = "Description of artificial sequence:   Synthetic sequence"

<400>  89

Ala Leu Ala Lys Ala Ala Ala Ala Ala Val
1               5                   10



<210>  90
<211>  10
<212>  PRT
<213>  Artificial sequence

<220>
<221> source
<223> /note = "Description of artificial sequence:   Synthetic sequence"

<400>  90

Ala Met Ala Ala Ala Ala Ala Ala Ala Lys
1               5                   10



<210>  91
<211>  10
<212>  PRT
<213>  Artificial sequence

<220>
<221> source
<223> /note = "Description of artificial sequence:   Synthetic sequence"

<400>  91

Ala Thr Ala Ala Ala Ala Ala Ala Ala Lys
1               5                   10

<210>  92
<211>  9
<212>  PRT
<213>  Artificial sequence

<220>
<221> source
<223> /note = "Description of artificial sequence:   Synthetic sequence"

<400>  92

Ala Leu Ala Ala Ala Ala Ala Ala Lys
1               5



<210>  93
<211>  9
<212>  PRT
<213>  Artificial sequence

<220>
<221> source
<223> /note = "Description of artificial sequence:   Synthetic sequence"

<400>  93

Ala Val Ala Ala Ala Ala Ala Ala Lys
1               5



<210>  94
<211>  9
<212>  PRT
<213>  Artificial sequence

<220>
<221> source
<223> /note = "Description of artificial sequence:   Synthetic sequence"

<400>  94

Ala Ser Ala Ala Ala Ala Ala Ala Lys
1               5

<210>  95
<211>  9
<212>  PRT
<213>  Artificial sequence

<220>
<221> source
<223> /note = "Description of artificial sequence:   Synthetic sequence"

<400>  95

Ala Ile Ala Ala Ala Ala Ala Ala Lys
1               5



<210>  96
<211>  9
<212>  PRT
<213>  Artificial sequence

<220>
<221> source
<223> /note = "Description of artificial sequence:   Synthetic sequence"

<400>  96

Ala Ala Ala Ala Ala Ala Ala Ala Lys
1               5



<210>  97
<211>  9
<212>  PRT
<213>  Artificial sequence

<220>
<221> source
<223> /note = "Description of artificial sequence:   Synthetic sequence"

<400>  97

Ala Phe Ala Ala Ala Ala Ala Ala Lys
1               5

<210>  98
<211>  9
<212>  PRT
<213>  Artificial sequence

<220>
<221> source
<223> /note = "Description of artificial sequence:   Synthetic sequence"

<400>  98

Ala Gly Ala Ala Ala Ala Ala Ala Lys
1               5



<210>  99
<211>  9
<212>  PRT
<213>  Artificial sequence

<220>
<221> source
<223> /note = "Description of artificial sequence:   Synthetic sequence"

<400>  99

Ala Cys Ala Ala Ala Ala Ala Ala Lys
1               5



<210>  100
<211>  9
<212>  PRT
<213>  Artificial sequence

<220>
<221> source
<223> /note = "Description of artificial sequence:   Synthetic sequence"

<400>  100

Ala Asp Ala Ala Ala Ala Ala Ala Lys
1               5

<210>  101
<211>  9
<212>  PRT
<213>  Artificial sequence

<220>
<221> source
<223> /note = "Description of artificial sequence:   Synthetic sequence"

<400>  101

Ala Asn Ala Ala Ala Ala Ala Ala Lys
1               5



<210>  102
<211>  9
<212>  PRT
<213>  Artificial sequence

<220>
<221> source
<223> /note = "Description of artificial sequence:   Synthetic sequence"

<400>  102

Ala Lys Ala Ala Ala Ala Ala Ala Lys
1               5



<210>  103
<211>  9
<212>  PRT
<213>  Artificial sequence

<220>
<221> source
<223> /note = "Description of artificial sequence:   Synthetic sequence"

<400>  103

Ala Tyr Ala Ala Ala Ala Ala Ala Lys
1               5

<210>  104
<211>  9
<212>  PRT
<213>  Artificial sequence

<220>
<221> source
<223> /note = "Description of artificial sequence:   Synthetic sequence"

<400>  104

Ala Pro Ala Ala Ala Ala Ala Ala Lys
1               5


<210>  105
<211>  9
<212>  PRT
<213>  Artificial sequence

<220>
<221> source
<223> /note = "Description of artificial sequence:   Synthetic sequence"

<400>  105

Ala Leu Ala Ala Ala Ala Ala Ala Arg
1               5


<210>  106
<211>  9
<212>  PRT
<213>  Artificial sequence

<220>
<221> source
<223> /note = "Description of artificial sequence:   Synthetic sequence"

<400>  106

Ala Leu Ala Ala Ala Ala Ala Ala Tyr
1               5

<210>  107
<211>  9
<212>  PRT
<213>  Artificial sequence

<220>
<221> source
<223> /note = "Description of artificial sequence:   Synthetic sequence"

<400>  107

Ala Leu Ala Ala Ala Ala Ala Ala Ala
1               5

<210>  108
<211>  9
<212>  PRT
<213>  Artificial sequence

<220>
<221> source
<223> /note = "Description of artificial sequence:   Synthetic sequence"

<400>  108

Ala Leu Ala Ala Ala Ala Ala Ala Gln
1               5

<210>  109
<211>  9
<212>  PRT
<213>  Artificial sequence

<220>
<221> source
<223> /note = "Description of artificial sequence:   Synthetic sequence"

<400>  109

Ala Leu Ala Ala Ala Ala Ala Ala Ser
1               5

<210>  110
<211>  9
<212>  PRT
<213>  Artificial sequence

<220>
<221> source
<223> /note = "Description of artificial sequence:   Synthetic sequence"

<400>  110

Ala Leu Ala Ala Ala Ala Ala Ala Thr
1               5


<210>  111
<211>  9
<212>  PRT
<213>  Artificial sequence

<220>
<221> source
<223> /note = "Description of artificial sequence:   Synthetic sequence"

<400>  111

Ala Leu Ala Ala Ala Ala Ala Ala Asn
1               5


<210>  112
<211>  9
<212>  PRT
<213>  Artificial sequence

<220>
<221> source
<223> /note = "Description of artificial sequence:   Synthetic sequence"

<400>  112

Ala Leu Ala Ala Ala Ala Ala Ala Glu
1               5

<210>  113
<211>  9
<212>  PRT
<213>  Artificial sequence

<220>
<221> source
<223> /note = "Description of artificial sequence:   Synthetic sequence"

<400>  113

Ala Phe Ala Lys Ala Ala Ala Ala Phe
1               5


<210>  114
<211>  9
<212>  PRT
<213>  Artificial sequence

<220>
<221> source
<223> /note = "Description of artificial sequence:   Synthetic sequence"

<400>  114

Ala Pro Ala Lys Ala Ala Ala Ala Phe
1               5


<210>  115
<211>  9
<212>  PRT
<213>  Artificial sequence

<220>
<221> source
<223> /note = "Description of artificial sequence:   Synthetic sequence"

<400>  115

Ala Ala Ala Lys Ala Ala Ala Ala Phe
1               5

<210>  116
<211>  9
<212>  PRT
<213>  Artificial sequence

<220>
<221> source
<223> /note = "Description of artificial sequence:   Synthetic sequence"

<400>  116

Ala Lys Ala Lys Ala Ala Ala Ala Phe
1               5



<210>  117
<211>  9
<212>  PRT
<213>  Artificial sequence

<220>
<221> source
<223> /note = "Description of artificial sequence:   Synthetic sequence"

<400>  117

Ala Tyr Ala Lys Ala Ala Ala Ala Ile
1               5



<210>  118
<211>  9
<212>  PRT
<213>  Artificial sequence

<220>
<221> source
<223> /note = "Description of artificial sequence:   Synthetic sequence"

<400>  118

Ala Tyr Ala Lys Ala Ala Ala Ala Leu
1               5

<210>  119
<211>  9
<212>  PRT
<213>  Artificial sequence

<220>
<221> source
<223> /note = "Description of artificial sequence:   Synthetic sequence"

<400>  119

Ala Tyr Ala Lys Ala Ala Ala Ala Val
1               5



<210>  120
<211>  9
<212>  PRT
<213>  Artificial sequence

<220>
<221> source
<223> /note = "Description of artificial sequence:   Synthetic sequence"

<400>  120

Ala Tyr Ala Lys Ala Ala Ala Ala Ala
1               5



<210>  121
<211>  9
<212>  PRT
<213>  Artificial sequence

<220>
<221> source
<223> /note = "Description of artificial sequence:   Synthetic sequence"

<400>  121

Ala Tyr Ala Lys Ala Ala Ala Ala Tyr
1               5

<210>  122
<211>  9
<212>  PRT
<213>  Artificial sequence

<220>
<221> source
<223> /note = "Description of artificial sequence:   Synthetic sequence"

<400>  122

Ala Tyr Ala Lys Ala Ala Ala Ala Lys
1               5



<210>  123
<211>  9
<212>  PRT
<213>  Artificial sequence

<220>
<221> source
<223> /note = "Description of artificial sequence:   Synthetic sequence"

<400>  123

Ala Ser Ala Lys Ala Ala Ala Ala Tyr
1               5



<210>  124
<211>  9
<212>  PRT
<213>  Artificial sequence

<220>
<221> source
<223> /note = "Description of artificial sequence:   Synthetic sequence"

<400>  124

Ala Met Ala Lys Ala Ala Ala Ala Tyr
1               5

<210>  125
<211>  9
<212>  PRT
<213>  Artificial sequence

<220>
<221> source
<223> /note = "Description of artificial sequence:   Synthetic sequence"

<400>  125

Ala Ala Ala Lys Ala Ala Ala Ala Tyr
1               5



<210>  126
<211>  9
<212>  PRT
<213>  Artificial sequence

<220>
<221> source
<223> /note = "Description of artificial sequence:   Synthetic sequence"

<400>  126

Ala Leu Ala Lys Ala Ala Ala Ala Tyr
1               5



<210>  127
<211>  9
<212>  PRT
<213>  Artificial sequence

<220>
<221> source
<223> /note = "Description of artificial sequence:   Synthetic sequence"

<400>  127

Ala Leu Ala Lys Ala Ala Ala Ala Tyr
1               5

<210> 128
<211> 9
<212> PRT
<213> Artificial sequence

<220>
<221> source
<223> /note = "Description of artificial sequence: Synthetic sequence"

<400> 128

Ala Val Ala Lys Ala Ala Ala Ala Tyr
1               5

<210> 129
<211> 9
<212> PRT
<213> Artificial sequence

<220>
<221> source
<223> /note = "Description of artificial sequence: Synthetic sequence"

<400> 129

Ala Lys Ala Lys Ala Ala Ala Ala Tyr
1               5

<210> 130
<211> 9
<212> PRT
<213> Artificial sequence

<220>
<221> source
<223> /note = "Description of artificial sequence: Synthetic sequence"

<400> 130

Ala Asn Ala Lys Ala Ala Ala Ala Tyr
1               5

<210>  131
<211>  9
<212>  PRT
<213>  Artificial sequence

<220>
<221> source
<223> /note = "Description of artificial sequence:   Synthetic sequence"

<400>  131

Ala Asp Ala Lys Ala Ala Ala Ala Tyr
1               5



<210>  132
<211>  9
<212>  PRT
<213>  Artificial sequence

<220>
<221> source
<223> /note = "Description of artificial sequence:   Synthetic sequence"

<400>  132

Ala Gly Ala Lys Ala Ala Ala Ala Tyr
1               5



<210>  133
<211>  9
<212>  PRT
<213>  Artificial sequence

<220>
<221> source
<223> /note = "Description of artificial sequence:   Synthetic sequence"

<400>  133

Ala Pro Ala Lys Ala Ala Ala Ala Tyr
1               5

<210>  134
<211>  9
<212>  PRT
<213>  Artificial sequence

<220>
<221> source
<223> /note = "Description of artificial sequence:   Synthetic sequence"

<400>  134

Ala Tyr Ala Lys Ala Ala Ala Ala Tyr
1               5

<210>  135
<211>  9
<212>  PRT
<213>  Artificial sequence

<220>
<221> source
<223> /note = "Description of artificial sequence:   Synthetic sequence"

<400>  135

Ala His Ala Lys Ala Ala Ala Ala Tyr
1               5

<210>  136
<211>  9
<212>  PRT
<213>  Artificial sequence

<220>
<221> source
<223> /note = "Description of artificial sequence:   Synthetic sequence"

<400>  136

Ala Thr Ala Lys Ala Ala Ala Ala Ala
1               5

<210>  137
<211>  9
<212>  PRT
<213>  Artificial sequence

<220>
<221> source
<223> /note = "Description of artificial sequence:   Synthetic sequence"

<400>  137

Ala Thr Ala Lys Ala Ala Ala Ala Phe
1               5



<210>  138
<211>  9
<212>  PRT
<213>  Artificial sequence

<220>
<221> source
<223> /note = "Description of artificial sequence:   Synthetic sequence"

<400>  138

Ala Thr Ala Lys Ala Ala Ala Ala His
1               5



<210>  139
<211>  9
<212>  PRT
<213>  Artificial sequence

<220>
<221> source
<223> /note = "Description of artificial sequence:   Synthetic sequence"

<400>  139

Ala Thr Ala Lys Ala Ala Ala Ala Val
1               5

<210>  140
<211>  9
<212>  PRT
<213>  Artificial sequence

<220>
<221> source
<223> /note = "Description of artificial sequence:   Synthetic sequence"

<400>  140

Ala Thr Ala Lys Ala Ala Ala Ala Asn
1               5

<210>  141
<211>  9
<212>  PRT
<213>  Artificial sequence

<220>
<221> source
<223> /note = "Description of artificial sequence:   Synthetic sequence"

<400>  141

Ala Thr Ala Lys Ala Ala Ala Ala Asp
1               5

<210>  142
<211>  9
<212>  PRT
<213>  Artificial sequence

<220>
<221> source
<223> /note = "Description of artificial sequence:   Synthetic sequence"

<400>  142

Ala Thr Ala Lys Ala Ala Ala Ala Trp
1               5

<210>  143
<211>  9
<212>  PRT
<213>  Artificial sequence

<220>
<221> source
<223> /note = "Description of artificial sequence:   Synthetic sequence"

<400>  143

Ala Thr Ala Lys Ala Ala Ala Ala Lys
1               5



<210>  144
<211>  9
<212>  PRT
<213>  Artificial sequence

<220>
<221> source
<223> /note = "Description of artificial sequence:   Synthetic sequence"

<400>  144

Ala Thr Ala Lys Ala Ala Ala Ala Ile
1               5



<210>  145
<211>  9
<212>  PRT
<213>  Artificial sequence

<220>
<221> source
<223> /note = "Description of artificial sequence:   Synthetic sequence"

<400>  145

Ala Thr Ala Lys Ala Ala Ala Ala Pro
1               5

<210>  146
<211>  9
<212>  PRT
<213>  Artificial sequence

<220>
<221> source
<223> /note = "Description of artificial sequence:   Synthetic sequence"

<400>  146

Ala Ala Glu Lys Ala Ala Ala Ala Tyr
1               5



<210>  147
<211>  9
<212>  PRT
<213>  Artificial sequence

<220>
<221> source
<223> /note = "Description of artificial sequence:   Synthetic sequence"

<400>  147

Ala Ala Ala Lys Ala Ala Ala Ala Tyr
1               5



<210>  148
<211>  9
<212>  PRT
<213>  Artificial sequence

<220>
<221> source
<223> /note = "Description of artificial sequence:   Synthetic sequence"

<400>  148

Ala Ala Ser Lys Ala Ala Ala Ala Tyr
1               5

<210>  149
<211>  9
<212>  PRT
<213>  Artificial sequence

<220>
<221> source
<223> /note = "Description of artificial sequence:   Synthetic sequence"

<400>  149

Ala Ala Asn Lys Ala Ala Ala Ala Tyr
1               5



<210>  150
<211>  9
<212>  PRT
<213>  Artificial sequence

<220>
<221> source
<223> /note = "Description of artificial sequence:   Synthetic sequence"

<400>  150

Ala Ala Gln Lys Ala Ala Ala Ala Tyr
1               5



<210>  151
<211>  9
<212>  PRT
<213>  Artificial sequence

<220>
<221> source
<223> /note = "Description of artificial sequence:   Synthetic sequence"

<400>  151

Ala Ala Lys Lys Ala Ala Ala Ala Tyr
1               5

<210>  152
<211>  9
<212>  PRT
<213>  Artificial sequence

<220>
<221> source
<223> /note = "Description of artificial sequence:   Synthetic sequence"

<400>  152

Ala Ala Asp Lys Ala Ala Ala Ala Ala
1               5



<210>  153
<211>  9
<212>  PRT
<213>  Artificial sequence

<220>
<221> source
<223> /note = "Description of artificial sequence:   Synthetic sequence"

<400>  153

Ala Ala Asp Lys Ala Ala Ala Ala Trp
1               5



<210>  154
<211>  9
<212>  PRT
<213>  Artificial sequence

<220>
<221> source
<223> /note = "Description of artificial sequence:   Synthetic sequence"

<400>  154

Ala Ala Asp Lys Ala Ala Ala Ala Phe
1               5

<210>  155
<211>  9
<212>  PRT
<213>  Artificial sequence

<220>
<221> source
<223> /note = "Description of artificial sequence:   Synthetic sequence"

<400>  155

Ala Ala Asp Lys Ala Ala Ala Ala Lys
1               5

<210>  156
<211>  9
<212>  PRT
<213>  Artificial sequence

<220>
<221> source
<223> /note = "Description of artificial sequence:   Synthetic sequence"

<400>  156

Ile Ser Glu Tyr Arg His Tyr Ala Tyr
1               5

<210>  157
<211>  9
<212>  PRT
<213>  Artificial sequence

<220>
<221> source
<223> /note = "Description of artificial sequence:   Synthetic sequence"

<400>  157

Val Ala Asp Lys Ala Leu Lys Phe Tyr
1               5

<210>  158
<211>  9
<212>  PRT
<213>  Artificial sequence

<220>
<221> source
<223> /note = "Description of artificial sequence:   Synthetic sequence"

<400>  158

Glu Ile Asp Gly Pro Ala Gly Gln Ala
1               5


<210>  159
<211>  9
<212>  PRT
<213>  Artificial sequence

<220>
<221> source
<223> /note = "Description of artificial sequence:   Synthetic sequence"

<400>  159

Thr Thr Asp Leu Tyr Ala Tyr Glu Gln
1               5


<210>  160
<211>  9
<212>  PRT
<213>  Artificial sequence

<220>
<221> source
<223> /note = "Description of artificial sequence:   Synthetic sequence"

<400>  160

Met Ser Ala Ala Arg Ser Ser Arg Thr
1               5

<210>  161
<211>  9
<212>  PRT
<213>  Artificial sequence

<220>
<221> source
<223> /note = "Description of artificial sequence:   Synthetic sequence"

<400>  161

His Val Asp Ile Arg Thr Leu Glu Asp
1               5



<210>  162
<211>  9
<212>  PRT
<213>  Artificial sequence

<220>
<221> source
<223> /note = "Description of artificial sequence:   Synthetic sequence"

<400>  162

Trp Thr Gly Arg Ala Met Ser Ala Ala
1               5



<210>  163
<211>  9
<212>  PRT
<213>  Artificial sequence

<220>
<221> source
<223> /note = "Description of artificial sequence:   Synthetic sequence"

<400>  163

Tyr Arg Asp Gly Asn Pro Tyr Ala Val
1               5

<210> 164
<211> 9
<212> PRT
<213> Artificial sequence

<220>
<221> source
<223> /note = "Description of artificial sequence: Synthetic sequence"

<400> 164

Leu Ile Arg Ala Ile Asn Ala Gln Lys
1               5

<210> 165
<211> 9
<212> PRT
<213> Artificial sequence

<220>
<221> source
<223> /note = "Description of artificial sequence: Synthetic sequence"

<400> 165

Ile Val Tyr Arg Asp Gly Asn Pro Tyr
1               5

<210> 166
<211> 9
<212> PRT
<213> Artificial sequence

<220>
<221> source
<223> /note = "Description of artificial sequence: Synthetic sequence"

<400> 166

Ile Val Ala Pro Ile Ala Ser Gln Lys
1               5

<210>  167
<211>  9
<212>  PRT
<213>  Artificial sequence

<220>
<221> source
<223> /note = "Description of artificial sequence:   Synthetic sequence"

<400>  167

Ile Ile Leu Glu Ala Val Tyr Ala Lys
1               5


<210>  168
<211>  9
<212>  PRT
<213>  Artificial sequence

<220>
<221> source
<223> /note = "Description of artificial sequence:   Synthetic sequence"

<400>  168

His Leu Asp Lys Lys Gln Arg Phe His
1               5


<210>  169
<211>  9
<212>  PRT
<213>  Artificial sequence

<220>
<221> source
<223> /note = "Description of artificial sequence:   Synthetic sequence"

<400>  169

Ala Met Ser Ala Ala Arg Ser Ser Arg
1               5

<210>  170
<211>  9
<212>  PRT
<213>  Artificial sequence

<220>
<221> source
<223> /note = "Description of artificial sequence:   Synthetic sequence"

<400>  170

Ala Met Phe Gln Asp Pro Gln Glu Arg
1               5



<210>  171
<211>  9
<212>  PRT
<213>  Artificial sequence

<220>
<221> source
<223> /note = "Description of artificial sequence:   Synthetic sequence"

<400>  171

Ala Val Tyr Ala Lys Gln Gln Leu Leu
1               5



<210>  172
<211>  9
<212>  PRT
<213>  Artificial sequence

<220>
<221> source
<223> /note = "Description of artificial sequence:   Synthetic sequence"

<400>  172

His Tyr Asn Ile Val Thr Phe Ala Ala
1               5

<210>  173
<211>  9
<212>  PRT
<213>  Artificial sequence

<220>
<221> source
<223> /note = "Description of artificial sequence:   Synthetic sequence"

<400>  173

Ser Ala Ala Arg Ser Ser Arg Thr Arg
1               5



<210>  174
<211>  9
<212>  PRT
<213>  Artificial sequence

<220>
<221> source
<223> /note = "Description of artificial sequence:   Synthetic sequence"

<400>  174

Lys Phe Tyr Ser Lys Ile Ser Glu Tyr
1               5



<210>  175
<211>  9
<212>  PRT
<213>  Artificial sequence

<220>
<221> source
<223> /note = "Description of artificial sequence:   Synthetic sequence"

<400>  175

Ser Leu Tyr Gly Thr Thr Leu Glu Gln
1               5

<210>  176
<211>  9
<212>  PRT
<213>  Artificial sequence

<220>
<221> source
<223> /note = "Description of artificial sequence:   Synthetic sequence"

<400>  176

Tyr Asn Ile Val Thr Phe Ala Ala Lys
1               5


<210>  177
<211>  9
<212>  PRT
<213>  Artificial sequence

<220>
<221> source
<223> /note = "Description of artificial sequence:   Synthetic sequence"

<400>  177

Gln Gln Leu Leu Arg Arg Glu Val Tyr
1               5


<210>  178
<211>  9
<212>  PRT
<213>  Artificial sequence

<220>
<221> source
<223> /note = "Description of artificial sequence:   Synthetic sequence"

<400>  178

Ala Val Ala Asp Lys Ala Leu Lys Phe
1               5

<210>  179
<211>  9
<212>  PRT
<213>  Artificial sequence

<220>
<221> source
<223> /note = "Description of artificial sequence:   Synthetic sequence"

<400>  179

Gln Gln Tyr Asn Lys Pro Leu Ala Asp
1               5

<210>  180
<211>  9
<212>  PRT
<213>  Artificial sequence

<220>
<221> source
<223> /note = "Description of artificial sequence:   Synthetic sequence"

<400>  180

Lys Ile Ser Glu Tyr Arg His Tyr Ala
1               5

<210>  181
<211>  9
<212>  PRT
<213>  Artificial sequence

<220>
<221> source
<223> /note = "Description of artificial sequence:   Synthetic sequence"

<400>  181

Arg His Tyr Ala Tyr Ser Leu Tyr Gly
1               5

<210>  182
<211>  9
<212>  PRT
<213>  Artificial sequence

<220>
<221> source
<223> /note = "Description of artificial sequence:   Synthetic sequence"

<400>  182

Ala Ala Arg Ser Ser Arg Thr Arg Arg
1               5



<210>  183
<211>  9
<212>  PRT
<213>  Artificial sequence

<220>
<221> source
<223> /note = "Description of artificial sequence:   Synthetic sequence"

<400>  183

Ala Ala Lys Ala Asp Ser Thr Leu Arg
1               5



<210>  184
<211>  9
<212>  PRT
<213>  Artificial sequence

<220>
<221> source
<223> /note = "Description of artificial sequence:   Synthetic sequence"

<400>  184

Val Tyr Ala Lys Gln Gln Leu Leu Arg
1               5

<210>  185
<211>  9
<212>  PRT
<213>  Artificial sequence

<220>
<221> source
<223> /note = "Description of artificial sequence:   Synthetic sequence"

<400>  185

Tyr Ala Val Ala Asp Lys Ala Leu Lys
1               5



<210>  186
<211>  9
<212>  PRT
<213>  Artificial sequence

<220>
<221> source
<223> /note = "Description of artificial sequence:   Synthetic sequence"

<400>  186

Lys Ala Asp Ser Thr Leu Arg Leu Ala
1               5



<210>  187
<211>  9
<212>  PRT
<213>  Artificial sequence

<220>
<221> source
<223> /note = "Description of artificial sequence:   Synthetic sequence"

<400>  187

Thr Gly Arg Ala Met Ser Ala Ala Arg
1               5

<210>  188
<211>  9
<212>  PRT
<213>  Artificial sequence

<220>
<221> source
<223> /note = "Description of artificial sequence:   Synthetic sequence"

<400>  188

Val Ala Pro Ile Ala Ser Gln Lys Pro
1               5


<210>  189
<211>  9
<212>  PRT
<213>  Artificial sequence

<220>
<221> source
<223> /note = "Description of artificial sequence:   Synthetic sequence"

<400>  189

Ala Tyr Ser Leu Tyr Gly Thr Thr Leu
1               5


<210>  190
<211>  9
<212>  PRT
<213>  Artificial sequence

<220>
<221> source
<223> /note = "Description of artificial sequence:   Synthetic sequence"

<400>  190

Lys Ala Leu Lys Phe Tyr Ser Lys Ile
1               5

<210>  191
<211>  9
<212>  PRT
<213>  Artificial sequence

<220>
<221> source
<223> /note = "Description of artificial sequence:   Synthetic sequence"

<400>  191

Arg Phe His Asn Ile Arg Gly Arg Trp
1               5

<210>  192
<211>  9
<212>  PRT
<213>  Artificial sequence

<220>
<221> source
<223> /note = "Description of artificial sequence:   Synthetic sequence"

<400>  192

Arg Ala His Tyr Asn Ile Val Thr Phe
1               5

<210>  193
<211>  9
<212>  PRT
<213>  Artificial sequence

<220>
<221> source
<223> /note = "Description of artificial sequence:   Synthetic sequence"

<400>  193

Val Tyr Asp Phe Ala Phe Arg Asp Leu
1               5

<210>  194
<211>  9
<212>  PRT
<213>  Artificial sequence

<220>
<221> source
<223> /note = "Description of artificial sequence:   Synthetic sequence"

<400>  194

Glu Tyr Arg His Tyr Ala Tyr Ser Leu
1               5

<210>  195
<211>  9
<212>  PRT
<213>  Artificial sequence

<220>
<221> source
<223> /note = "Description of artificial sequence:   Synthetic sequence"

<400>  195

Leu Gln Thr Thr Ile His Asp Ile Ile
1               5

<210>  196
<211>  9
<212>  PRT
<213>  Artificial sequence

<220>
<221> source
<223> /note = "Description of artificial sequence:   Synthetic sequence"

<400>  196

Pro Tyr Ala Val Ala Asp Lys Ala Leu
1               5

<210>  197
<211>  9
<212>  PRT
<213>  Artificial sequence

<220>
<221> source
<223> /note = "Description of artificial sequence:   Synthetic sequence"

<400>  197

Met His Gln Lys Arg Thr Ala Met Phe
1               5



<210>  198
<211>  9
<212>  PRT
<213>  Artificial sequence

<220>
<221> source
<223> /note = "Description of artificial sequence:   Synthetic sequence"

<400>  198

His Tyr Ala Tyr Ser Leu Tyr Gly Thr
1               5



<210>  199
<211>  9
<212>  PRT
<213>  Artificial sequence

<220>
<221> source
<223> /note = "Description of artificial sequence:   Synthetic sequence"

<400>  199

Leu Leu Arg Arg Glu Val Tyr Asp Phe
1               5

<210>  200
<211>  9
<212>  PRT
<213>  Homo sapiens

<400>  200

His Leu Asp Met Leu Arg His Leu Tyr
1               5


<210>  201
<211>  9
<212>  PRT
<213>  Homo sapiens

<400>  201

Leu Leu Asp Ile Asp Glu Thr Glu Tyr
1               5


<210>  202
<211>  9
<212>  PRT
<213>  Homo sapiens

<400>  202

Gly Thr Gln Leu Phe Glu Asp Asn Tyr
1               5


<210>  203
<211>  9
<212>  PRT
<213>  Homo sapiens

<400>  203

Leu Thr Cys Ser Pro Gln Pro Glu Tyr
1               5

<210>  204
<211>  9
<212>  PRT
<213>  Homo sapiens

<400>  204

Glu Thr Leu Glu Glu Ile Thr Gly Tyr
1               5

<210>  205
<211>  10
<212>  PRT
<213>  Homo sapiens

<400>  205

Phe Thr His Gln Ser Asp Val Trp Ser Tyr
1               5                   10

<210>  206
<211>  10
<212>  PRT
<213>  Homo sapiens

<400>  206

Arg Leu Leu Asp Ile Asp Glu Thr Glu Tyr
1               5                   10

<210>  207
<211>  10
<212>  PRT
<213>  Homo sapiens

<400>  207

Thr Leu Glu Glu Ile Thr Gly Tyr Leu Tyr
1               5                   10

<210>  208
<211>  10
<212>  PRT
<213>  Homo sapiens

<400>  208

Tyr Val Met Ala Gly Val Gly Ser Pro Tyr
1               5                   10

<210>  209
<211>  10
<212>  PRT
<213>  Homo sapiens

<400>  209

Gly Thr Pro Thr Ala Glu Asn Pro Glu Tyr
1               5                   10

<210>  210
<211>  10
<212>  PRT
<213>  Homo sapiens

<400>  210

Leu Ile Gln Arg Asn Pro Gln Leu Cys Tyr
1               5                   10

<210>  211
<211>  10
<212>  PRT
<213>  Homo sapiens

<400>  211

Val Val Gln Gly Asn Leu Glu Leu Thr Tyr
1               5                   10

<210> 212
<211> 10
<212> PRT
<213> Homo sapiens

<400> 212

Met Gly Asp Leu Val Asp Ala Glu Glu Tyr
1               5                   10


<210> 213
<211> 9
<212> PRT
<213> Homo sapiens

<400> 213

Lys Ile Arg Lys Tyr Thr Met Arg Arg
1               5


<210> 214
<211> 9
<212> PRT
<213> Homo sapiens

<400> 214

Val Val Phe Gly Ile Leu Ile Lys Arg
1               5


<210> 215
<211> 9
<212> PRT
<213> Homo sapiens

<400> 215

Leu Val Lys Ser Pro Asn His Val Lys
1               5

<210>  216
<211>  9
<212>  PRT
<213>  Homo sapiens

<400>  216

Val Leu Arg Glu Asn Thr Ser Pro Lys
1               5


<210>  217
<211>  9
<212>  PRT
<213>  Homo sapiens

<400>  217

Ile Leu Ile Lys Arg Arg Gln Gln Lys
1               5


<210>  218
<211>  9
<212>  PRT
<213>  Homo sapiens

<400>  218

Ile Leu Trp Lys Asp Ile Phe His Lys
1               5


<210>  219
<211>  9
<212>  PRT
<213>  Homo sapiens

<400>  219

Lys Ile Thr Asp Phe Gly Leu Ala Arg
1               5

<210>  220
<211>  9
<212>  PRT
<213>  Homo sapiens

<400>  220

Gly Val Val Phe Gly Ile Leu Ile Lys
1               5

<210>  221
<211>  9
<212>  PRT
<213>  Homo sapiens

<400>  221

Gln Val Cys Thr Gly Thr Asp Met Lys
1               5

<210>  222
<211>  9
<212>  PRT
<213>  Homo sapiens

<400>  222

Leu Leu Asp His Val Arg Glu Asn Arg
1               5

<210>  223
<211>  9
<212>  PRT
<213>  Homo sapiens

<400>  223

Cys Val Asn Cys Ser Gln Phe Leu Arg
1               5

<210>  224
<211>  9
<212>  PRT
<213>  Homo sapiens

<400>  224

Thr Val Cys Ala Gly Gly Cys Ala Arg
1               5

<210>  225
<211>  9
<212>  PRT
<213>  Homo sapiens

<400>  225

Ile Leu Lys Glu Thr Glu Leu Arg Lys
1               5

<210>  226
<211>  9
<212>  PRT
<213>  Homo sapiens

<400>  226

Val Thr Ala Glu Asp Gly Thr Gln Arg
1               5

<210>  227
<211>  9
<212>  PRT
<213>  Homo sapiens

<400>  227

Asp Leu Ser Tyr Met Pro Ile Trp Lys
1               5

<210>  228
<211>  10
<212>  PRT
<213>  Homo sapiens

<400>  228

Thr Ile Leu Trp Lys Asp Ile Phe His Lys
1               5                   10


<210>  229
<211>  10
<212>  PRT
<213>  Homo sapiens

<400>  229

Gly Thr Gln Arg Cys Glu Lys Cys Ser Lys
1               5                   10


<210>  230
<211>  10
<212>  PRT
<213>  Homo sapiens

<400>  230

Lys Val Leu Arg Glu Asn Thr Ser Pro Lys
1               5                   10


<210>  231
<211>  10
<212>  PRT
<213>  Homo sapiens

<400>  231

Gln Leu Arg Ser Leu Thr Glu Ile Leu Lys
1               5                   10

<210>  232
<211>  10
<212>  PRT
<213>  Homo sapiens

<400>  232

Arg Leu Val His Arg Asp Leu Ala Ala Arg
1               5                   10


<210>  233
<211>  10
<212>  PRT
<213>  Homo sapiens

<400>  233

Leu Leu Asn Trp Cys Met Gln Ile Ala Lys
1               5                   10


<210>  234
<211>  10
<212>  PRT
<213>  Homo sapiens

<400>  234

Thr Ile Asp Val Tyr Met Ile Met Val Lys
1               5                   10


<210>  235
<211>  10
<212>  PRT
<213>  Homo sapiens

<400>  235

Arg Ile Leu Lys Glu Thr Glu Leu Arg Lys
1               5                   10

<210>  236
<211>  10
<212>  PRT
<213>  Homo sapiens

<400>  236

Val Leu Val Lys Ser Pro Asn His Val Lys
1               5                   10


<210>  237
<211>  10
<212>  PRT
<213>  Homo sapiens

<400>  237

Ser Val Phe Gln Asn Leu Gln Val Ile Arg
1               5                   10


<210>  238
<211>  10
<212>  PRT
<213>  Homo sapiens

<400>  238

His Thr Val Pro Trp Asp Gln Leu Phe Arg
1               5                   10


<210>  239
<211>  10
<212>  PRT
<213>  Homo sapiens

<400>  239

Ile Leu Lys Gly Gly Val Leu Ile Gln Arg
1               5                   10

<210>  240
<211>  10
<212>  PRT
<213>  Homo sapiens

<400>  240

Leu Val Ser Glu Phe Ser Arg Met Ala Arg
1               5                   10

<210>  241
<211>  10
<212>  PRT
<213>  Homo sapiens

<400>  241

Gly Val Val Phe Gly Ile Leu Ile Lys Arg
1               5                   10

<210>  242
<211>  10
<212>  PRT
<213>  Homo sapiens

<400>  242

Cys Val Ala Arg Cys Pro Ser Gly Val Lys
1               5                   10

<210>  243
<211>  10
<212>  PRT
<213>  Homo sapiens

<400>  243

Val Val Phe Gly Ile Leu Ile Lys Arg Arg
1               5                   10

<210>  244
<211>  10
<212>  PRT
<213>  Homo sapiens

<400>  244

Gly Ile Leu Ile Lys Arg Arg Gln Gln Lys
1               5                   10

<210>  245
<211>  10
<212>  PRT
<213>  Homo sapiens

<400>  245

Arg Thr Val Cys Ala Gly Gly Cys Ala Arg
1               5                   10

<210>  246
<211>  10
<212>  PRT
<213>  Homo sapiens

<400>  246

Gly Leu Ala Cys His Gln Leu Cys Ala Arg
1               5                   10

<210>  247
<211>  10
<212>  PRT
<213>  Homo sapiens

<400>  247

Lys Ile Pro Val Ala Ile Lys Val Leu Arg
1               5                   10

<210> 248
<211> 9
<212> PRT
<213> Epstein-Barr virus

<400> 248

Val Gly Glu Ala Asp Tyr Phe Glu Tyr
1               5

<210> 249
<211> 9
<212> PRT
<213> Epstein-Barr virus

<400> 249

Pro Leu Arg Glu Ser Ile Val Cys Tyr
1               5

<210> 250
<211> 10
<212> PRT
<213> Epstein-Barr virus

<400> 250

Pro Val Gly Glu Ala Asp Tyr Phe Glu Tyr
1               5                   10

<210> 251
<211> 10
<212> PRT
<213> Epstein-Barr virus

<400> 251

Gly Thr Trp Val Ala Gly Val Phe Val Tyr
1               5                   10

<210>  252
<211>  9
<212>  PRT
<213>  Epstein-Barr virus

<400>  252

Gly Val Phe Val Tyr Gly Gly Ser Lys
1               5

<210>  253
<211>  9
<212>  PRT
<213>  Epstein-Barr virus

<400>  253

Lys Thr Ser Leu Tyr Asn Leu Arg Arg
1               5

<210>  254
<211>  9
<212>  PRT
<213>  Epstein-Barr virus

<400>  254

Ala Ile Lys Asp Leu Val Met Thr Lys
1               5

<210>  255
<211>  10
<212>  PRT
<213>  Epstein-Barr virus

<400>  255

Gln Thr His Ile Phe Ala Glu Val Leu Lys
1               5                   10

<210>  256
<211>  10
<212>  PRT
<213>  Epstein-Barr virus

<400>  256

Gly Thr Ala Leu Ala Ile Pro Gln Cys Arg
1               5                   10



<210>  257
<211>  9
<212>  PRT
<213>  Flu virus

<400>  257

Cys Thr Glu Leu Lys Leu Ser Asp Tyr
1               5



<210>  258
<211>  9
<212>  PRT
<213>  Flu virus

<400>  258

Ser Thr Leu Glu Leu Arg Ser Arg Tyr
1               5



<210>  259
<211>  9
<212>  PRT
<213>  Flu virus

<400>  259

Ile Leu Arg Gly Ser Val Ala His Lys
1               5

<210>  260
<211>  9
<212>  PRT
<213>  Flu virus

<400>  260

Arg Met Cys Asn Ile Leu Lys Gly Lys
1               5

<210>  261
<211>  9
<212>  PRT
<213>  Flu virus

<400>  261

Leu Met Gln Gly Ser Thr Leu Pro Arg
1               5

<210>  262
<211>  9
<212>  PRT
<213>  Flu virus

<400>  262

Met Ile Asp Gly Ile Gly Arg Phe Tyr
1               5

<210>  263
<211>  9
<212>  PRT
<213>  Flu virus

<400>  263

Met Val Leu Ser Ala Phe Asp Glu Arg
1               5

<210>  264
<211>  9
<212>  PRT
<213>  Flu virus

<400>  264

Tyr Ile Gln Met Cys Thr Glu Leu Lys
1               5

<210>  265
<211>  9
<212>  PRT
<213>  Flu virus

<400>  265

Gly Ile Asn Asp Arg Asn Phe Trp Arg
1               5

<210>  266
<211>  10
<212>  PRT
<213>  Flu virus

<400>  266

Ser Leu Met Gln Gly Ser Thr Leu Pro Arg
1               5                   10

<210>  267
<211>  10
<212>  PRT
<213>  Flu virus

<400>  267

Lys Met Ile Asp Gly Ile Gly Arg Phe Tyr
1               5                   10

<210>  268
<211>  10
<212>  PRT
<213>  Flu virus

<400>  268

Leu Ile Leu Arg Gly Ser Val Ala His Lys
1               5                   10

<210>  269
<211>  10
<212>  PRT
<213>  Flu virus

<400>  269

Arg Ser Gly Ala Ala Gly Ala Ala Val Lys
1               5                   10

<210>  270
<211>  10
<212>  PRT
<213>  Flu virus

<400>  270

Ser Ser Thr Leu Glu Leu Arg Ser Arg Tyr
1               5                   10

<210>  271
<211>  10
<212>  PRT
<213>  Flu virus

<400>  271

Arg Ser Arg Tyr Trp Ala Ile Arg Thr Arg
1               5                   10

<210>  272
<211>  10
<212>  PRT
<213>  Flu virus

<400>  272

Arg Met Val Leu Ser Ala Phe Asp Glu Arg
1               5                   10

<210>  273
<211>  9
<212>  PRT
<213>  Flu virus

<400>  273

Phe Tyr Ile Gln Met Cys Thr Glu Leu
1               5

<210>  274
<211>  9
<212>  PRT
<213>  Flu virus

<400>  274

Ala Tyr Glu Arg Met Cys Asn Ile Leu
1               5

<210>  275
<211>  10
<212>  PRT
<213>  Flu virus

<400>  275

Arg Phe Tyr Ile Gln Met Cys Thr Glu Leu
1               5                   10

<210>  276
<211>  9
<212>  PRT
<213>  Hepatitis B virus

<400>  276

Leu Leu Asp Thr Ala Ser Ala Leu Tyr
1               5

<210>  277
<211>  9
<212>  PRT
<213>  Hepatitis B virus

<400>  277

Ser Leu Asp Val Ser Ala Ala Phe Tyr
1               5

<210>  278
<211>  9
<212>  PRT
<213>  Hepatitis B virus

<400>  278

Pro Thr Thr Gly Arg Thr Ser Leu Tyr
1               5

<210>  279
<211>  9
<212>  PRT
<213>  Hepatitis B virus

<400>  279

Met Ser Thr Thr Asp Leu Glu Ala Tyr
1               5

<210>  280
<211>  9
<212>  PRT
<213>  Hepatitis B virus

<400>  280

Leu Thr Lys Gln Tyr Leu Asn Leu Tyr
1               5

<210>  281
<211>  9
<212>  PRT
<213>  Hepatitis B virus

<400>  281

Lys Val Gly Asn Phe Thr Gly Leu Tyr
1               5

<210>  282
<211>  9
<212>  PRT
<213>  Hepatitis B virus

<400>  282

Met Ser Pro Thr Asp Leu Glu Ala Tyr
1               5

<210>  283
<211>  9
<212>  PRT
<213>  Hepatitis B virus

<400>  283

Phe Ser Gln Phe Ser Arg Gly Asn Tyr
1               5

<210>  284
<211>  9
<212>  PRT
<213>  Hepatitis B virus

<400>  284

Pro Ser Ser Trp Ala Phe Ala Lys Tyr
1               5

<210>  285
<211>  9
<212>  PRT
<213>  Hepatitis B virus

<400>  285

Gln Ser Ala Val Arg Lys Glu Ala Tyr
1               5

<210>  286
<211>  9
<212>  PRT
<213>  Hepatitis B virus

<400>  286

Pro Leu Asp Lys Gly Ile Lys Pro Tyr
1               5

<210>  287
<211>  9
<212>  PRT
<213>  Hepatitis B virus

<400>  287

Ser Leu Met Leu Leu Tyr Lys Thr Tyr
1               5

<210>  288
<211>  9
<212>  PRT
<213>  Hepatitis B virus

<400>  288

Ala Ser Arg Asp Leu Val Val Ser Tyr
1               5

<210>  289
<211>  9
<212>  PRT
<213>  Hepatitis B virus

<400>  289

Pro Ser Arg Gly Arg Leu Gly Leu Tyr
1               5

<210>  290
<211>  9
<212>  PRT
<213>  Hepatitis B virus

<400>  290

Ser Ser Thr Ser Arg Asn Ile Asn Tyr
1               5

<210>  291
<211>  10
<212>  PRT
<213>  Hepatitis B virus

<400>  291

Asp Leu Leu Asp Thr Ala Ser Ala Leu Tyr
1               5                   10

<210>  292
<211>  10
<212>  PRT
<213>  Hepatitis B virus

<400>  292

Leu Leu Asp Pro Arg Val Arg Gly Leu Tyr
1               5                   10

<210>  293
<211>  10
<212>  PRT
<213>  Hepatitis B virus

<400>  293

Leu Ser Leu Asp Val Ser Ala Ala Phe Tyr
1               5                   10

<210>  294
<211>  10
<212>  PRT
<213>  Hepatitis B virus

<400>  294

Phe Leu Cys Gln Gln Tyr Leu His Leu Tyr
1               5                   10

<210>  295
<211>  10
<212>  PRT
<213>  Hepatitis B virus

<400>  295

Gln Thr Phe Gly Arg Lys Leu His Leu Tyr
1               5                   10

<210>  296
<211>  10
<212>  PRT
<213>  Hepatitis B virus

<400>  296

Lys Thr Tyr Gly Arg Lys Leu His Leu Tyr
1               5                   10

<210>  297
<211>  10
<212>  PRT
<213>  Hepatitis B virus

<400>  297

Lys Thr Phe Gly Arg Lys Leu His Leu Tyr
1               5                   10

<210>  298
<211>  10
<212>  PRT
<213>  Hepatitis B virus

<400>  298

Leu Gln Asp Pro Arg Val Arg Ala Leu Tyr
1               5                   10

<210>  299
<211>  10
<212>  PRT
<213>  Hepatitis B virus

<400>  299

Thr Thr Pro Ala Gln Gly Thr Ser Met Tyr
1               5                   10

<210> 300
<211> 10
<212> PRT
<213> Hepatitis B virus

<400> 300

Leu Ser Ser Thr Ser Arg Asn Ile Asn Tyr
1               5                   10

<210> 301
<211> 10
<212> PRT
<213> Hepatitis B virus

<400> 301

Pro Leu Asp Lys Gly Ile Lys Pro Tyr Tyr
1               5                   10

<210> 302
<211> 10
<212> PRT
<213> Hepatitis B virus

<400> 302

His Ser Ala Ser Phe Cys Gly Ser Pro Tyr
1               5                   10

<210> 303
<211> 10
<212> PRT
<213> Hepatitis B virus

<400> 303

Phe Leu Thr Lys Gln Tyr Leu Asn Leu Tyr
1               5                   10

<210> 304
<211> 10
<212> PRT
<213> Hepatitis B virus

<400> 304

Arg Ser Ala Ser Phe Cys Gly Ser Pro Tyr
1               5                   10

<210> 305
<211> 10
<212> PRT
<213> Hepatitis B virus

<400> 305

Trp Leu Trp Gly Met Asp Ile Asp Pro Tyr
1               5                   10

<210> 306
<211> 10
<212> PRT
<213> Hepatitis B virus

<400> 306

Thr Thr Pro Ala Gln Gly Thr Ser Met Tyr
1               5                   10

<210> 307
<211> 10
<212> PRT
<213> Hepatitis B virus

<400> 307

His Thr Leu Trp Lys Ala Gly Ile Leu Tyr
1               5                   10

<210>  308
<211>  10
<212>  PRT
<213>  Hepatitis B virus

<400>  308

Thr Ser Cys Pro Pro Ile Cys Pro Gly Tyr
1               5                   10

<210>  309
<211>  10
<212>  PRT
<213>  Hepatitis B virus

<400>  309

Lys Ser Val Gln His Leu Glu Ser Leu Tyr
1               5                   10

<210>  310
<211>  10
<212>  PRT
<213>  Hepatitis B virus

<400>  310

Asn Leu Tyr Val Ser Leu Leu Leu Leu Tyr
1               5                   10

<210>  311
<211>  10
<212>  PRT
<213>  Hepatitis B virus

<400>  311

Trp Met Met Trp Tyr Trp Gly Pro Ser Leu
1               5                   10

<210>  312
<211>  9
<212>  PRT
<213>  Hepatitis B virus

<400>  312

Leu Leu Tyr Gln Thr Phe Gly Arg Lys
1               5

<210>  313
<211>  9
<212>  PRT
<213>  Hepatitis B virus

<400>  313

Ile Met Pro Ala Arg Phe Tyr Pro Lys
1               5

<210>  314
<211>  9
<212>  PRT
<213>  Hepatitis B virus

<400>  314

Cys Leu His Gln Ser Pro Val Arg Lys
1               5

<210>  315
<211>  9
<212>  PRT
<213>  Hepatitis B virus

<400>  315

Ser Ala Ile Cys Ser Val Val Arg Arg
1               5

<210>  316
<211>  9
<212>  PRT
<213>  Hepatitis B virus

<400>  316

His Leu His Gln Asp Ile Ile Lys Lys
1               5

<210>  317
<211>  10
<212>  PRT
<213>  Hepatitis B virus

<400>  317

Ser Leu Pro Gln Glu His Ile Ile Gln Lys
1               5                   10

<210>  318
<211>  10
<212>  PRT
<213>  Hepatitis B virus

<400>  318

Ser Met Phe Pro Ser Cys Cys Cys Thr Lys
1               5                   10

<210>  319
<211>  10
<212>  PRT
<213>  Hepatitis B virus

<400>  319

Ser Met Tyr Pro Ser Cys Cys Cys Thr Lys
1               5                   10

<210>  320
<211>  10
<212>  PRT
<213>  Hepatitis B virus

<400>  320

Gln Ala Phe Thr Phe Ser Pro Thr Tyr Lys
1               5                   10

<210>  321
<211>  10
<212>  PRT
<213>  Hepatitis B virus

<400>  321

Leu Leu Leu Tyr Gln Thr Phe Gly Arg Lys
1               5                   10

<210>  322
<211>  10
<212>  PRT
<213>  Hepatitis B virus

<400>  322

Tyr Met Asp Asp Val Val Leu Gly Ala Lys
1               5                   10

<210>  323
<211>  10
<212>  PRT
<213>  Hepatitis B virus

<400>  323

Thr Ser Ala Ile Cys Ser Val Val Arg Arg
1               5                   10

<210>  324
<211>  9
<212>  PRT
<213>  Hepatitis B virus

<400>  324

Pro Thr Tyr Lys Ala Phe Leu Cys Lys
1               5

<210>  325
<211>  9
<212>  PRT
<213>  Hepatitis B virus

<400>  325

Pro Thr Asp Leu Glu Ala Tyr Phe Lys
1               5

<210>  326
<211>  9
<212>  PRT
<213>  Hepatitis B virus

<400>  326

Lys Tyr Thr Ser Phe Pro Trp Leu Leu
1               5

<210>  327
<211>  9
<212>  PRT
<213>  Hepatitis B virus

<400>  327

Leu Tyr Ala Ala Val Thr Asn Phe Leu
1               5

<210>  328
<211>  9
<212>  PRT
<213>  Hepatitis B virus

<400>  328

Phe Tyr Pro Asn Leu Thr Lys Tyr Leu
1               5

<210>  329
<211>  9
<212>  PRT
<213>  Hepatitis B virus

<400>  329

Leu Tyr Ser Ser Thr Val Pro Ser Phe
1               5

<210>  330
<211>  9
<212>  PRT
<213>  Hepatitis B virus

<400>  330

Phe Tyr Pro Lys Val Thr Lys Tyr Leu
1               5

<210>  331
<211>  9
<212>  PRT
<213>  Hepatitis B virus

<400>  331

Phe Tyr Pro Asn Val Thr Lys Tyr Leu
1               5

<210>  332
<211>  9
<212>  PRT
<213>  Hepatitis B virus

<400>  332

Leu Tyr Ser Ile Leu Ser Pro Phe Leu
1               5

<210>  333
<211>  9
<212>  PRT
<213>  Hepatitis B virus

<400>  333

Leu Tyr Ser Ser Thr Val Pro Val Leu
1               5

<210>  334
<211>  9
<212>  PRT
<213>  Hepatitis B virus

<400>  334

Leu Tyr Asn Ile Leu Ser Pro Phe Leu
1               5

<210>  335
<211>  9
<212>  PRT
<213>  Hepatitis B virus

<400>  335

Asn Tyr Arg Val Ser Trp Pro Lys Phe
1               5

<210>  336
<211>  9
<212>  PRT
<213>  Hepatitis B virus

<400>  336

His Tyr Phe Gln Thr Arg His Tyr Leu
1               5

<210>  337
<211>  9
<212>  PRT
<213>  Hepatitis B virus

<400>  337

His Tyr Phe Lys Thr Arg His Tyr Leu
1               5

<210>  338
<211>  9
<212>  PRT
<213>  Hepatitis B virus

<400>  338

Gly Tyr Pro Ala Leu Met Pro Leu Tyr
1               5

<210>  339
<211>  9
<212>  PRT
<213>  Hepatitis B virus

<400>  339

Ala Tyr Arg Pro Pro Asn Ala Pro Ile
1               5

<210> 340
<211> 9
<212> PRT
<213> Hepatitis B virus

<400> 340

Leu Tyr Gln Thr Phe Gly Arg Lys Leu
1               5

<210> 341
<211> 9
<212> PRT
<213> Hepatitis B virus

<400> 341

Ser Tyr Gln His Phe Arg Arg Leu Leu
1               5

<210> 342
<211> 10
<212> PRT
<213> Hepatitis B virus

<400> 342

Leu Tyr Ser His Pro Ile Ile Leu Gly Phe
1               5                   10

<210> 343
<211> 10
<212> PRT
<213> Hepatitis B virus

<400> 343

Leu Tyr Ala Ala Val Thr Asn Phe Leu Leu
1               5                   10

<210> 344
<211> 10
<212> PRT
<213> Hepatitis B virus

<400> 344

Leu Tyr Arg Pro Leu Leu Ser Leu Pro Phe
1               5                   10

<210> 345
<211> 10
<212> PRT
<213> Hepatitis B virus

<400> 345

Ser Tyr Gln His Phe Arg Arg Leu Leu Leu
1               5                   10

<210> 346
<211> 10
<212> PRT
<213> Hepatitis B virus

<400> 346

Ser Tyr Gln His Phe Arg Lys Leu Leu Leu
1               5                   10

<210> 347
<211> 10
<212> PRT
<213> Hepatitis B virus

<400> 347

Tyr Tyr Pro Glu His Leu Val Asn His Tyr
1               5                   10

<210>  348
<211>  10
<212>  PRT
<213>  Hepatitis B virus

<400>  348

Ala Tyr Arg Pro Pro Asn Ala Pro Ile Leu
1               5                   10

<210>  349
<211>  10
<212>  PRT
<213>  Hepatitis B virus

<400>  349

Gly Tyr Arg Trp Met Cys Leu Arg Arg Phe
1               5                   10

<210>  350
<211>  10
<212>  PRT
<213>  Hepatitis B virus

<400>  350

Asn Phe Leu Leu Ser Leu Gly Ile His Leu
1               5                   10

<210>  351
<211>  9
<212>  PRT
<213>  Hepatitis B virus

<400>  351

Tyr Val Ser Leu Met Leu Leu Tyr Lys
1               5

<210>  352
<211>  9
<212>  PRT
<213>  Hepatitis B virus

<400>  352

Leu Leu Tyr Lys Thr Phe Gly Arg Lys
1               5

<210>  353
<211>  9
<212>  PRT
<213>  Hepatitis B virus

<400>  353

Leu Leu Tyr Lys Thr Tyr Gly Arg Lys
1               5

<210>  354
<211>  9
<212>  PRT
<213>  Hepatitis B virus

<400>  354

Val Thr Lys Tyr Leu Pro Leu Asp Lys
1               5

<210>  355
<211>  9
<212>  PRT
<213>  Hepatitis B virus

<400>  355

Arg His Tyr Leu His Thr Leu Trp Lys
1               5

<210>  356
<211>  9
<212>  PRT
<213>  Hepatitis B virus

<400>  356

Ser Thr Val Pro Ser Phe Asn Pro Lys
1               5

<210>  357
<211>  9
<212>  PRT
<213>  Hepatitis B virus

<400>  357

Thr Thr Asp Leu Glu Ala Tyr Phe Lys
1               5

<210>  358
<211>  9
<212>  PRT
<213>  Hepatitis B virus

<400>  358

Tyr Val Ser Leu Leu Leu Leu Tyr Lys
1               5

<210>  359
<211>  9
<212>  PRT
<213>  Hepatitis B virus

<400>  359

Pro Thr Tyr Lys Ala Phe Leu Thr Lys
1               5

<210>  360
<211>  9
<212>  PRT
<213>  Hepatitis B virus

<400>  360

His Leu Tyr Pro Val Ala Arg Gln Arg
1               5

<210>  361
<211>  9
<212>  PRT
<213>  Hepatitis B virus

<400>  361

Ser Thr Asn Arg Gln Leu Gly Arg Lys
1               5

<210>  362
<211>  9
<212>  PRT
<213>  Hepatitis B virus

<400>  362

Ala Leu Arg Phe Thr Ser Ala Arg Arg
1               5

<210>  363
<211>  9
<212>  PRT
<213>  Hepatitis B virus

<400>  363

Pro Val Asn Arg Pro Ile Asp Trp Lys
1               5

<210>  364
<211>  9
<212>  PRT
<213>  Hepatitis B virus

<400>  364

Thr Val Asn Glu Asn Arg Arg Leu Lys
1               5

<210>  365
<211>  9
<212>  PRT
<213>  Hepatitis B virus

<400>  365

Val Val Asn His Tyr Phe Gln Thr Arg
1               5

<210>  366
<211>  9
<212>  PRT
<213>  Hepatitis B virus

<400>  366

Ser Thr Thr Ser Thr Gly Pro Cys Lys
1               5

<210>  367
<211>  9
<212>  PRT
<213>  Hepatitis B virus

<400>  367

Gln Val Leu Pro Lys Leu Leu His Lys
1               5

<210>  368
<211>  9
<212>  PRT
<213>  Hepatitis B virus

<400>  368

Leu Thr Lys Tyr Leu Pro Leu Asp Lys
1               5

<210>  369
<211>  9
<212>  PRT
<213>  Hepatitis B virus

<400>  369

Cys Leu His Gln Ser Ala Val Arg Lys
1               5

<210>  370
<211>  9
<212>  PRT
<213>  Hepatitis B virus

<400>  370

Val Val Asp Phe Ser Gln Phe Ser Arg
1               5

<210>  371
<211>  9
<212>  PRT
<213>  Hepatitis B virus

<400>  371

Pro Leu Tyr Ala Cys Ile Gln Ala Lys
1               5

<210>  372
<211>  9
<212>  PRT
<213>  Hepatitis B virus

<400>  372

Tyr Val Asn Thr Asn Met Gly Leu Lys
1               5

<210>  373
<211>  9
<212>  PRT
<213>  Hepatitis B virus

<400>  373

Pro Leu Tyr Ala Cys Ile Gln Ser Lys
1               5

<210>  374
<211>  9
<212>  PRT
<213>  Hepatitis B virus

<400>  374

Arg Leu Ala Asp Glu Gly Leu Asn Arg
1               5

<210>  375
<211>  9
<212>  PRT
<213>  Hepatitis B virus

<400>  375

Ala Val Asn His Tyr Phe Lys Thr Arg
1               5

<210>  376
<211>  9
<212>  PRT
<213>  Hepatitis B virus

<400>  376

Arg Leu Lys Leu Ile Met Pro Ala Arg
1               5

<210>  377
<211>  9
<212>  PRT
<213>  Hepatitis B virus

<400>  377

Ile Leu Tyr Lys Arg Glu Thr Thr Arg
1               5

<210>  378
<211>  9
<212>  PRT
<213>  Hepatitis B virus

<400>  378

Lys Val Phe Val Leu Gly Gly Cys Arg
1               5

<210>  379
<211>  9
<212>  PRT
<213>  Hepatitis B virus

<400>  379

Asn Val Ser Ile Pro Trp Thr His Lys
1               5

<210>  380
<211>  9
<212>  PRT
<213>  Hepatitis B virus

<400>  380

Leu Leu Leu Tyr Lys Thr Phe Gly Arg
1               5

<210>  381
<211>  9
<212>  PRT
<213>  Hepatitis B virus

<400>  381

Arg Leu Val Phe Gln Thr Ser Thr Arg
1               5

<210>  382
<211>  9
<212>  PRT
<213>  Hepatitis B virus

<400>  382

Phe Val Leu Gly Gly Cys Arg His Lys
1               5

<210>  383
<211>  9
<212>  PRT
<213>  Hepatitis B virus

<400>  383

Arg Leu Val Leu Gln Thr Ser Thr Arg
1               5

<210>  384
<211>  9
<212>  PRT
<213>  Hepatitis B virus

<400>  384

Thr Val Asn Glu Lys Arg Arg Leu Lys
1               5

<210>  385
<211>  9
<212>  PRT
<213>  Hepatitis B virus

<400>  385

Thr Val Asn Glu Lys Arg Arg Leu Lys
1               5

<210>  386
<211>  9
<212>  PRT
<213>  Hepatitis B virus

<400>  386

Asn Leu Tyr Pro Val Ala Arg Gln Arg
1               5

<210>  387
<211>  9
<212>  PRT
<213>  Hepatitis B virus

<400>  387

Leu Pro Tyr Arg Pro Thr Thr Gly Arg
1               5

<210> 388
<211> 9
<212> PRT
<213> Hepatitis B virus

<400> 388

Leu Val Ser Phe Gly Val Trp Ile Arg
1               5

<210> 389
<211> 9
<212> PRT
<213> Hepatitis B virus

<400> 389

Leu Val Gly Ser Ser Gly Leu Pro Arg
1               5

<210> 390
<211> 9
<212> PRT
<213> Hepatitis B virus

<400> 390

His Ile Ser Cys Leu Thr Phe Gly Arg
1               5

<210> 391
<211> 9
<212> PRT
<213> Hepatitis B virus

<400> 391

Ser Val Pro Ser Arg Leu Pro Asp Arg
1               5

<210>  392
<211>  9
<212>  PRT
<213>  Hepatitis B virus

<400>  392

Ser Val Pro Ser His Leu Pro Asp Arg
1               5

<210>  393
<211>  10
<212>  PRT
<213>  Hepatitis B virus

<400>  393

Thr Leu Pro Gln Glu His Ile Val Leu Lys
1               5                   10

<210>  394
<211>  10
<212>  PRT
<213>  Hepatitis B virus

<400>  394

Thr Val Pro Val Phe Asn Pro His Trp Lys
1               5                   10

<210>  395
<211>  10
<212>  PRT
<213>  Hepatitis B virus

<400>  395

Thr Leu Trp Lys Ala Gly Ile Leu Tyr Lys
1               5                   10

<210>  396
<211>  10
<212>  PRT
<213>  Hepatitis B virus

<400>  396

Ser Met Tyr Pro Ser Cys Cys Cys Thr Lys
1               5                   10

<210>  397
<211>  10
<212>  PRT
<213>  Hepatitis B virus

<400>  397

Arg Leu Pro Tyr Arg Pro Thr Thr Gly Arg
1               5                   10

<210>  398
<211>  10
<212>  PRT
<213>  Hepatitis B virus

<400>  398

Ser Thr Thr Asp Leu Glu Ala Tyr Phe Lys
1               5                   10

<210>  399
<211>  10
<212>  PRT
<213>  Hepatitis B virus

<400>  399

Leu Leu Leu Tyr Lys Thr Phe Gly Arg Lys
1               5                   10

<210>  400
<211>  10
<212>  PRT
<213>  Hepatitis B virus

<400>  400

Thr Val Asn Ala His Arg Asn Leu Pro Lys
1               5                   10

<210>  401
<211>  10
<212>  PRT
<213>  Hepatitis B virus

<400>  401

Glu Ala Tyr Phe Lys Asp Cys Leu Phe Lys
1               5                   10

<210>  402
<211>  10
<212>  PRT
<213>  Hepatitis B virus

<400>  402

Leu Val Val Asp Phe Ser Gln Phe Ser Arg
1               5                   10

<210>  403
<211>  10
<212>  PRT
<213>  Hepatitis B virus

<400>  403

Met Leu Leu Tyr Lys Thr Tyr Gly Arg Lys
1               5                   10

<210>  404
<211>  10
<212>  PRT
<213>  Hepatitis B virus

<400>  404

Thr Ala Tyr Ser His Leu Ser Thr Ser Lys
1               5                   10

<210>  405
<211>  10
<212>  PRT
<213>  Hepatitis B virus

<400>  405

Ser Leu Gly Ile His Leu Asn Pro Asn Lys
1               5                   10

<210>  406
<211>  10
<212>  PRT
<213>  Hepatitis B virus

<400>  406

Arg Leu Gly Leu Tyr Arg Pro Leu Leu Arg
1               5                   10

<210>  407
<211>  10
<212>  PRT
<213>  Hepatitis B virus

<400>  407

Val Thr Gly Gly Val Phe Leu Val Asp Lys
1               5                   10

<210>  408
<211>  10
<212>  PRT
<213>  Hepatitis B virus

<400>  408

Arg Ile Arg Thr Pro Arg Thr Pro Ala Arg
1               5                   10


<210>  409
<211>  10
<212>  PRT
<213>  Hepatitis B virus

<400>  409

Thr Val Asn Gly His Gln Val Leu Pro Lys
1               5                   10


<210>  410
<211>  10
<212>  PRT
<213>  Hepatitis B virus

<400>  410

Ser Leu Pro Phe Gln Pro Thr Thr Gly Arg
1               5                   10


<210>  411
<211>  10
<212>  PRT
<213>  Hepatitis B virus

<400>  411

Thr Leu Pro Glu Thr Thr Val Val Arg Arg
1               5                   10

<210>  412
<211>  10
<212>  PRT
<213>  Hepatitis B virus

<400>  412

Gly Thr Asp Asn Ser Val Val Leu Ser Arg
1               5                   10

<210>  413
<211>  10
<212>  PRT
<213>  Hepatitis B virus

<400>  413

Ser Thr Leu Pro Glu Thr Thr Val Val Arg
1               5                   10

<210>  414
<211>  10
<212>  PRT
<213>  Hepatitis B virus

<400>  414

Lys Val Thr Lys Tyr Leu Pro Leu Asp Lys
1               5                   10

<210>  415
<211>  10
<212>  PRT
<213>  Hepatitis B virus

<400>  415

Ser Thr Arg His Gly Asp Lys Ser Phe Arg
1               5                   10

<210>  416
<211>  10
<212>  PRT
<213>  Hepatitis B virus

<400>  416

Val Leu Ser Cys Trp Trp Leu Gln Phe Arg
1               5                   10

<210>  417
<211>  10
<212>  PRT
<213>  Hepatitis B virus

<400>  417

Asn Val Thr Lys Tyr Leu Pro Leu Asp Lys
1               5                   10

<210>  418
<211>  10
<212>  PRT
<213>  Hepatitis B virus

<400>  418

Arg Val Cys Cys Gln Leu Asp Pro Ala Arg
1               5                   10

<210>  419
<211>  10
<212>  PRT
<213>  Hepatitis B virus

<400>  419

Ser Leu Gly Ile His Leu Asn Pro Gln Lys
1               5                   10

<210>  420
<211>  10
<212>  PRT
<213>  Hepatitis B virus

<400>  420

Tyr Leu Val Ser Phe Gly Val Trp Ile Arg
1               5                   10

<210>  421
<211>  10
<212>  PRT
<213>  Hepatitis B virus

<400>  421

Phe Val Gly Pro Leu Thr Val Asn Glu Lys
1               5                   10

<210>  422
<211>  10
<212>  PRT
<213>  Hepatitis B virus

<400>  422

Tyr Val Gly Pro Leu Thr Val Asn Glu Lys
1               5                   10

<210>  423
<211>  10
<212>  PRT
<213>  Hepatitis B virus

<400>  423

Arg Leu Ala Asp Glu Gly Leu Asn Arg Arg
1               5                   10

<210>  424
<211>  10
<212>  PRT
<213>  Hepatitis B virus

<400>  424

Ile Val Leu Lys Leu Lys Gln Cys Phe Arg
1               5                   10

<210>  425
<211>  10
<212>  PRT
<213>  Hepatitis B virus

<400>  425

Pro Ile Pro Ser Ser Trp Ala Phe Ala Lys
1               5                   10

<210>  426
<211>  10
<212>  PRT
<213>  Hepatitis B virus

<400>  426

Leu Thr Val Asn Glu Asn Arg Arg Leu Lys
1               5                   10

<210>  427
<211>  9
<212>  PRT
<213>  Hepatitis C virus

<400>  427

Cys Thr Cys Gly Ser Ser Asp Leu Tyr
1               5

<210>  428
<211>  9
<212>  PRT
<213>  Hepatitis C virus

<400>  428

Asn Ile Val Asp Val Gln Tyr Leu Tyr
1               5

<210>  429
<211>  9
<212>  PRT
<213>  Hepatitis C virus

<400>  429

Val Gln Asp Cys Asn Cys Ser Ile Tyr
1               5

<210>  430
<211>  9
<212>  PRT
<213>  Hepatitis C virus

<400>  430

Leu Thr Pro Arg Cys Met Val Asp Tyr
1               5

<210>  431
<211>  9
<212>  PRT
<213>  Hepatitis C virus

<400>  431

Arg Val Cys Glu Lys Met Ala Leu Tyr
1               5

<210>  432
<211>  9
<212>  PRT
<213>  Hepatitis C virus

<400>  432

Asp Val Val Cys Cys Ser Met Ser Tyr
1               5

<210>  433
<211>  9
<212>  PRT
<213>  Hepatitis C virus

<400>  433

Phe Thr Ile Phe Lys Ile Arg Met Tyr
1               5

<210>  434
<211>  10
<212>  PRT
<213>  Hepatitis C virus

<400>  434

Gly Leu Ser Ala Phe Ser Leu His Ser Tyr
1               5                   10

<210>  435
<211>  10
<212>  PRT
<213>  Hepatitis C virus

<400>  435

Thr Leu His Gly Pro Thr Pro Leu Leu Tyr
1               5                   10

<210>  436
<211>  9
<212>  PRT
<213>  Hepatitis C virus

<400>  436

Glu Tyr Val Leu Leu Leu Phe Leu Leu
1               5

<210>  437
<211>  10
<212>  PRT
<213>  Hepatitis C virus

<400>  437

Met Tyr Val Gly Gly Val Glu His Arg Leu
1               5                   10

<210>  438
<211>  10
<212>  PRT
<213>  Hepatitis C virus

<400>  438

Glu Tyr Val Leu Leu Leu Phe Leu Leu Leu
1               5                   10

<210>  439
<211>  9
<212>  PRT
<213>  Hepatitis C virus

<400>  439

Ser Val Pro Ala Glu Ile Leu Arg Lys
1               5

<210> 440
<211> 9
<212> PRT
<213> Hepatitis C virus

<400> 440

Gln Leu Phe Thr Phe Ser Pro Arg Arg
1               5

<210> 441
<211> 9
<212> PRT
<213> Hepatitis C virus

<400> 441

Arg Leu Gly Val Arg Ala Thr Arg Lys
1               5

<210> 442
<211> 9
<212> PRT
<213> Hepatitis C virus

<400> 442

Leu Ile Phe Cys His Ser Lys Lys Lys
1               5

<210> 443
<211> 9
<212> PRT
<213> Hepatitis C virus

<400> 443

His Leu Ile Phe Cys His Ser Lys Lys
1               5

<210>  444
<211>  9
<212>  PRT
<213>  Hepatitis C virus

<400>  444

Lys Thr Ser Glu Arg Ser Gln Pro Arg
1               5

<210>  445
<211>  9
<212>  PRT
<213>  Hepatitis C virus

<400>  445

Ala Val Cys Thr Arg Gly Val Ala Lys
1               5

<210>  446
<211>  9
<212>  PRT
<213>  Hepatitis C virus

<400>  446

Glu Val Phe Cys Val Gln Pro Glu Lys
1               5

<210>  447
<211>  9
<212>  PRT
<213>  Hepatitis C virus

<400>  447

Ile Thr Arg Val Glu Ser Glu Asn Lys
1               5

<210>  448
<211>  9
<212>  PRT
<213>  Hepatitis C virus

<400>  448

Cys Ile Ile Thr Ser Leu Thr Gly Arg
1               5

<210>  449
<211>  10
<212>  PRT
<213>  Hepatitis C virus

<400>  449

Gly Val Ala Gly Ala Leu Val Ala Phe Lys
1               5                   10

<210>  450
<211>  10
<212>  PRT
<213>  Hepatitis C virus

<400>  450

His Leu His Ala Pro Thr Gly Ser Gly Lys
1               5                   10

<210>  451
<211>  10
<212>  PRT
<213>  Hepatitis C virus

<400>  451

Arg Met Tyr Val Gly Gly Val Glu His Arg
1               5                   10

<210> 452
<211> 10
<212> PRT
<213> Hepatitis C virus

<400> 452

His Leu Ile Phe Cys His Ser Lys Lys Lys
1               5                   10

<210> 453
<211> 10
<212> PRT
<213> Hepatitis C virus

<400> 453

Thr Leu Gly Phe Gly Ala Tyr Met Ser Lys
1               5                   10

<210> 454
<211> 10
<212> PRT
<213> Hepatitis C virus

<400> 454

Gly Val Gly Ile Tyr Leu Leu Pro Asn Arg
1               5                   10

<210> 455
<211> 10
<212> PRT
<213> Hepatitis C virus

<400> 455

Leu Leu Phe Leu Leu Leu Ala Asp Ala Arg
1               5                   10

<210>  456
<211>  9
<212>  PRT
<213>  Human immunodeficiency virus

<400>  456

Phe Arg Asp Tyr Val Asp Arg Phe Tyr
1               5

<210>  457
<211>  9
<212>  PRT
<213>  Human immunodeficiency virus

<400>  457

Ile Tyr Gln Tyr Met Asp Asp Leu Tyr
1               5

<210>  458
<211>  9
<212>  PRT
<213>  Human immunodeficiency virus

<400>  458

Thr Val Leu Asp Val Gly Asp Ala Tyr
1               5

<210>  459
<211>  10
<212>  PRT
<213>  Human immunodeficiency virus

<400>  459

Val Thr Val Leu Asp Val Gly Asp Ala Tyr
1               5                   10

<210>  460
<211>  10
<212>  PRT
<213>  Human immunodeficiency virus

<400>  460

Val Ile Tyr Gln Tyr Met Asp Asp Leu Tyr
1               5                   10

<210>  461
<211>  10
<212>  PRT
<213>  Human immunodeficiency virus

<400>  461

Glu Val Asn Ile Val Thr Asp Ser Gln Tyr
1               5                   10

<210>  462
<211>  10
<212>  PRT
<213>  Human immunodeficiency virus

<400>  462

Leu Val Ala Val His Val Ala Ser Gly Tyr
1               5                   10

<210>  463
<211>  10
<212>  PRT
<213>  Human immunodeficiency virus

<400>  463

Pro Ala Glu Thr Gly Gln Glu Thr Ala Tyr
1               5                   10

<210>  464
<211>  10
<212>  PRT
<213>  Human immunodeficiency virus

<400>  464

Ile Ser Lys Ile Gly Pro Glu Asn Pro Tyr
1               5                   10


<210>  465
<211>  10
<212>  PRT
<213>  Human immunodeficiency virus

<400>  465

Gln Met Ala Val Phe Ile His Asn Phe Lys
1               5                   10


<210>  466
<211>  9
<212>  PRT
<213>  Human immunodeficiency virus

<400>  466

Arg Tyr Leu Lys Asp Gln Gln Leu Leu
1               5


<210>  467
<211>  9
<212>  PRT
<213>  Human immunodeficiency virus

<400>  467

Thr Tyr Gln Ile Tyr Gln Glu Pro Phe
1               5

<210>  468
<211>  9
<212>  PRT
<213>  Human immunodeficiency virus

<400>  468

Ile Tyr Gln Glu Pro Phe Lys Asn Leu
1               5

<210>  469
<211>  9
<212>  PRT
<213>  Human immunodeficiency virus

<400>  469

Ile Tyr Gln Tyr Met Asp Asp Leu Tyr
1               5

<210>  470
<211>  10
<212>  PRT
<213>  Human immunodeficiency virus

<400>  470

Ile Tyr Lys Arg Trp Ile Ile Leu Gly Leu
1               5                   10

<210>  471
<211>  10
<212>  PRT
<213>  Human immunodeficiency virus

<400>  471

Leu Tyr Pro Leu Ala Ser Leu Arg Ser Leu
1               5                   10

<210> 472
<211> 9
<212> PRT
<213> Human immunodeficiency virus

<400> 472

Lys Leu Ala Gly Arg Trp Pro Val Lys
1               5

<210> 473
<211> 9
<212> PRT
<213> Human immunodeficiency virus

<400> 473

Ala Val Phe Ile His Asn Phe Lys Arg
1               5

<210> 474
<211> 9
<212> PRT
<213> Human immunodeficiency virus

<400> 474

Ala Ile Phe Gln Ser Ser Met Thr Lys
1               5

<210> 475
<211> 9
<212> PRT
<213> Human immunodeficiency virus

<400> 475

Ile Val Ile Trp Gly Lys Thr Pro Lys
1               5

<210>  476
<211>  9
<212>  PRT
<213>  Human immunodeficiency virus

<400>  476

Lys Leu Thr Glu Asp Arg Trp Asn Lys
1               5

<210>  477
<211>  9
<212>  PRT
<213>  Human immunodeficiency virus

<400>  477

Gln Ile Ile Glu Gln Leu Ile Lys Lys
1               5

<210>  478
<211>  9
<212>  PRT
<213>  Human immunodeficiency virus

<400>  478

Gln Ile Ile Glu Gln Leu Ile Lys Lys
1               5

<210>  479
<211>  9
<212>  PRT
<213>  Human immunodeficiency virus

<400>  479

Lys Ile Trp Pro Ser Tyr Lys Gly Arg
1               5

<210>  480
<211>  9
<212>  PRT
<213>  Human immunodeficiency virus

<400>  480

Ile Ile Ala Thr Asp Ile Gln Thr Lys
1               5

<210>  481
<211>  9
<212>  PRT
<213>  Human immunodeficiency virus

<400>  481

Met Gly Tyr Glu Leu His Pro Asp Lys
1               5

<210>  482
<211>  9
<212>  PRT
<213>  Human immunodeficiency virus

<400>  482

Tyr Leu Ala Trp Val Pro Ala His Lys
1               5

<210>  483
<211>  9
<212>  PRT
<213>  Human immunodeficiency virus

<400>  483

Lys Ile Trp Pro Ser His Lys Gly Arg
1               5

<210>  484
<211>  9
<212>  PRT
<213>  Human immunodeficiency virus

<400>  484

Phe Val Asn Thr Pro Pro Leu Val Lys
1               5

<210>  485
<211>  9
<212>  PRT
<213>  Human immunodeficiency virus

<400>  485

Asn Thr Pro Val Phe Ala Ile Lys Lys
1               5

<210>  486
<211>  9
<212>  PRT
<213>  Human immunodeficiency virus

<400>  486

Thr Val Gln Cys Thr His Gly Ile Lys
1               5

<210>  487
<211>  9
<212>  PRT
<213>  Human immunodeficiency virus

<400>  487

Ile Leu Asp Ile Arg Gln Gly Pro Lys
1               5

<210> 488
<211> 9
<212> PRT
<213> Human immunodeficiency virus

<400> 488

Arg Asp Tyr Val Asp Arg Phe Tyr Lys
1               5

<210> 489
<211> 9
<212> PRT
<213> Human immunodeficiency virus

<400> 489

Gly Ile Ile Gln Ala Gln Pro Asp Lys
1               5

<210> 490
<211> 9
<212> PRT
<213> Human immunodeficiency virus

<400> 490

Val Leu Phe Leu Asp Gly Ile Asp Lys
1               5

<210> 491
<211> 9
<212> PRT
<213> Human immunodeficiency virus

<400> 491

Leu Val Asp Phe Arg Glu Leu Asn Lys
1               5

<210> 492
<211> 9
<212> PRT
<213> Human immunodeficiency virus

<400> 492

Lys Val Val Pro Arg Arg Lys Ala Lys
1               5

<210> 493
<211> 9
<212> PRT
<213> Human immunodeficiency virus

<400> 493

Met Thr Lys Ile Leu Glu Pro Phe Arg
1               5

<210> 494
<211> 10
<212> PRT
<213> Human immunodeficiency virus

<400> 494

Thr Val Tyr Tyr Gly Val Pro Val Trp Lys
1               5                   10

<210> 495
<211> 10
<212> PRT
<213> Human immunodeficiency virus

<400> 495

Thr Val Gln Pro Ile Val Leu Pro Glu Lys
1               5                   10

<210>  496
<211>  10
<212>  PRT
<213>  Human immunodeficiency virus

<400>  496

Ala Val Phe Ile His Asn Phe Lys Arg Lys
1               5                   10


<210>  497
<211>  10
<212>  PRT
<213>  Human immunodeficiency virus

<400>  497

Lys Val Leu Phe Leu Asp Gly Ile Asp Lys
1               5                   10


<210>  498
<211>  10
<212>  PRT
<213>  Human immunodeficiency virus

<400>  498

Lys Leu Val Asp Phe Arg Glu Leu Asn Lys
1               5                   10


<210>  499
<211>  10
<212>  PRT
<213>  Human immunodeficiency virus

<400>  499

Lys Leu Lys Pro Gly Met Asp Gly Pro Lys
1               5                   10

<210> 500
<211> 10
<212> PRT
<213> Human immunodeficiency virus

<400> 500

Phe Leu Gly Lys Ile Trp Pro Ser Tyr Lys
1               5                   10

<210> 501
<211> 10
<212> PRT
<213> Human immunodeficiency virus

<400> 501

Lys Ile Gln Asn Phe Arg Val Tyr Tyr Arg
1               5                   10

<210> 502
<211> 10
<212> PRT
<213> Human immunodeficiency virus

<400> 502

Gly Ile Pro His Pro Ala Gly Leu Lys Lys
1               5                   10

<210> 503
<211> 10
<212> PRT
<213> Human immunodeficiency virus

<400> 503

Leu Val Lys Leu Trp Tyr Gln Leu Glu Lys
1               5                   10

<210>  504
<211>  10
<212>  PRT
<213>  Human immunodeficiency virus

<400>  504

Met Ile Gly Gly Ile Gly Gly Phe Ile Lys
1               5                   10

<210>  505
<211>  10
<212>  PRT
<213>  Human immunodeficiency virus

<400>  505

Met Thr Lys Ile Leu Glu Pro Phe Arg Lys
1               5                   10

<210>  506
<211>  10
<212>  PRT
<213>  Human immunodeficiency virus

<400>  506

Val Val Ile Gln Asp Asn Ser Asp Ile Lys
1               5                   10

<210>  507
<211>  10
<212>  PRT
<213>  Human immunodeficiency virus

<400>  507

Phe Leu Gly Lys Ile Trp Pro Ser His Lys
1               5                   10

<210>  508
<211>  10
<212>  PRT
<213>  Human immunodeficiency virus

<400>  508

Ile Val Gln Gln Gln Asn Asn Leu Leu Arg
1               5                   10

<210>  509
<211>  10
<212>  PRT
<213>  Human immunodeficiency virus

<400>  509

Phe Thr Thr Pro Asp Lys Lys His Gln Lys
1               5                   10

<210>  510
<211>  10
<212>  PRT
<213>  Human immunodeficiency virus

<400>  510

Leu Val Glu Ile Cys Thr Glu Met Glu Lys
1               5                   10

<210>  511
<211>  10
<212>  PRT
<213>  Human immunodeficiency virus

<400>  511

Leu Val Gln Asn Ala Asn Pro Asp Cys Lys
1               5                   10

<210>  512
<211>  9
<212>  PRT
<213>  Human papilloma virus

<400>  512

Ile Ser Glu Tyr Arg His Tyr Cys Tyr
1               5

<210>  513
<211>  9
<212>  PRT
<213>  Human papilloma virus

<400>  513

Gln Ala Glu Pro Asp Arg Ala His Tyr
1               5

<210>  514
<211>  10
<212>  PRT
<213>  Human papilloma virus

<400>  514

Leu Gln Asp Ile Glu Ile Thr Cys Val Tyr
1               5                   10

<210>  515
<211>  10
<212>  PRT
<213>  Human papilloma virus

<400>  515

Tyr Ser Lys Ile Ser Glu Tyr Arg His Tyr
1               5                   10

<210> 516
<211> 10
<212> PRT
<213> Human papilloma virus

<400> 516

His Gly Asp Thr Pro Thr Leu His Glu Tyr
1               5                   10


<210> 517
<211> 10
<212> PRT
<213> Human papilloma virus

<400> 517

Gln Pro Glu Thr Thr Asp Leu Tyr Cys Tyr
1               5                   10


<210> 518
<211> 10
<212> PRT
<213> Human papilloma virus

<400> 518

Ile His Asp Ile Ile Leu Glu Cys Val Tyr
1               5                   10


<210> 519
<211> 10
<212> PRT
<213> Human papilloma virus

<400> 519

Tyr Ser Arg Ile Arg Glu Leu Arg His Tyr
1               5                   10

<210>  520
<211>  10
<212>  PRT
<213>  Human papilloma virus

<400>  520

Tyr Ser Arg Ile Arg Glu Leu Arg His Tyr
1               5                   10

<210>  521
<211>  10
<212>  PRT
<213>  Human papilloma virus

<400>  521

Ala Val Cys Asp Lys Cys Leu Lys Phe Tyr
1               5                   10

<210>  522
<211>  10
<212>  PRT
<213>  Human papilloma virus

<400>  522

Leu Leu Ile Arg Cys Leu Arg Cys Gln Lys
1               5                   10

<210>  523
<211>  9
<212>  PRT
<213>  Human papilloma virus

<400>  523

His Thr Met Leu Cys Met Cys Cys Lys
1               5

<210>  524
<211>  9
<212>  PRT
<213>  Human papilloma virus

<400>  524

Val Tyr Cys Lys Thr Val Leu Glu Leu
1               5

<210>  525
<211>  9
<212>  PRT
<213>  Human papilloma virus

<400>  525

Cys Tyr Ser Leu Tyr Gly Thr Thr Leu
1               5

<210>  526
<211>  9
<212>  PRT
<213>  Human papilloma virus

<400>  526

Val Tyr Asp Phe Ala Phe Arg Asp Leu
1               5

<210>  527
<211>  9
<212>  PRT
<213>  Human papilloma virus

<400>  527

Leu Tyr Asn Leu Leu Ile Arg Cys Leu
1               5

<210>  528
<211>  9
<212>  PRT
<213>  Human papilloma virus

<400>  528

Val Tyr Gly Asp Thr Leu Glu Lys Leu
1               5

<210>  529
<211>  9
<212>  PRT
<213>  Human papilloma virus

<400>  529

Ser Val Tyr Gly Asp Thr Leu Glu Lys
1               5

<210>  530
<211>  9
<212>  PRT
<213>  Human papilloma virus

<400>  530

Thr Thr Leu Glu Gln Gln Tyr Asn Lys
1               5

<210>  531
<211>  9
<212>  PRT
<213>  Human papilloma virus

<400>  531

Ser Ile Pro His Ala Ala Cys His Lys
1               5

<210> 532
<211> 9
<212> PRT
<213> Human papilloma virus

<400> 532

Ile Val Cys Pro Ile Cys Ser Gln Lys
1               5

<210> 533
<211> 9
<212> PRT
<213> Human papilloma virus

<400> 533

Lys Leu Arg His Leu Asn Glu Lys Arg
1               5

<210> 534
<211> 9
<212> PRT
<213> Human papilloma virus

<400> 534

Leu Ile Arg Cys Leu Arg Cys Gln Lys
1               5

<210> 535
<211> 9
<212> PRT
<213> Human papilloma virus

<400> 535

Ile Ile Leu Glu Cys Val Tyr Cys Lys
1               5

<210>  536
<211>  9
<212>  PRT
<213>  Human papilloma virus

<400>  536

Cys Ile Asp Phe Tyr Ser Arg Ile Arg
1               5

<210>  537
<211>  10
<212>  PRT
<213>  Human papilloma virus

<400>  537

Gly Thr Thr Leu Glu Gln Gln Tyr Asn Lys
1               5                   10

<210>  538
<211>  10
<212>  PRT
<213>  Human papilloma virus

<400>  538

Leu Leu Ile Arg Cys Leu Arg Cys Gln Lys
1               5                   10

<210>  539
<211>  10
<212>  PRT
<213>  Human papilloma virus

<400>  539

Leu Leu Ile Arg Cys Ile Asn Cys Gln Lys
1               5                   10

<210> 540
<211> 10
<212> PRT
<213> Human papilloma virus

<400> 540

Leu Thr Glu Val Phe Glu Phe Ala Phe Lys
1               5                   10

<210> 541
<211> 10
<212> PRT
<213> Human papilloma virus

<400> 541

Gly Ile Val Cys Pro Ile Cys Ser Gln Lys
1               5                   10

<210> 542
<211> 10
<212> PRT
<213> Human papilloma virus

<400> 542

Asp Ile Ile Leu Glu Cys Val Tyr Cys Lys
1               5                   10

<210> 543
<211> 10
<212> PRT
<213> Human papilloma virus

<400> 543

Lys Leu Arg His Leu Asn Glu Lys Arg Arg
1               5                   10

<210>  544
<211>  10
<212>  PRT
<213>  Human papilloma virus

<400>  544

Cys Val Tyr Cys Lys Gln Gln Leu Leu Arg
1               5                   10

<210>  545
<211>  9
<212>  PRT
<213>  Homo sapiens

<400>  545

Glu Val Asp Pro Ile Gly His Leu Tyr
1               5

<210>  546
<211>  9
<212>  PRT
<213>  Homo sapiens

<400>  546

Glu Ala Asp Pro Thr Ser Asn Thr Tyr
1               5

<210>  547
<211>  9
<212>  PRT
<213>  Homo sapiens

<400>  547

Thr Gln Asp Leu Val Gln Glu Lys Tyr
1               5

<210>  548
<211>  9
<212>  PRT
<213>  Homo sapiens

<400>  548

Glu Val Asp Pro Ile Gly His Val Tyr
1               5

<210>  549
<211>  9
<212>  PRT
<213>  Homo sapiens

<400>  549

Glu Ala Asp Pro Thr Gly His Ser Tyr
1               5

<210>  550
<211>  9
<212>  PRT
<213>  Homo sapiens

<400>  550

Leu Val Gln Glu Lys Tyr Leu Glu Tyr
1               5

<210>  551
<211>  9
<212>  PRT
<213>  Homo sapiens

<400>  551

Thr Ser Tyr Val Lys Val Leu Glu Tyr
1               5

<210>  552
<211>  9
<212>  PRT
<213>  Homo sapiens

<400>  552

Ser Ser Leu Pro Thr Thr Met Asn Tyr
1               5


<210>  553
<211>  9
<212>  PRT
<213>  Homo sapiens

<400>  553

Gly Ser Val Val Gly Asn Trp Gln Tyr
1               5


<210>  554
<211>  9
<212>  PRT
<213>  Homo sapiens

<400>  554

Ser Ser Phe Ser Thr Thr Ile Asn Tyr
1               5


<210>  555
<211>  9
<212>  PRT
<213>  Homo sapiens

<400>  555

Met Leu Glu Ser Val Ile Lys Asn Tyr
1               5

<210>  556
<211>  10
<212>  PRT
<213>  Homo sapiens

<400>  556

Ala Ser Ser Leu Pro Thr Thr Met Asn Tyr
1               5                   10


<210>  557
<211>  10
<212>  PRT
<213>  Homo sapiens

<400>  557

Leu Thr Gln Asp Leu Val Gln Glu Lys Tyr
1               5                   10


<210>  558
<211>  10
<212>  PRT
<213>  Homo sapiens

<400>  558

Glu Thr Ser Tyr Val Lys Val Leu Glu Tyr
1               5                   10


<210>  559
<211>  10
<212>  PRT
<213>  Homo sapiens

<400>  559

Ala Ser Ser Phe Ser Thr Thr Ile Asn Tyr
1               5                   10

<210>  560
<211>  10
<212>  PRT
<213>  Homo sapiens

<400>  560

Asp Leu Val Gln Glu Lys Tyr Leu Glu Tyr
1               5                   10


<210>  561
<211>  9
<212>  PRT
<213>  Homo sapiens

<400>  561

Thr Ser Tyr Val Lys Val Leu Glu Tyr
1               5


<210>  562
<211>  9
<212>  PRT
<213>  Homo sapiens

<400>  562

Thr Thr Ile Asn Phe Thr Arg Gln Arg
1               5


<210>  563
<211>  9
<212>  PRT
<213>  Homo sapiens

<400>  563

Ala Leu Ala Glu Thr Ser Tyr Val Lys
1               5

<210>  564
<211>  9
<212>  PRT
<213>  Homo sapiens

<400>  564

Leu Thr Gln Asp Leu Val Gln Glu Lys
1               5

<210>  565
<211>  9
<212>  PRT
<213>  Homo sapiens

<400>  565

Leu Val Gln Glu Lys Tyr Leu Glu Tyr
1               5

<210>  566
<211>  9
<212>  PRT
<213>  Homo sapiens

<400>  566

His Ser Ala Tyr Gly Glu Pro Arg Lys
1               5

<210>  567
<211>  9
<212>  PRT
<213>  Homo sapiens

<400>  567

Leu Phe Arg Ala Val Ile Thr Lys Lys
1               5

<210> 568
<211> 10
<212> PRT
<213> Homo sapiens

<400> 568

Arg Val Arg Phe Phe Phe Pro Ser Leu Arg
1               5                   10

<210> 569
<211> 10
<212> PRT
<213> Homo sapiens

<400> 569

Ala Asp Leu Val Gly Phe Leu Leu Leu Lys
1               5                   10

<210> 570
<211> 10
<212> PRT
<213> Homo sapiens

<400> 570

Glu Ser Leu Phe Arg Ala Val Ile Thr Lys
1               5                   10

<210> 571
<211> 10
<212> PRT
<213> Homo sapiens

<400> 571

Asp Leu Val Gln Glu Lys Tyr Leu Glu Tyr
1               5                   10

<210>  572
<211>  10
<212>  PRT
<213>  Homo sapiens

<400>  572

Tyr Val Ile Lys Val Ser Ala Arg Val Arg
1               5                   10


<210>  573
<211>  10
<212>  PRT
<213>  Homo sapiens

<400>  573

Leu Ser Val Met Glu Val Tyr Asp Gly Arg
1               5                   10


<210>  574
<211>  10
<212>  PRT
<213>  Homo sapiens

<400>  574

Lys Ala Glu Met Leu Glu Ser Val Ile Lys
1               5                   10


<210>  575
<211>  10
<212>  PRT
<213>  Homo sapiens

<400>  575

Arg Ala Leu Ala Glu Thr Ser Tyr Val Lys
1               5                   10

<210>  576
<211>  9
<212>  PRT
<213>  Homo sapiens

<400>  576

Asn Tyr Pro Leu Trp Ser Gln Ser Tyr
1               5

<210>  577
<211>  10
<212>  PRT
<213>  Homo sapiens

<400>  577

Asn Tyr Lys His Cys Phe Pro Glu Ile Phe
1               5                   10

<210>  578
<211>  10
<212>  PRT
<213>  Homo sapiens

<400>  578

Leu Tyr Ile Phe Ala Thr Cys Leu Gly Leu
1               5                   10

<210>  579
<211>  10
<212>  PRT
<213>  Homo sapiens

<400>  579

Ser Tyr Val Lys Val Leu Glu Tyr Val Ile
1               5                   10

<210> 580
<211> 9
<212> PRT
<213> Homo sapiens

<400> 580

Ser Leu Phe Arg Ala Val Ile Thr Lys
1               5

<210> 581
<211> 9
<212> PRT
<213> Homo sapiens

<400> 581

Ser Val Met Glu Val Tyr Asp Gly Arg
1               5

<210> 582
<211> 9
<212> PRT
<213> Homo sapiens

<400> 582

Thr Thr Ile Asn Phe Thr Arg Gln Arg
1               5

<210> 583
<211> 9
<212> PRT
<213> Homo sapiens

<400> 583

Leu Thr Gln Asp Leu Val Gln Glu Lys
1               5

<210>  584
<211>  10
<212>  PRT
<213>  Homo sapiens

<400>  584

Ser Leu Phe Arg Ala Val Ile Thr Lys Lys
1               5                   10


<210>  585
<211>  10
<212>  PRT
<213>  Homo sapiens

<400>  585

Leu Leu Thr Gln Asp Leu Val Gln Glu Lys
1               5                   10


<210>  586
<211>  10
<212>  PRT
<213>  Homo sapiens

<400>  586

Met Leu Glu Ser Val Ile Lys Asn Tyr Lys
1               5                   10


<210>  587
<211>  10
<212>  PRT
<213>  Homo sapiens

<400>  587

Leu Leu Gly Asp Asn Gln Ile Met Pro Lys
1               5                   10

<210>  588
<211>  10
<212>  PRT
<213>  Homo sapiens

<400>  588

Ser Leu Glu Gln Arg Ser Leu His Cys Lys
1               5                   10



<210>  589
<211>  9
<212>  PRT
<213>  Homo sapiens

<400>  589

Gly Ser Asp Cys Thr Thr Ile His Tyr
1               5



<210>  590
<211>  10
<212>  PRT
<213>  Homo sapiens

<400>  590

Gly Thr Ala Lys Ser Val Thr Cys Thr Tyr
1               5                   10



<210>  591
<211>  10
<212>  PRT
<213>  Homo sapiens

<400>  591

Arg Val Glu Gly Asn Leu Arg Val Glu Tyr
1               5                   10

<210>  592
<211>  9
<212>  PRT
<213>  Homo sapiens

<400>  592

Arg Val Arg Ala Met Ala Ile Tyr Lys
1               5

<210>  593
<211>  9
<212>  PRT
<213>  Homo sapiens

<400>  593

Cys Thr Tyr Ser Pro Ala Leu Asn Lys
1               5

<210>  594
<211>  9
<212>  PRT
<213>  Homo sapiens

<400>  594

Asn Thr Ser Ser Ser Pro Gln Pro Lys
1               5

<210>  595
<211>  9
<212>  PRT
<213>  Homo sapiens

<400>  595

Arg Thr Glu Glu Glu Asn Leu Arg Lys
1               5

<210>  596
<211>  9
<212>  PRT
<213>  Homo sapiens

<400>  596

Glu Leu Asn Glu Ala Leu Glu Leu Lys
1               5

<210>  597
<211>  10
<212>  PRT
<213>  Homo sapiens

<400>  597

Arg Thr Glu Glu Glu Asn Leu Arg Lys Lys
1               5                   10

<210>  598
<211>  10
<212>  PRT
<213>  Homo sapiens

<400>  598

Lys Thr Tyr Gln Gly Ser Tyr Gly Phe Arg
1               5                   10

<210>  599
<211>  10
<212>  PRT
<213>  Homo sapiens

<400>  599

Val Val Arg Arg Cys Pro His His Glu Arg
1               5                   10

<210>  600
<211>  10
<212>  PRT
<213>  Homo sapiens

<400>  600

Asn Thr Ser Ser Ser Pro Gln Pro Lys Lys
1               5                   10


<210>  601
<211>  10
<212>  PRT
<213>  Homo sapiens

<400>  601

Arg Val Cys Ala Cys Pro Gly Arg Asp Arg
1               5                   10


<210>  602
<211>  10
<212>  PRT
<213>  Homo sapiens

<400>  602

Gly Leu Ala Pro Pro Gln His Leu Ile Arg
1               5                   10


<210>  603
<211>  9
<212>  PRT
<213>  Unknown

<220>
<221> source
<223> /note= "Description of unknown sequence: PAP"

<400>  603

Lys Gly Glu Tyr Phe Val Glu Met Tyr
1               5



<210>  604
<211>  9
<212>  PRT
<213>  Unknown

<220>
<221> source
<223> /note= "Description of unknown sequence: PAP"

<400>  604

Leu Gly Glu Tyr Ile Arg Lys Arg Tyr
1               5



<210>  605
<211>  9
<212>  PRT
<213>  Unknown

<220>
<221> source
<223> /note= "Description of unknown sequence: PAP"

<400>  605

Ala Ser Cys His Leu Thr Glu Leu Tyr
1               5



<210>  606
<211>  9
<212>  PRT
<213>  Unknown

<220>
<221> source
<223> /note= "Description of unknown sequence: PAP"

<400>  606

Glu Ser Tyr Lys His Glu Gln Val Tyr
1               5

<210>  607
<211>  10
<212>  PRT
<213>  Unknown

<220>
<221> source
<223> /note= "Description of unknown sequence: PAP"

<400>  607

Leu Ser Glu Leu Ser Leu Leu Ser Leu Tyr
1               5                   10

<210>  608
<211>  10
<212>  PRT
<213>  Unknown

<220>
<221> source
<223> /note= "Description of unknown sequence: PAP"

<400>  608

Leu Thr Gln Leu Gly Met Glu Gln His Tyr
1               5                   10

<210>  609
<211>  10
<212>  PRT
<213>  Unknown

<220>
<221> source
<223> /note= "Description of unknown sequence: PAP"

<400>  609

Lys Gly Glu Tyr Phe Val Glu Met Tyr Tyr
1               5                   10



<210>  610
<211>  10
<212>  PRT
<213>  Unknown

<220>
<221> source
<223> /note= "Description of unknown sequence: PAP"

<400>  610

Leu Val Asn Glu Ile Leu Asn His Met Lys
1               5                   10



<210>  611
<211>  9
<212>  PRT
<213>  Unknown

<220>
<221> source
<223> /note= "Description of unknown sequence: PAP"

<400>  611

Ala Thr Gln Ile Pro Ser Tyr Lys Lys
1               5

<210>  612
<211>  10
<212>  PRT
<213>  Unknown

<220>
<221> source
<223> /note= "Description of unknown sequence: PAP"

<400>  612

Glu Thr Leu Lys Ser Glu Glu Phe Gln Lys
1               5                   10



<210>  613
<211>  9
<212>  PRT
<213>  Unknown

<220>
<221> source
<223> /note= "Description of unknown sequence: PAP"

<400>  613

Leu Tyr Phe Glu Lys Gly Glu Tyr Phe
1               5



<210>  614
<211>  9
<212>  PRT
<213>  Unknown

<220>
<221> source
<223> /note= "Description of unknown sequence: PAP"

<400>  614

Leu Tyr Cys Glu Ser Val His Asn Phe
1               5

<210>  615
<211>  9
<212>  PRT
<213>  Unknown

<220>
<221> source
<223> /note= "Description of unknown sequence: PAP"

<400>  615

Pro Tyr Lys Asp Phe Ile Ala Thr Leu
1               5


<210>  616
<211>  9
<212>  PRT
<213>  Unknown

<220>
<221> source
<223> /note= "Description of unknown sequence: PAP"

<400>  616

Val Tyr Asn Gly Leu Leu Pro Pro Tyr
1               5


<210>  617
<211>  10
<212>  PRT
<213>  Unknown

<220>
<221> source
<223> /note= "Description of unknown sequence: PAP"

<400>  617

Pro Tyr Ala Ser Cys His Leu Thr Glu Leu
1               5                   10

<210> 618
<211> 9
<212> PRT
<213> Homo sapiens

<400> 618

Ala Leu Pro Glu Arg Pro Ser Leu Tyr
1               5

<210> 619
<211> 10
<212> PRT
<213> Homo sapiens

<400> 619

Val Ser His Ser Phe Pro His Pro Leu Tyr
1               5                   10

<210> 620
<211> 9
<212> PRT
<213> Homo sapiens

<400> 620

Pro Leu Tyr Asp Met Ser Leu Leu Lys
1               5

<210> 621
<211> 9
<212> PRT
<213> Homo sapiens

<400> 621

Val Val His Tyr Arg Lys Trp Ile Lys
1               5

<210>  622
<211>  9
<212>  PRT
<213>  Homo sapiens

<400>  622

Tyr Thr Lys Val Val His Tyr Arg Lys
1               5

<210>  623
<211>  9
<212>  PRT
<213>  Homo sapiens

<400>  623

Ser Leu Leu Lys Asn Arg Phe Leu Arg
1               5

<210>  624
<211>  9
<212>  PRT
<213>  Homo sapiens

<400>  624

Ile Val Gly Gly Trp Glu Cys Glu Lys
1               5

<210>  625
<211>  9
<212>  PRT
<213>  Homo sapiens

<400>  625

Gln Val His Pro Gln Lys Val Thr Lys
1               5

<210>  626
<211>  10
<212>  PRT
<213>  Homo sapiens

<400>  626

Ser Leu Tyr Thr Lys Val Val His Tyr Arg
1               5                   10


<210>  627
<211>  10
<212>  PRT
<213>  Homo sapiens

<400>  627

Leu Thr Ala Ala His Cys Ile Arg Asn Lys
1               5                   10


<210>  628
<211>  10
<212>  PRT
<213>  Homo sapiens

<400>  628

Arg Ile Val Gly Gly Trp Glu Cys Glu Lys
1               5                   10


<210>  629
<211>  10
<212>  PRT
<213>  Homo sapiens

<400>  629

Lys Val Val His Tyr Arg Lys Trp Ile Lys
1               5                   10

<210>  630
<211>  10
<212>  PRT
<213>  Homo sapiens

<400>  630

Val Thr Lys Phe Met Leu Cys Ala Gly Arg
1               5                   10


<210>  631
<211>  9
<212>  PRT
<213>  Homo sapiens

<400>  631

Met Leu Leu Arg Leu Ser Glu Pro Ala
1               5


<210>  632
<211>  9
<212>  PRT
<213>  Homo sapiens

<400>  632

Glu Val Asp Pro Ile Gly His Leu Tyr
1               5


<210>  633
<211>  10
<212>  PRT
<213>  Homo sapiens

<400>  633

Ala Ser Ser Leu Pro Thr Thr Met Asn Tyr
1               5                   10

<210>  634
<211>  9
<212>  PRT
<213>  Homo sapiens

<400>  634

Glu Ala Asp Pro Thr Gly His Ser Tyr
1               5

<210>  635
<211>  9
<212>  PRT
<213>  Homo sapiens

<400>  635

Ser Ser Leu Pro Thr Thr Met Asn Tyr
1               5

<210>  636
<211>  9
<212>  PRT
<213>  Homo sapiens

<400>  636

Gly Ser Val Val Gly Asn Trp Gln Tyr
1               5

<210>  637
<211>  9
<212>  PRT
<213>  Homo sapiens

<400>  637

Ala Leu Ala Glu Thr Ser Tyr Val Lys
1               5

<210>  638
<211>  9
<212>  PRT
<213>  Homo sapiens

<400>  638

Ser Leu Phe Arg Ala Val Ile Thr Lys
1               5

<210>  639
<211>  10
<212>  PRT
<213>  Homo sapiens

<400>  639

Arg Ala Leu Ala Glu Thr Ser Tyr Val Lys
1               5                   10

<210>  640
<211>  10
<212>  PRT
<213>  Homo sapiens

<400>  640

Glu Ser Leu Phe Arg Ala Val Ile Thr Lys
1               5                   10

<210>  641
<211>  11
<212>  PRT
<213>  Human immunodeficiency virus

<400>  641

Lys Val Tyr Leu Ala Trp Val Pro Ala His Lys
1               5                   10

<210>  642
<211>  10
<212>  PRT
<213>  Human immunodeficiency virus

<400>  642

Thr Val Tyr Tyr Gly Val Pro Val Trp Lys
1               5                   10

<210>  643
<211>  9
<212>  PRT
<213>  Human immunodeficiency virus

<400>  643

Lys Leu Ala Gly Arg Trp Pro Val Lys
1               5

<210>  644
<211>  11
<212>  PRT
<213>  Human immunodeficiency virus

<400>  644

Lys Met Ile Gly Gly Ile Gly Gly Phe Ile Lys
1               5                   10

<210>  645
<211>  9
<212>  PRT
<213>  Human immunodeficiency virus

<400>  645

Ala Ile Phe Gln Ser Ser Met Thr Lys
1               5

<210>  646
<211>  11
<212>  PRT
<213>  Human immunodeficiency virus

<400>  646

Trp Thr Tyr Gln Ile Tyr Gln Glu Pro Phe Lys
1               5                   10

<210>  647
<211>  10
<212>  PRT
<213>  Human immunodeficiency virus

<400>  647

Phe Leu Gly Lys Ile Trp Pro Ser His Lys
1               5                   10

<210>  648
<211>  10
<212>  PRT
<213>  Human immunodeficiency virus

<400>  648

Thr Val Tyr Tyr Gly Val Pro Val Trp Lys
1               5                   10

<210>  649
<211>  11
<212>  PRT
<213>  Human immunodeficiency virus

<400>  649

Val Thr Val Tyr Tyr Gly Val Pro Val Trp Lys
1               5                   10

<210>  650
<211>  10
<212>  PRT
<213>  Hepatitis C virus

<400>  650

Gly Val Ala Gly Ala Leu Val Ala Phe Lys
1               5                   10

<210>  651
<211>  9
<212>  PRT
<213>  Hepatitis C virus

<400>  651

Cys Thr Cys Gly Ser Ser Asp Leu Tyr
1               5

<210>  652
<211>  9
<212>  PRT
<213>  Hepatitis B virus

<400>  652

Leu Leu Asp Thr Ala Ser Ala Leu Tyr
1               5

<210>  653
<211>  10
<212>  PRT
<213>  Hepatitis B virus

<400>  653

Thr Leu Trp Lys Ala Gly Ile Leu Tyr Lys
1               5                   10

<210>  654
<211>  9
<212>  PRT
<213>  Hepatitis B virus

<400>  654

Phe Leu Leu Thr Arg Ile Leu Thr Ile
1               5

<210>  655
<211>  10
<212>  PRT
<213>  Hepatitis B virus

<400>  655

Phe Leu Pro Ser Asp Phe Phe Pro Ser Val
1               5                   10

<210>  656
<211>  9
<212>  PRT
<213>  Hepatitis B virus

<400>  656

Gly Leu Tyr Ser Ser Thr Val Pro Val
1               5

<210>  657
<211>  9
<212>  PRT
<213>  Homo sapiens

<400>  657

Glu Ala Asp Pro Thr Gly His Ser Tyr
1               5

<210>  658
<211>  9
<212>  PRT
<213>  Homo sapiens

<400>  658

Val Leu Val His Pro Gln Trp Val Leu
1               5


<210>  659
<211>  10
<212>  PRT
<213>  Hepatitis B virus

<400>  659

Phe Leu Pro Ser Asp Tyr Phe Pro Ser Val
1               5                   10


<210>  660
<211>  9
<212>  PRT
<213>  Unknown

<220>
<221> source
<223> /note= "Description of unknown sequence: PAP"

<400>  660

Ile Leu Leu Trp Asp Pro Ile Pro Val
1               5

<210>  661
<211>  10
<212>  PRT
<213>  Homo sapiens

<400>  661

Lys Leu Gln Cys Val Asp Leu Val His Ile
1               5                   10


<210>  662
<211>  11
<212>  PRT
<213>  Homo sapiens

<400>  662

Met Leu Leu Arg Leu Ser Glu Pro Ala Glu Leu
1               5                   10

## Description

The present application is a continuation in part of USSN 08/027,746 which is a continuation in part of USSN 07/926,666, which are incorporated herein by reference.

### BACKGROUND OF THE INVENTION

The present invention relates to compositions and methods for preventing, treating or diagnosing a number of pathological states such as viral diseases and cancers. In particular, it provides novel peptides capable of binding selected major histocompatibility complex (MHC) molecules and inducing an immune response.

MHC molecules are classified as either Class I or Class II molecules. Class II MHC molecules are expressed primarily on cells involved in initiating and sustaining immune responses, such as T lymphocytes, B lymphocytes, macrophages, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral infections. The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit β2 microglobulin. The peptide-MHC class I complex is then routed to the cell surface for expression and potential recognition by specific CTLs.

Investigations of the crystal structure of the human MHC class I molecule, HLA-A2.1, indicate that a peptide binding groove is created by the folding of the α1 and α2 domains of the class I heavy chain (Bjorkman et al., Nature 329:506 (1987). In these investigations, however, the identity of peptides bound to the groove was not determined.

Buus et al., Science 242:1065 (1988) first described a method for acid elution of bound peptides from MHC. Subsequently, Rammensee and his coworkers (Falk et al., Nature 351:290 (1991) have developed an approach to characterize naturally processed peptides bound to class I molecules. Other investigators have successfully achieved direct amino acid sequencing of the more abundant peptides in various HPLC fractions by conventional automated sequencing of peptides eluted from class I molecules of the B type (Jardetzky, et al., Nature 353:326 (1991) and of the A2.1 type by mass spectrometry (Hunt, et al., Science 225:1261 (1992). A review of the characterization of naturally processed peptides in MHC Class I has been presented by Rötzschke and Falk (Rötzschke and Falk, Immunol. Today 12:447 (1991).

Sette et al., Proc. Natl. Acad. Sci. USA 86:3296 (1989) showed that MHC allele specific motifs could be used to predict MHC binding capacity. Schaeffer et al., Proc. Natl. Acad. Sci. USA 86:4649 (1989) showed that MHC binding was related to immunogenicity. Several authors (De Bruijn et al., Eur. J. Immunol., 21:2963-2970 (1991); Pamer et al., 991 Nature 353:852-955 (1991)) have provided preliminary evidence that class I binding motifs can be applied to the identification of potential immunogenic peptides in animal models. Class I motifs specific for a number of human alleles of a given class I isotype have yet to be described. It is desirable that the combined frequencies of these different alleles should be high enough to cover a large fraction or perhaps the majority of the human outbred population.

Despite the developments in the art, the prior art has yet to provide a useful human peptide-based vaccine or therapeutic agent based on this work. The present invention provides these and other advantages.

### SUMMARY OF THE INVENTION

The present invention provides compositions comprising immunogenic peptides having binding motifs for MHC Class I molecules. The immunogenic peptides are typically between about 8 and about 11 residues and comprise conserved residues involved in binding proteins encoded by the appropriate MHC allele. A number of allele specific motifs have been identified.

For instance, the motif for HLA-A3.2 comprises from the N-terminus to C-terminus a first conserved residue of L, M, I, V, S, A, T and F at position 2 and a second conserved residue of K, R or Y at the C-terminal end. Other first conserved residues are C, G or D and alternatively E. Other second conserved residues are H or F. The first and second conserved residues are preferably separated by 6 to 7 residues.

The motif for HLA-A1 comprises from the N-terminus to the C-terminus a first conserved residue of T, S or M, a second conserved residue of D or E, and a third conserved residue of Y. Other second conserved residues are A, S or T. The first and second conserved residues are adjacent and are preferably separated from the third conserved residue by 6 to 7 residues. A second motif consists of a first conserved residue of E or D and a second conserved residue of Y where the first and second conserved residues are separated by 5 to 6 residues.

The motif for HLA-A11 comprises from the N-terminus to the C-terminus a first conserved residue of T or V at position 2 and a C-terminal conserved residue of K. The first and second conserved residues are preferably separated by 6 or 7 residues.

The motif for HLA-A24.1 comprises from the N-terminus to the C-terminus a first conserved residue of Y, F or W at position 2 and a C terminal conserved residue of F, I, W, M or L. The first and second conserved residues are preferably separated by 6 to 7 residues.

Epitopes on a number of potential target proteins can be identified in this manner. Examples of suitable antigens include prostate specific antigen (PSA), hepatitis B core and surface antigens (HBVc, HBVs) hepatitis C antigens, malignant melanoma antigen (MAGE-1) Epstein-Barr virus antigens, human immunodeficiency type-1 virus (HIV1) and papilloma virus antigens. The peptides are thus useful in pharmaceutical compositions for both in vivo and ex vivo therapeutic and diagnostic applications.

### Definitions

The term "peptide" is used interchangeably with "oligopeptide" in the present specification to designate a series of residues, typically L-amino.acids, connected one to the other typically by peptide bonds between the alpha-amino and carbonyl groups of adjacent amino acids. The oligopeptides of the invention are less than about 15 residues in length and usually consist of between about 8 and about 11 residues, preferably 9 or 10 residues.

An "immunogenic peptide" is a peptide which comprises an allele-specific motif such that the peptide will bind the MHC allele and be capable of inducing a CTL response. Thus, immunogenic peptides are capable of binding to an appropriate class I MHC molecule and inducing a cytotoxic T cell response against the antigen from which the immunogenic peptide is derived.

A "conserved residue" is an amino acid which occurs in a significantly higher frequency than would be expected by random distribution at a particular position in a peptide motif. Typically a conserved residue is one at which the immunogenic peptide may provide a contact point with the MHC molecule. One to three, preferably two, conserved residues within a peptide of defined length defines a motif for an immunogenic peptide. These residues are typically in close contact with the peptide binding groove, with their side chains buried in specific pockets of the groove itself. Typically, an immunogenic peptide will comprise up to three conserved residues, more usually two conserved residues.

As used herein, "negative binding residues" are amino acids which if present at certain positions will result in a peptide being a nonbinder or poor binder and in turn fail to induce a CTL response despite the presence of the appropriate conserved residues within the peptide.

The term "motif" refers to the pattern of residues in a peptide of defined length, usually about 8 to about 11 amino acids, which is recognized by a particular MHC allele. The peptide motifs are typically different for each human MHC allele and differ in the pattern of the highly conserved residues.

The binding motif for an allele can be defined with increasing degrees of precision. In one case, all of the conserved residues are present in the correct positions in a peptide and there are no negative binding residues present.

The phrases "isolated" or "biologically pure" refer to material which is substantially or essentially free from components which normally accompany it as found in its native state. Thus, the peptides of this invention do not contain materials normally associated with their in situ environment, e.g., MHC I molecules on antigen presenting cells. Even where a protein has been isolated to a homogenous or dominant band, there are trace contaminants in the range of 5-10% of native protein which co-purify with the desired protein. Isolated peptides of this invention do not contain such endogenous co-purified protein.

The term "residue" refers to an amino acid or amino acid mimetic incorporated in a oligopeptide by an amide bond or amide bond mimetic.

### BRIEF DESCRIPTION OF THE DRAWINGS

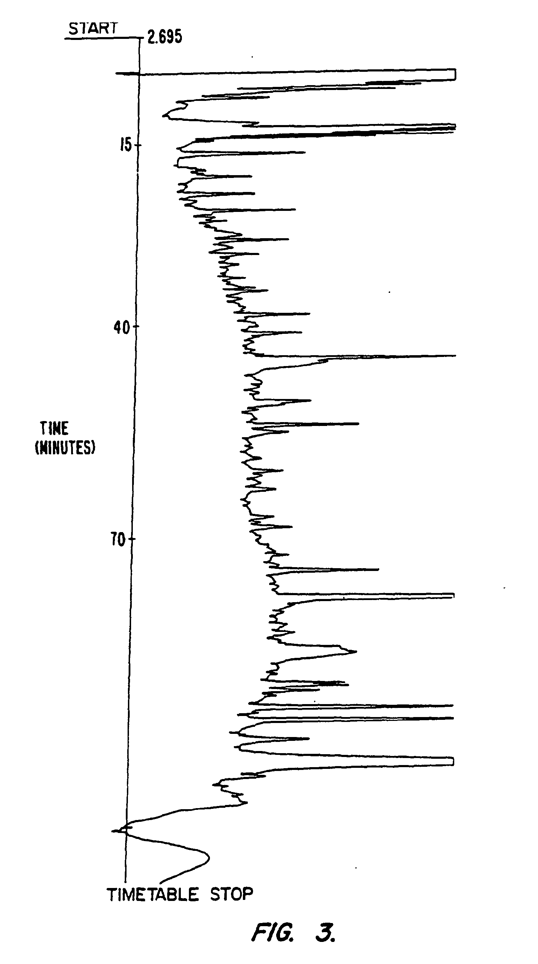

Fig. 1 is a flow diagram of an HLA-A purification scheme.
Fig. 2 is an SDS-PAGE analysis of affinity purified. HLA-A3.2 from the cell line EHM using an affinity column prepared with the mAb GAP A3 coupled to protein A-Sepharose.
   Lane 1 - Molecular weight standards.
   Lane 2 - A3.2 acid eluate
   Lane 3 - A3.2 a second acid eluate
   Lane 4 - Base elution #1
   Lane 5 - Base elution #2
   Lane 6 - Concentrated base elution 1
   Lane 7 - Concentrated base elution 2
   Lane 8 - BSA - 10 µg
   Lane 9 - BSA - 3 *µ*g
   Lane 10 - BSA - 1 *µ*g
Fig. 3 shows reverse phase high performance liquid' chromatography (RP-HPLC) separation of HLA-A3 acid eluted peptides.

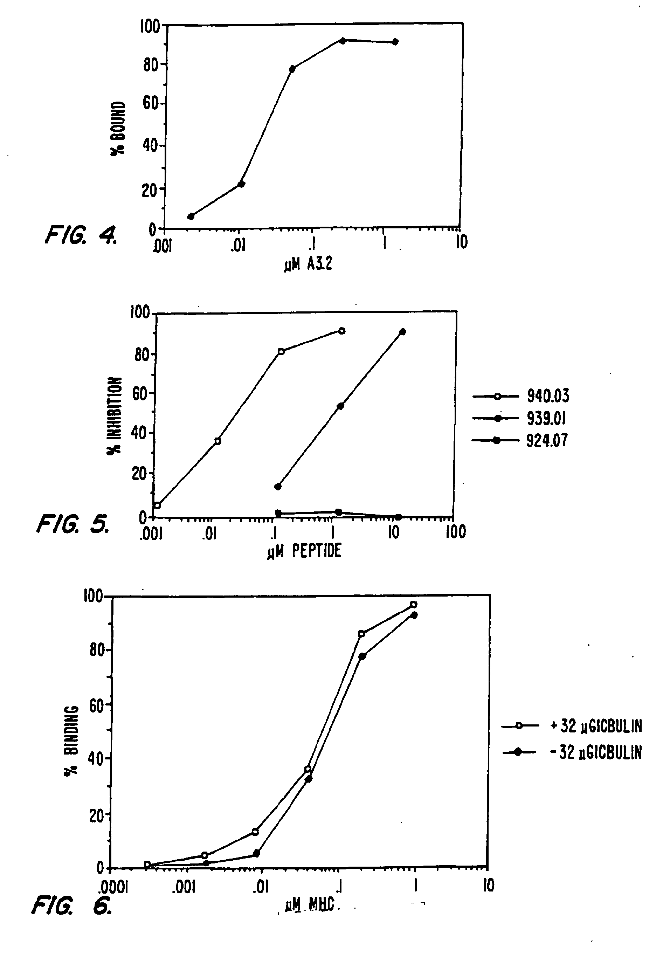

Fig. 4 shows binding of a radioactively labeled peptide of the invention to MHC molecules as measured by the % bound radioactivity.
Fig. 5 shows inhibition of binding of a peptide of the invention to MHC molecules in the presence of three peptides [HBc 18-27 (924.07), a Prostate Specific Antigen peptide (939.01), and HIV nef 73-82 (940.03)].

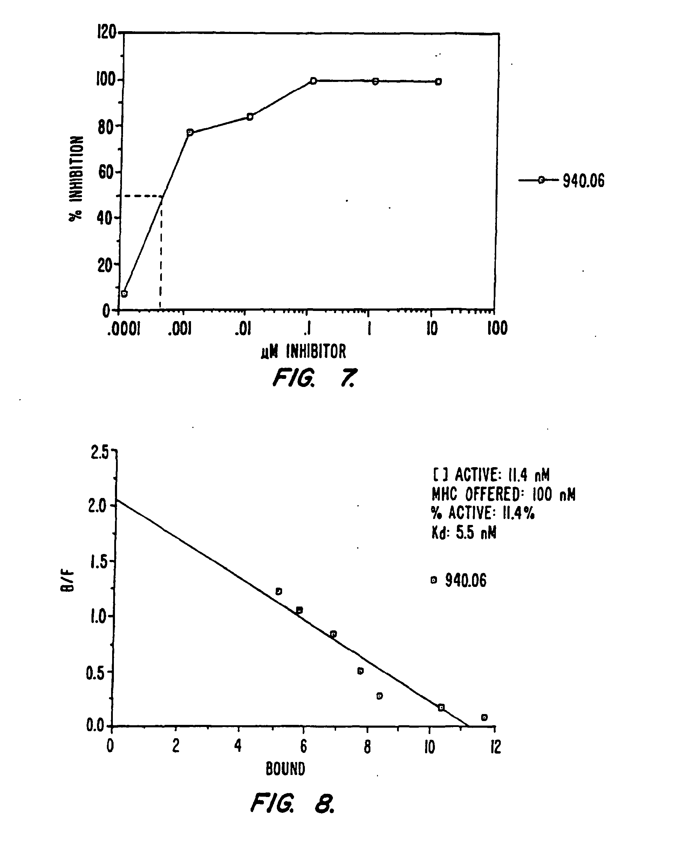

Fig. 6 shows the dependency of the binding on MHC concentration in the presence or absence of β₂ microglobulin.
Fig. 7 shows close dependent inhibition of binding with the addition of unlabeled peptide.

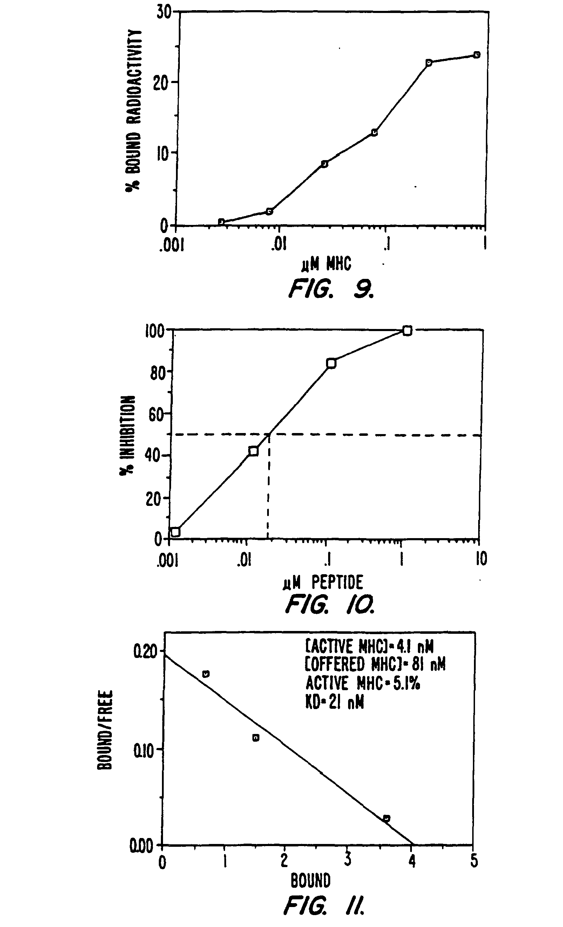

Fig. 8 Scatchard Analysis of binding to MHC A11 confirming an apparent K_{D} of 6nM.
Fig. 9 shows the binding of a radioactively labeled peptide of the invention to MHC A1 as measured by % bound reactivity.

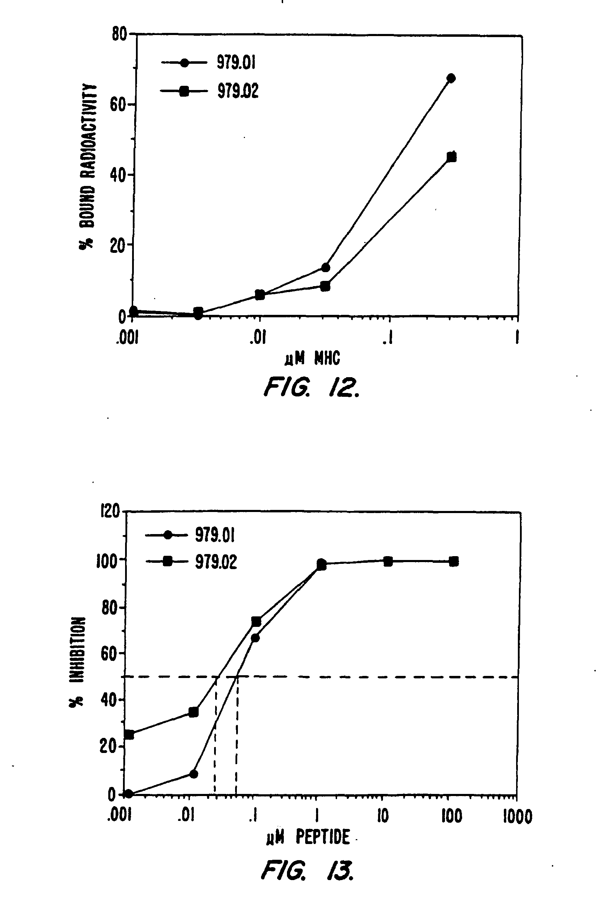

Fig. 10 shows dose dependent inhibition of binding with the addition of unlabeled peptide.
Fig. 11 Scatchard Analysis of binding to MHC A1 confirming an apparent K_{D} of 21nM.
Fig. 12. shows the binding of two peptides of this invention as a function of MHC A24 concentration as measured by % bound reactivity.
Fig. 13 shows the dose dependent inhibition of binding to MHC A24 with the addition of unlabeled peptides.

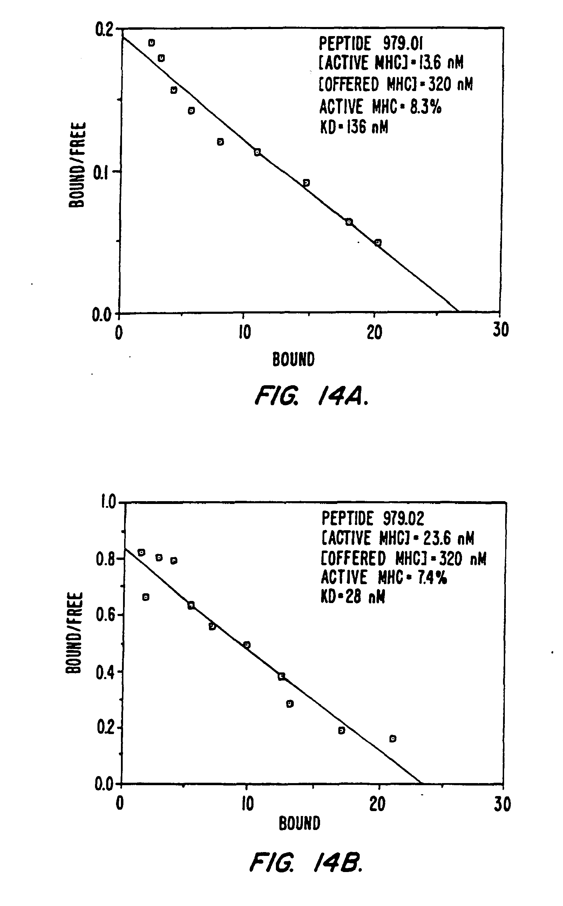

Figs. 14(a) and 14(b) show the Scatchard Analysis of binding to MHC A24 of the two peptides confirming a K_{D} of 30 and 60nM, respectively.

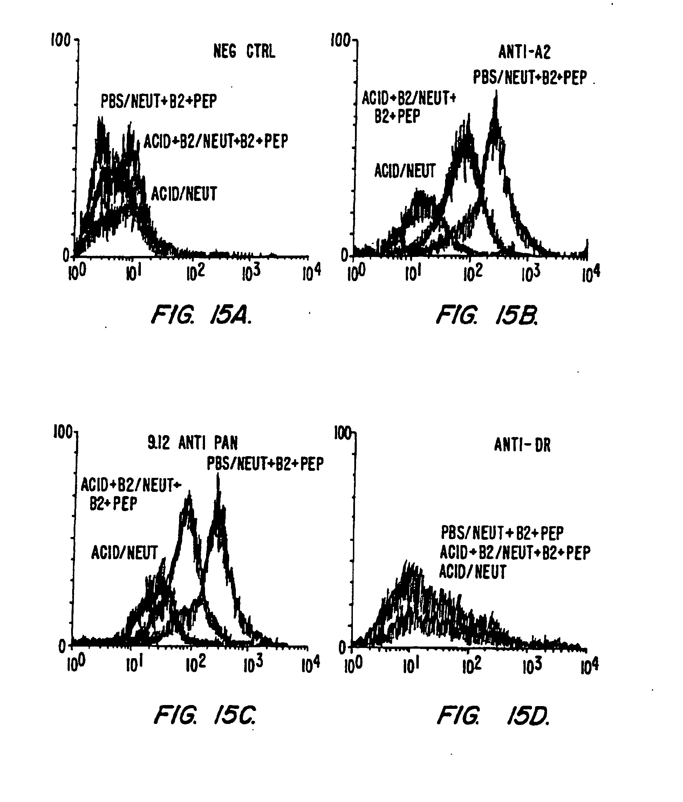

Fig. 15 shows the effect on MHC class 1 molecules of β₂microglobulin and a peptide of choice on acid-stripped PHA blasts.

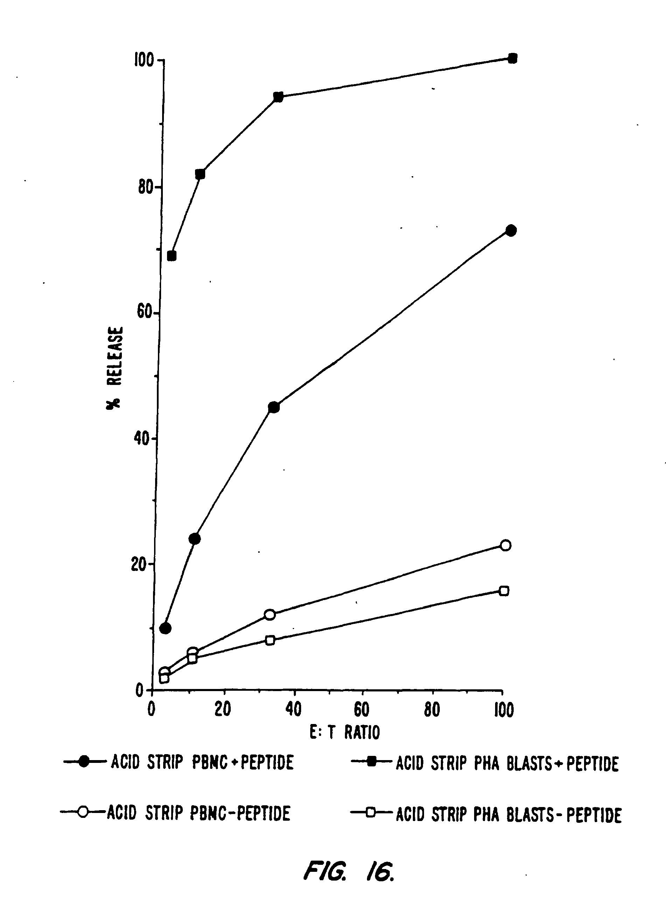

Fig. 16 shows CTL induction using GC43 A2.1 responders and autologous acid-stripped PBMCs or PHA blasts loaded with the 777.03-924.07-927.32 peptide pool.

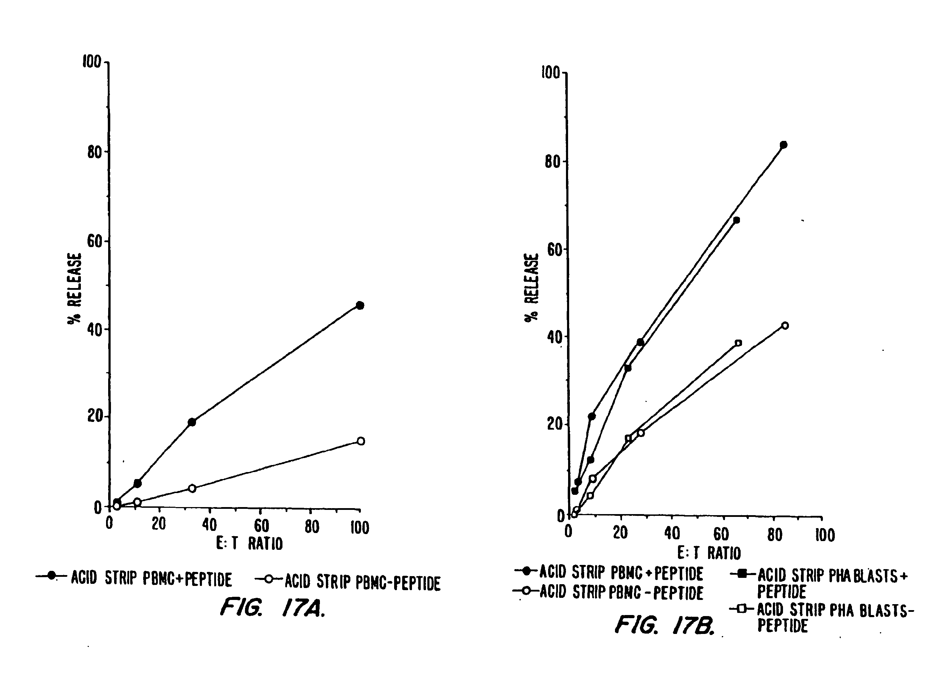

Fig. 17 shows CTL induction using X351 or X355 A2.1 responders and autologous acid stripped PBMCs or PHA blasts as stimulators after loading with the 1044.04-1044.05-1044.06 peptide pool.

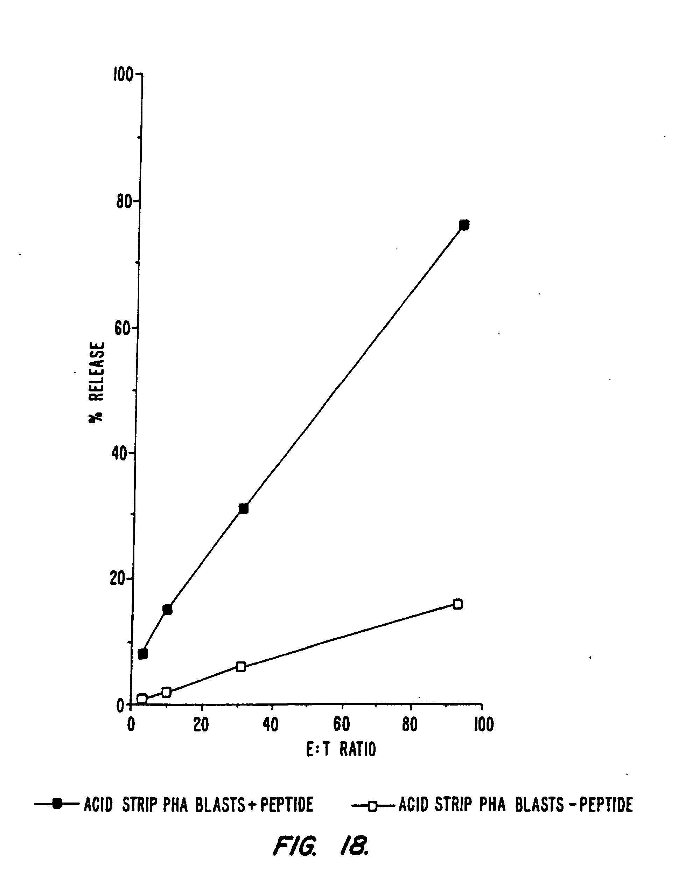

Fig. 18 shows CTL induction using GC49 A2.1 responders and Autologous Acid stripped PHA blasts as stimulators after loading with 939.03 peptide.

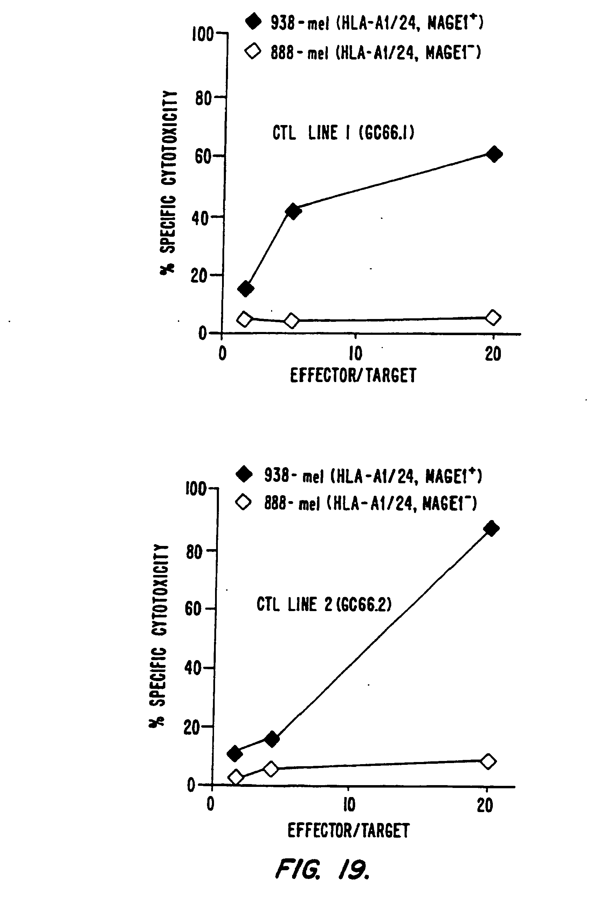

Fig. 19 shows CTL induction using GC66 A1 responders and autologous acid stripped PBMCs as stimulators after loading of peptide 938.01.

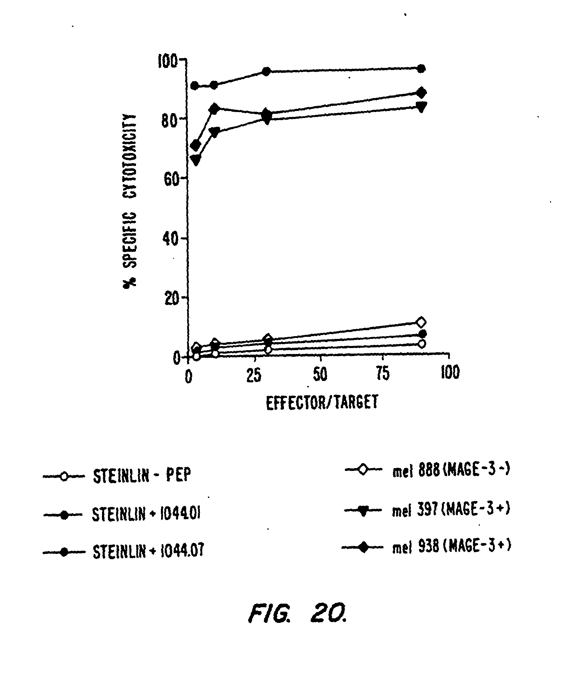

Fig. 20 illustrates the lysis of peptide sensitized targets and endogenous targets following stimulation with SAC-I activated PBMCs loaded with a MAGE 3 peptide.

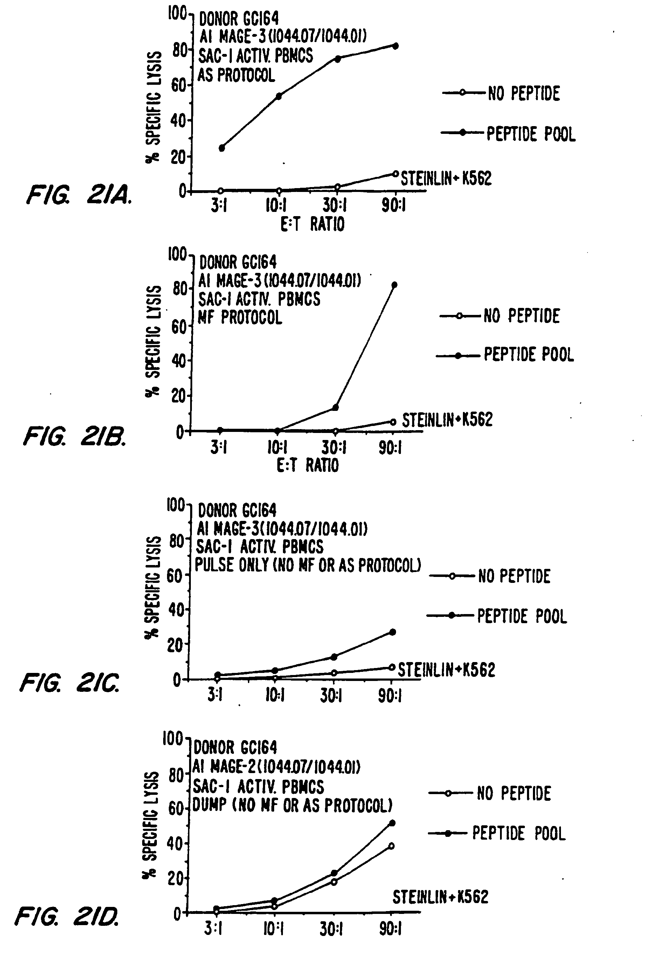

Fig. 21 shows a comparison of the acid strip loading with the cold temperature incubation.

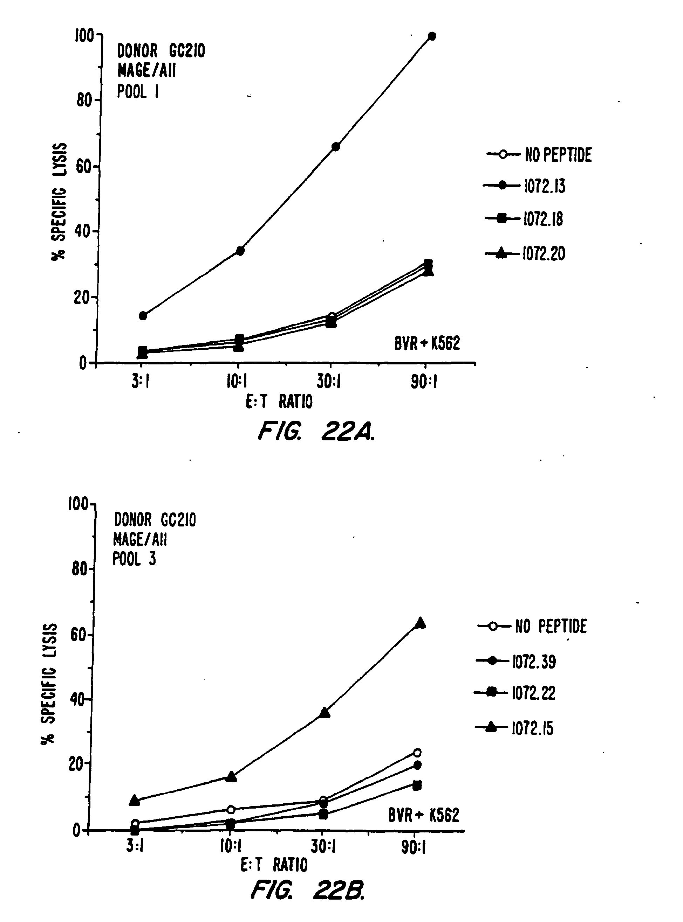

Fig. 22 shows a CTL response to an immunogenic peptide for MAGE/A11.

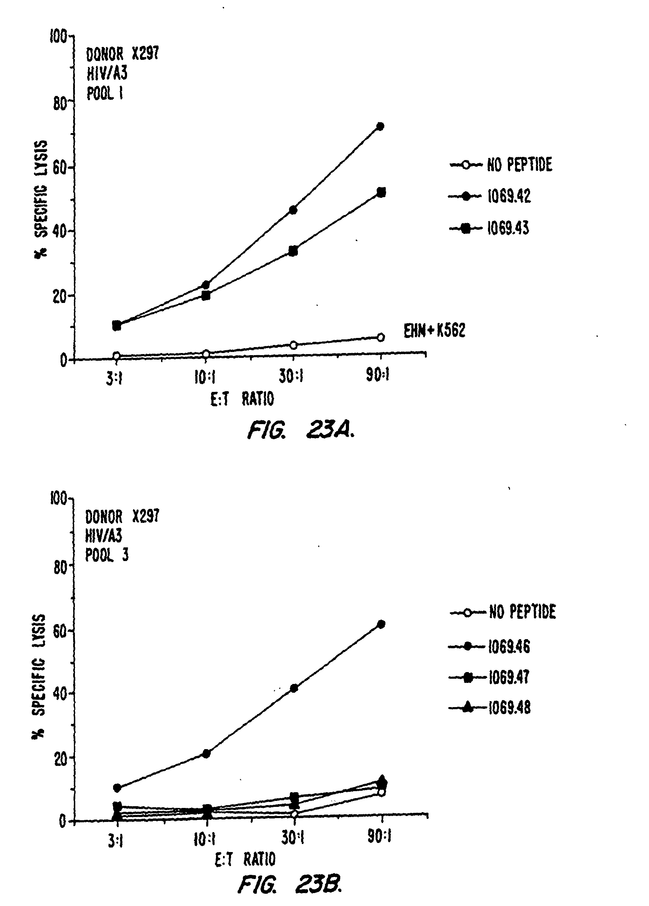

Fig. 23 shows a CTL response to an immunogenic peptide for HIV/A3.

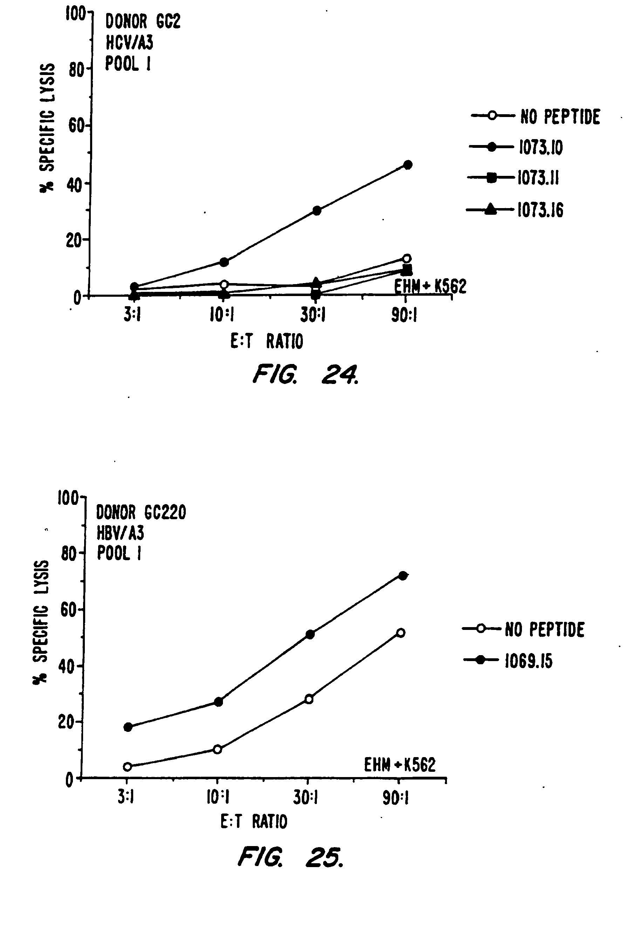

Fig. 24 shows a CTL response to an immunogenic peptide for HCV/A3.
Fig. 25 shows a CTL response to an immunogenic peptide for HBV/A1.

### DESCRIPTION OF THE PREFERRED EMBODIMENTS

The present invention relates to the determination of allele-specific peptide motifs for human Class I MHC (sometimes referred to as HLA) allele subtypes. These motifs are then used to define T cell epitopes from any desired antigen, particularly those associated with human viral diseases or cancers, for which the amino acid sequence of the potential antigen targets is known.

Epitopes on a number of potential target proteins can be identified in this manner. Examples of suitable antigens include prostate specific antigen (PSA), hepatitis B core and surface antigens (HBVc, HBVs) hepatitis C antigens, Epstein-Barr virus antigens, melanoma antigens (e.g., MAGE-1), human immunodeficiency virus (HIV) antigens and human papilloma virus (HPV) antigens.

Peptides comprising these epitopes are synthesized and then tested for their ability to bind to the appropriate MHC molecules in assays using, for example, purified class I molecules and radioiodonated peptides and/or cells expressing empty class I molecules by, for instance, immunofluorescent staining and flow microfluorimetry, peptide-dependent class I assembly assays, and inhibition of CTL recognition by peptide competition. Those peptides that bind to the class I molecule are further evaluated for their ability to serve as targets for CTLs derived from infected or immunized individuals, as well as for their capacity to induce primary in vitro or in vivo CTL responses that can give rise to CTL populations capable of reacting with virally infected target cells or tumor cells as potential therapeutic agents.

The MHC class I antigens are encoded by the HLA-A, B, and C loci. HLA-A and B antigens are expressed at the cell surface at approximately equal densities, whereas the expression of HLA-C is significantly lower (perhaps as much as 10-fold lower). Each of these loci have a number of alleles. The peptide binding motifs of the invention are relatively specific for each allelic subtype.

For peptide-based vaccines, the peptides of the present invention preferably comprise a motif recognized by an MHC I molecule having a wide distribution in the human population. Since the MHC alleles occur at different frequencies within different ethnic groups and races, the choice of target MHC allele may depend upon the target population. Table 1 shows the frequency of various alleles at the HLA-A locus products among different races. For instance, the majority of the Caucasoid population can be covered by peptides which bind to four HLA-A allele subtypes, specifically HLA-A2.1, A1**,** A3.2, and A24.1. Similarly, the majority of the Asian population is encompassed with the addition of peptides binding to a fifth allele FiLA-All.2.

**TABLE 1**

| A Allele/Subtype | N(69)* | A(54) | C(502) |
|---|---|---|---|
| A1 | 10.1(7) | 1.8(1) | 27.4(138) |
| A2.1 | 11.5(8) | 37.0(20) | 39.8(199) |
| A2.2 | 10.1(7) | 0 | 3.3(17) |
| A2.3 | 1.4(1) | 5.5(3) | 0.8(4) |
| A2.4 | - | - | - |
| A2.5 | - | - | - |
| A3.1 | 1.4(1) | 0 | 0.2(0) |
| A3.2 | 5.7(4) | 5.5(3) | 21.5(108) |
| A11.1 | 0 | 5.5(3) | 0 |
| A11.2 | 5.7(4) | 31.4(17) | 8.7(44) |
| A11.3 | 0 | 3.7(2) | 0 |
| A23 | 4.3(3) | - | 3.9(20) |
| A24 | 2.9(2) | 27.7(15) | 15.3(77) |
| A24.2 | - | | - |
| A24.3 | - | - | - |
| A25 | 1.4(1) | - | 6.9(35) |
| A26.1 | 4.3(3) | 9.2(5) | 5.9(30) |
| A26.2 | 7.2(5) | - | 1.0(5) |
| A26V | - | 3.7(2) | - |
| A28.1 | 10.1(7) | - | 1.6(8) |
| A28.2 | 1.4(1) | - | 7.5(38) |
| A29.1 | 1.4(1) | - | 1.4(7) |
| A29.2 | 10.1(7) | 1.8(1) | 5.3(27) |
| A30.1 | 8.6(6) | - | 4.9(25) |
| A30.2 | 1.4(1) | - | 0.2(1) |
| A30.3 | 7.2(5) | - | 3.9(20) |
| A31 | 4.3(3) | 7.4(4) | 6.9(35) |
| A32 | 2.8(2) | - | 7.1(36) |
| Aw33.1 | 8.6(6) | - | 2.5(13) |
| Aw33.2 | 2.8(2) | 16.6(9) | 1.2(6) |
| Aw34.1 | 1.4(1) | - | - |
| Aw34.2 | 14.5(10) | - | 0.8(4) |
| Aw36 | 5.9(4) | - | - |

| | | | |
|---|---|---|---|
| Table compiled from B. DuPont, Immunobiology of HLA, Vol. I, Histocompatibility Testing 1987, Springer-Verlag, New York 1989. * N - negroid; A = Asian; C = caucasoid. Numbers in parenthesis represent the number of individuals included in the analysis. | | | |

The nomenclature used to describe peptide compounds follows the conventional practice wherein the amino group is presented to the left (the N-terminus) and the carboxyl group to the right (the C-terminus) of each amino acid residue. In the formulae representing selected specific embodiments of the present invention, the amino- and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids is represented by a lower case single letter or a lower case three letter symbol. Glycine has no asymmetric carbon atom and is simply referred to as "Gly" or G.

The procedures used to identify peptides of the present invention generally follow the methods disclosed in Falk et al., Nature 351:290 (1991), which is incorporated herein by reference. Briefly, the methods involve large-scale isolation of MHC class I molecules, typically by immunoprecipitation or affinity chromatography, from the appropriate cell or cell line. Examples of other methods for isolation of the desired MHC molecule equally well known to the artisan include ion exchange chromatography, lectin chromatography, size exclusion, high performance ligand chromatography, and a combination of all of the above techniques.

A large number of cells with defined MHC molecules, particularly MHC Class I molecules, are known and readily available. For example, human EBV-transformed B cell lines have been shown to be excellent sources for the preparative isolation of class I and class II MHC molecules. Well-characterized cell lines are available from private and commercial sources, such as American Type Culture Collection ("Catalogue of Cell Lines and Hybridomas," 6th edition (1988) Rockville, Maryland, U.S.A.); National Institute of General Medical Sciences 1990/1991 Catalog of Cell Lines (NIGMS) Human Genetic Mutant Cell Repository, Camden, NJ; and ASHI Repository, Bingham and Women's Hospital, 75 Francis Street, Boston, MA 02115. Table 2 lists some B cell lines suitable for use as sources for HLA-A alleles. All of these cell lines can be grown in large batches and are therefore useful for large scale production of MHC molecules. One of skill will recognize that these are merely exemplary cell lines and that many other cell sources can be employed. Similar EBV B cell lines homozygous for HLA-B and HLA-C could serve as sources for HLA-B and HLA-C alleles, respectively.

**TABLE 2 HUMAN CELL LINES (HLA-A SOURCES)**

| HLA-A allele | B cell line |
|---|---|
| A1 | MAT |
| | COX (9022) |
| | STEINLIN |
| | (9087) |
| | |
| A2.1 | JY |
| | |
| A3.2 | EHM (9080) |
| | HO301 (9055)GM3107 |
| | |
| A24.1 | KT3 (9107), TISI (9042) |
| | |
| A11 | BVR (GM6828A) |
| | WT100 (GM8602)WT52 |
| | (GM8603) |

In the typical case, immunoprecipitation is used to isolate the desired allele. A number of protocols can be used, depending upon the specificity of the antibodies used. For example, allele-specific mAb reagents can be used for the affinity purification of the HLA-A, HLA-B, and HLA-C molecules. Several mAb reagents for the isolation of HLA-A molecules are available (Table 3). Thus, for each of the targeted HLA-A alleles, reagents are available that may be used for the direct isolation of the HLA-A molecules. Affinity columns prepared with these mAbs using standard techniques are successfully used to purify the respective HLA-A allele products.

In addition to allele-specific mAbs, broadly reactive anti-HLA-A, B, C mAbs, such as W6/32 and B9.12.1, and one anti-HLA-B, C mAb, B1.23.2, could be used in alternative affinity purification protocols as described in the example section below.

**TABLE 3 ANTIBODY REAGENTS**

| anti-HLA | Name |
|---|---|
| HLA-A1 | 12/18 |
| HLA-A3 | GAPA3 (ATCC, HB122) |
| HLA-11,24.1 | A11.1M (ATCC, HB164) |
| HLA-A,B,C | W6/32 (ATCC, HB95) |
| monomorphic | B9.12.1 (INSERM-CNRS) |
| HLA-B,C | B.1.23.2 (INSERM-CNRS) |
| monomorphic | |

The peptides bound to the peptide binding groove of the isolated MHC molecules are eluted typically using acid treatment. Peptides can also be dissociated from class I molecules by a variety of standard denaturing means, such as heat, pH, detergents, salts, chaotropic agents, or a combination thereof.

Peptide fractions are further separated from the MHC molecules by reversed-phase high performance liquid chromatography (HPLC) and sequenced. Peptides can be separated by a variety of other standard means well known to the artisan, including filtration, ultrafiltration, electrophoresis, size chromatography, precipitation with specific antibodies, ion exchange chromatography, isoelectrofocusing, and the like.

Sequencing of the isolated peptides can be performed according to standard techniques such as Edman degradation (Hunkapiller, M.W., et al., Methods Enzymol. 91, 399 [1983]). Other methods suitable for sequencing include mass spectrometry sequencing of individual peptides as previously described (Hunt, et al., Science 225:1261 (1992), which is incorporated herein by reference). Amino acid sequencing of bulk heterogenous peptides (e.g., pooled HPLC fractions) from different class I molecules typically reveals a characteristic sequence motif for each class I allele.

Definition of motifs specific for different class I alleles allows the identification of potential peptide epitopes from an antigenic protein whose amino acid sequence is known. Typically, identification of potential peptide epitopes is initially carried out using a computer to scan the amino acid sequence of a desired antigen for the presence of motifs. The epitopic sequences are then synthesized. The capacity to bind MHC Class molecules is measured in a variety of different ways. One means is a Class I molecular binding assay as described in Example 10, below. Other alternatives described in the literature include inhibition of antigen presentation (Sette, et al., J. Immunol. 141:3893 (1991), in vitro assembly assays (Townsend, et al., Cell 62:285 (1990), and FACS based assays using mutated ells, such as RMA.S (Melief, et al., Eur. J. Immunol. 21:2963 [1991]).

Next, peptides that test positive in the MHC class I binding assay are assayed for the ability of the peptides to induce specific CTL responses in vitro. For instance, antigen-presenting cells that have been incubated with a peptide can be assayed for the ability to induce CTL responses in responder cell populations. Antigen-presenting cells can be normal cells such as peripheral blood mononuclear cells or dendritic cells (Inaba, et al., J. Exp. Med. 166:182 (1987); Boog, Eur. J. Immunol. 18:219 [1988]).

Alternatively, mutant mammalian cell lines that are deficient in their ability to load class I molecules with internally processed peptides, such as the mouse cell lines RMA-S (Karre, et al.. Nature, 319:675 (1986); Ljunggren, et al., Eur. J. Immunol. 21:2963-2970 (1991)), and the human somatic T cell hybridoma, T-2 (Cerundolo, et al., Nature 345:449-452 (1990)) and which have been transfected with the appropriate human class I genes are conveniently used, when peptide is added to them, to test for the capacity of the peptide to induce in vitro primary CTL responses. Other eukaryotic cell lines which could be used include various insect cell lines such as mosquito larvae (ATCC cell lines CCL 125, 126, 1660, 1591, 6585, 6586), silkworm (ATTC CRL 8851), armyworm (ATCC CRL 1711), moth (ATCC CCL 80) and Drosophila cell lines such as a Schneider cell line (see Schneider J. Embryol. Exp. Morphol. 27:353-365 [1927]). That have been transfected with the appropriate human class I MHC allele encoding genes and the human B₂ microglobulin genes.

Peripheral blood lymphocytes are conveniently isolated following simple venipuncture or leukapheresis of normal donors or patients and used as the responder cell sources of CTL precursors. In one embodiment, the appropriate antigen-presenting cells are incubated with 10-100 µM of peptide in serum-free media for 4 hours under appropriate culture conditions. The peptide-loaded antigen-presenting cells are then incubated with the responder cell populations in vitro for 7 to 10 days under optimized culture conditions. Positive CTL activation can be determined by assaying the cultures for the presence of CTLs that kill radiolabeled target cells, both specific peptide-pulsed targets as well as target cells expressing endogenously processed form of the relevant virus or tumor antigen from which the peptide sequence was derived.

Specificity and MHC restriction of the CTL is determined by testing against different peptide target cells expressing appropriate or inappropriate human MHC class I. The peptides that test positive in the MHC binding assays and give rise to specific CTL responses are referred to herein as immunogenic peptides.

The immunogenic peptides can be prepared synthetically, or by recombinant DNA technology or isolated from natural sources such as whole viruses or tumors. Although the peptide will preferably be substantially free of other naturally occurring host cell proteins and fragments thereof, in some embodiments the peptides can be synthetically conjugated to native fragments or particles. The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

Desirably, the peptide will be as small as possible while still maintaining substantially all of the biological activity of the large peptide. When possible, it may be desirable to optimize peptides of the invention to a length of 9 or 10 amino acid residues, commensurate in size with endogenously processed viral peptides or tumor cell peptides that are bound to MHC class I molecules on the cell surface.

Peptides having the desired activity may be modified as necessary to provide certain desired attributes, e.g., improved pharmacological characteristics, while increasing or at least retaining substantially all of the biological activity of the unmodified peptide to bind the desired MHC molecule and activate the appropriate T cell. For instance, the peptides may be subject to various changes, such as substitutions, either conservative or non-conservative, where such changes might provide for certain advantages in their use, such as improved MHC binding. By conservative substitutions is meant replacing an amino acid residue with another which is biologically and/or chemically similar, e.g., one hydrophobic residue for another, or one polar residue for another. The substitutions include combinations such as Gly, Ala; Val, Ile, Leu, Met; Asp, Glu; Asn, Gln; Ser, Thr; Lys, Arg; and Phe, Tyr. The effect of single amino acid substitutions may also be probed using D-amino acids. Such modifications may be made using well known peptide synthesis procedures, as described in e.g., Merrifield, Science 232:341-347 (1986), Barany and Merrifield, The Peptides. Gross and Meienhofer, eds. (N.Y., Academic Press), pp. 1-284 (1979); and Stewart and Young, Solid Phase peptide synthesis, (Rockford, Ill., Pierce), 2d Ed. (1984), incorporated by reference herein.

The peptides can also be modified by extending or decreasing the compound's amino acid sequence, e.g., by the addition or deletion of amino acids. The peptides or analogs of the invention can also be modified by altering the order or composition of certain residues, it being readily appreciated that certain amino acid residues essential for biological activity, e.g., those at critical contact sites or conserved residues, may generally not be altered without an adverse effect on biological activity. The non-critical amino acids need not be limited to those naturally occurring in proteins, such as L-α-amino acids, or their D-isomers, but may include non-natural amino acids as well, such as β-γ-δ-amino acids, as well as many derivatives of L-α-amino acids.

Typically, a series of peptides with single amino acid substitutions are employed to determine the effect of electrostatic charge, hydrophobicity, etc. on binding. For instance, a series of positively charged (e.g., Lys or Arg) or negatively charged (e.g., Glu) amino acid substitutions are made along the length of the peptide revealing different patterns of sensitivity towards various MHC molecules and T cell receptors. In addition, multiple substitutions using small, relatively neutral moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (e.g., hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding.

Amino acid substitutions are typically or single residues. Substitutions, deletions, insertions or any combination thereof may be combined to arrive at a final peptide. Substitutional variants are those in which at least one residue of a peptide has been removed and a different residue inserted in its place. Such substitutions generally are made in accordance with the following Table 4 when it is desired to finely modulate the characteristics of the peptide.

**TABLE 4**

| Original Residue | Exemplary Substitution |
|---|---|
| Ala | ser |
| Arg | lys |
| Asn | gIn; his |
| Asp | glu |
| Cys | ser |
| GIn | asn |
| Glu | asp |
| Gly | pro |
| His | asn; gln |
| Ile | leu; val |
| Leu | ile; val |
| Lys | arg |
| Met | leu; ile |
| Phe | met; leu; tyr |
| Ser | thr |
| Thr | ser |
| Trp | tyr |
| Tyr | trp; phe |
| Val | ile; leu |

Substantial changes in function (e.g., affinity for MHC molecules or T cell receptors) are made by selecting substitutions that are less conservative than those in Table 4, i.e., selecting residues that differ more significantly in their effect on maintaining (a) the structure of the peptide backbone in the area of the substitution, for example as a sheet or helical conformation, (b) the charge or hydrophobicity of the molecule at the target site or (c) the bulk of the side chain. The substitutions which in general are expected to produce the greatest changes in peptide properties will be those in which (a) hydrophilic residue, e.g. seryl, is substituted for (or by) a hydrophobic residue, e.g. leucyl, isoleucyl, phenylalanyl, valyl or alanyl; (b) a residue having an electropositive side chain, e.g., lysyl, arginyl, or histidyl, is substituted for (or by) an electronegative residue, e.g. glutamyl or aspartyl; or (c) a residue having a bulky side chain, e.g. phenylalanine, is substituted for (or by) one not having a side chain, e.g., glycine.

The peptides may also comprise isosteres of two or more residues in the immunogenic peptide. An isostere as defined here is a sequence of two or more residues that can be substituted for a second sequence because the steric conformation of the first sequence fits a binding site specific for the second sequence. The term specifically includes peptide backbone modifications well known to those skilled in the art. Such modifications include modifications of the amide nitrogen, the α-carbon, amide carbonyl, complete replacement of the amide bond, extensions, deletions or backbone crosslinks. See, generally, Spatola, Chemistry and Biochemistry of Amino Acids, peptides and Proteins, Vol. VII (Weinstein ed., 1983).

Modifications of peptides with various amino acid mimetics or unnatural amino acids are particularly useful in increasing the stability of the peptide in vivo. Stability can be assayed in a number of ways. For instance, peptidases and various biological media, such as human plasma and serum, have been used to test stability. See, e.g., Verhoef et al., Eur. J. Drug Metab Pharmacokin. 11:291-302 (1986). Half life of the peptides of the present invention is conveniently determined using a 25% human serum (v/v) assay. The protocol is generally as follows. Pooled human serum (Type AB, non-heat inactivated) is delipidated by centrifugation before use. The serum is then diluted to 25% with RPMI tissue culture media and used to test peptide stability. At predetermined time intervals a small amount of reaction solution is removed and added to either 6% aqueous trichloracetic acid or ethanol. The cloudy reaction sample is cooled (4°C) for 15 minutes and then spun to pellet the precipitated serum proteins. The presence of the peptides is then determined by reversed-phase HPLC using stability-specific chromatography conditions.

The peptides of the present invention or analogs thereof which have CTL stimulating activity may be modified to provide desired attributes other than improved serum half life. For instance, the ability of the peptides to induce CTL activity can be enhanced by linkage to a sequence which contains at least one epitope that is capable of inducing a T helper cell response. Particularly preferred immunogenic peptides/T helper conjugates are linked by a spacer molecule. The spacer is typically comprised of relatively small, neutral molecules, such as amino acids or amino acid mimetics, which are substantially uncharged under physiological conditions. The spacers are typically selected from, e.g., Ala, Gly, or other neutral spacers of nonpolar amino acids or neutral polar amino acids. It will be understood that the optionally present spacer need not be comprised of the same residues and thus may be a hetero- or homo-oligomer. When present, the spacer will usually be at least one or two residues, more usually three to six residues. Alternatively, the CTL peptide may be linked to the T helper peptide without a spacer.

The immunogenic peptide may be linked to the T helper peptide either directly or via a spacer either at the amino or carboxy terminus of the CTL peptide. The amino terminus of either the immunogenic peptide or the T helper peptide may be acylated.

In some embodiments it may be desirable to include in the pharmaceutical compositions of the invention at least one component which assists in priming CTL. Lipids have been identified as agents capable of assisting the priming CTL in vivo against viral antigens. For example, palmitic acid residues can be attached to the alpha and epsilon amino groups of a Lys residue and then linked, e.g., via one or more linking residues such as Gly, Gly-Gly-, Ser, Ser-Ser, or the like, to an immunogenic peptide. The lipidated peptide can then be injected directly in a micellar form, incorporated into a liposome or emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of the immunogenic peptide.

As another example of lipid priming of CTL responses, E. coli lipoproteins, such as tripalmitoyl-S-glycerylcysteinlyseryl-serine (P₃CSS) can be used to prime virus specific CTL when covalently attached to an appropriate peptide. See, Deres et al., Nature 342:561-564 (1989), incorporated herein by reference. Peptides of the invention can be coupled to P₃CSS, for example, and the lipopeptide administered to an individual to specifically prime a CTL response to the target antigen. Further, as the induction of neutralizing antibodies can also be primed with P₃CSS conjugated to a peptide which displays an appropriate epitope, the two compositions can be combined to more effectively elicit both humoral and cell-mediated responses to infection.

In addition, additional amino acids can be added to the termini of a peptide to provide for ease of linking peptides one to another, for coupling to a carrier support, or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. Modification at the C terminus in some cases may alter binding characteristics of the peptide. In addition, the peptide or oligopeptide sequences can differ from the natural sequence by being modified by terminal-NH₂ acylation, e.g., by alkanoyl (C₁-C₂₀) or thioglycolyl acetylation, terminal-carboxyl amidation, e.g., ammonia, methylamine, etc. In some instances these modifications may provide sites for linking to a support or other molecule.

The peptides of the invention can be prepared in a wide variety of ways. Because of their relatively short size, the peptides can be synthesized in solution or on a solid support in accordance with conventional techniques. Various automatic synthesizers are commercially available and can be used in accordance with known protocols. See, for example, Stewart and Young, Solid Phase Peptide Synthesis, 2d. ed., Pierce Chemical Co. (1984), supra.

Alternatively, recombinant DNA technology may be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook et al., Molecular Cloning, A Laboratory Manual, Cold Spring Harbor Press, Cold Spring Harbor, New York (1982), which is incorporated herein by reference. Thus, fusion proteins which comprise one or more peptide sequences of the invention can be used to present the appropriate T cell epitope.

As the coding sequence for peptides of the length contemplated herein can be synthesized by chemical techniques, for example, the phosphotriester method of Matteucci et al., J. Am. Chem. Soc. 103:3185 (1981), modification can be made simply by substituting the appropriate base(s) for those encoding the native peptide sequence. The coding sequence can then be provided with appropriate linkers and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Of course, yeast or mammalian cell hosts may also be used, employing suitable vectors and control' sequences.

The peptides of the present invention and pharmaceutical and vaccine compositions thereof are useful for administration to mammals, particularly humans, to treat and/or prevent viral infection and cancer. Examples of diseases which can be treated using the immunogenic peptides of the invention include prostate cancer, hepatitis B, hepatitis C, AIDS, renal carcinoma, cervical carcinoma, lymphoma, CMV and condlyloma acuminatum.

For pharmaceutical compositions, the immunogenic peptides of the invention are administered to an individual already suffering from cancer or infected with the virus of interest. Those in the incubation phase or the acute phase of infection can be treated with the immunogenic peptides separately or in conjunction with other treatments, as appropriate. In therapeutic applications, compositions are administered to a patient in an amount sufficient to elicit an effective CTL response to the virus or tumor antigen and to cure or at least partially arrest symptoms and/or complications. An amount adequate to accomplish this is defined as "therapeutically effective dose." Amounts effective for this use will depend on, e.g., the peptide composition, the manner of administration, the stage and severity of the disease being treated, the weight and general state of health of the patient, and the judgment of the prescribing physician, but generally range for the initial immunization (that is for therapeutic or prophylactic administration) from about 1.0 µg to about 5000 µg of peptide for a 70 kg patient, followed by boosting dosages of from about 1.0 µg to about 1000 µg of peptide pursuant to a boosting regimen over weeks to months depending upon the patient's response and condition by measuring specific CTL activity in the patient's blood. It must be kept in mind that the peptides and compositions of the present invention may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

For therapeutic use, administration should begin at the first sign of viral infection or the detection or surgical removal of tumors or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide compositions can also be used for the treatment of chronic infection and to stimulate the immune system to eliminate virus-infected cells in carriers. It is important to provide an amount of immuno-potentiating peptide in a formulation and mode of administration sufficient to effectively stimulate a cytotoxic T cell response. Thus, for treatment of chronic infection, a representative dose is in the range of about 1.0 µg to about 5000 µg, preferably about 5 µg to 1000 µg for a 70 kg patient per dose. Immunizing doses followed by boosting doses at established intervals, e.g., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic treatment are intended for parenteral, topical, oral or local administration. Preferably, the pharmaceutical compositions are administered parenterally, e.g., intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used, e.g., water, buffered water, 0.9% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of CTL stimulatory peptides of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

The peptides of the invention may also be administered via liposomes, which serve to target the peptides to a particular tissue, such as lymphoid tissue, or targeted selectively to infected cells, as well as increase the half-life of the peptide composition. Liposomes include emulsions, foams, micelles, insoluble monolayers, liquid crystals, phospholipid dispersions, lamellar layers and the like, In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes filled with a desired peptide of the invention can be directed to the site of lymphoid cells, where the liposomes then deliver the selected therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al., Ann. Rev. Biophys. Bioeng. 9:467 (1980), U.S. Patent Nos. 4,235,871, 4,501,728, 4,837,028, and 5,019,369, incorporated herein by reference.

For targeting to the immune cells, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, inter alia, the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, the immunogenic peptides are preferably supplied in finely divided form along with a surfactant and propellant. Typical percentages of peptides are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, e.g., lecithin for intranasal delivery.

In another aspect the present invention is directed to vaccines which contain as an active ingredient an immunogenically effective amount of an immunogenic peptide as described herein. The peptide(s) may be introduced into a host, including humans, linked to its own carrier or as a homopolymer or heteropolymer of active peptide units. Such a polymer has the advantage of increased immunological reaction and, where different peptides are used to make up the polymer, the additional ability to induce antibodies and/or CTLs that react with different antigenic determinants of the virus or tumor cells. Useful carriers are well known in the art, and include, e.g., thyroglobulin, albumins such as bovine serum albumin, tetanus toxoid, polyamino acids such as poly(lysine:glutamic acid), hepatitis B virus core protein, hepatitis B virus recombinant vaccine and the like. The vaccines can also contain a physiologically coleraoie (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art. And, as mentioned above, CTL responses can be primed by conjugating peptides of the invention to lipids, such as P₃CSS. Upon immunization with a peptide composition as described herein, via injection, aerosol, oral, transdermal or other route, the immune system of the host responds to the vaccine by producing large amounts of CTLs specific for the desired antigen, and the host becomes at least partially immune to later infection, or resistant to developing chronic infection.

Vaccine compositions containing the peptides of the invention are administered to a patient susceptible to or otherwise at risk of viral infection or cancer to elicit an immune response against the antigen and thus enhance the patient's own immune response capabilities. Such an amount is defined to be an "immunogenically effective dose." In this use, the precise amounts again depend on the patient's state of health and weight, the mode of administration, the nature of the formulation, etc., but generally range from about 1.0 µg to about 5000 µg per 70 kilogram patient, more commonly from about 10 µg to about 500 µg mg per 70 kg of body weight.

In some instances it may be desirable to combine the peptide vaccines of the invention with vaccines which induce neutralizing antibody responses to the virus of interest, particularly to viral envelope antigens.

For therapeutic or immunization purposes, the peptides of the invention can also be expressed by attenuated viral hosts, such as vaccinia or fowlpox. This approach involves the use of vaccinia virus as a vector to express nucleotide sequences that encode the peptides of the invention. Upon introduction into an acutely or chronically infected host or into a non-infected host, the recombinant vaccinia virus expresses the immunogenic peptide, and thereby elicits a host CTL response. Vaccinia vectors and methods useful in immunization protocols are described in, e.g.,U.S. Patent No. 4,722,848, incorporated herein by reference. Another vector is BCG (Bacille Calmette Guerin). BCG vectors are described in Stover et al. (Nature 351:456-460 (1991)) which is incorporated herein by reference. A wide variety of other vectors useful for therapeutic administration or immunization of the peptides of the invention, e.g., Salmonella typhi vectors and the like, will be apparent to those skilled in the art from the description herein.

Antigenic peptides may be used to elicit CTL ex vivo, as well. The resulting CTL, can be used to treat chronic infections (viral or bacterial) or tumors in patients that do not respond to other conventional forms of therapy, or will not respond to a peptide vaccine approach of therapy. Ex vivo CTL responses to a particular pathogen (infectious agent or tumor antigen) are induced by incubating in tissue culture the patient's CTL precursor cells (CTLp) together with a source of antigen-presenting cells (APC) and the appropriate immunogenic peptide. After an appropriate incubation time (typically 1-4 weeks), in which the CTLp are activated and mature and expand into effector CTL, the cells are infused back into the patient, where they will destroy their specific target cell (an infected cell or a tumor cell). In order to optimize the *in vitro* conditions for the generation of specific cytotoxic T cells, the culture of stimulator cells is maintained in an appropriate serum-free medium.

Prior to incubation of the stimulator cells with the cells to be activated, e.g., precursor CD8+ cells, an amount of antigenic peptide is added to the stimulator cell culture, of sufficient quantity to become loaded onto the human Class I molecules to be expressed on the surface of the stimulator cells. In the present invention, a sufficient amount of peptide is an amount that will allow about 200, and preferably 200 or more, human Class I MHC molecules loaded with peptide to be expressed on the surface of each stimulator cell. Preferably, the stimulator cells are incubated with >20µg/ml peptide.

Resting or precursor CD8+ cells are then incubated in culture with the appropriate stimulator cells for a time period sufficient to activate the CD8+ cells. Preferably, the CD8+ cells are activated in an antigen-specific manner. The ratio of resting or precursor CD8+ (effector) cells to stimulator cells may vary from individual to individual and may further depend upon variables such as the amenability of an individual's lymphocytes to culturing conditions and the nature and severity of the disease condition or other condition for which the within-described treatment modality is used. Preferably, however, the lymphocyte:stimulator cell ratio is in the range of about 30:1 to 300:1. The effector/stimulator culture may be maintained for as long a time as is necessary to stimulate a therapeutically useable or effective number of CD8+ cells.

The induction of CTL in *vitro* requires the specific recognition of peptides that are bound to allele specific MHC class I molecules on APC. The number of specific MHC/peptide complexes per APC is crucial for the stimulation of CTL, particularly in primary immune responses. While small amounts of peptide/MHC complexes per cell are sufficient to render a cell susceptible to lysis by CTL, or to stimulate a secondary CTL response, the successful activation of a CTL precursor (pCTL) during primary response requires a significantly higher number of MHC/peptide complexes. Peptide loading of empty major histocompatability complex molecules on cells allows the induction of primary cytotoxic T lymphocyte responses. Peptide loading of empty major histocompatability complex molecules on cells enables the induction of primary cytotoxic T lymphocyte responses.

Since mutant cell lines do not exist for every human MHC allele, it is advantageous to use a technique to remove endogenous MHC-associated peptides from the surface of APC, followed by loading the resulting empty MHC molecules with the immunogenic peptides of interest. The use of non-transformed (non-tumorigenic), non-infected cells, and preferably, autologous cells of patients as APC is desirable for the design of CTL induction protocols directed towards development of *ex vivo* CTL therapies. This application discloses methods for stripping the endogenous MHC-associated peptides from the surface of APC followed by the loading of desired peptides.

A stable MHC class I molecule is a trimeric complex formed of the following elements: 1) a peptide usually of 8 - 10 residues, 2) a transmembrane heavy polymorphic protein chain which bears the peptide-binding site in its α1 and α2 domains, and 3) a non-covalently associated non-polymorphic light chain, β₂microglobulin. Removing the bound peptides and/or dissociating the β₂microglobulin from the complex renders the MHC class I molecules nonfunctional and unstable, resulting in rapid degradation. All MHC class I molecules isolated from PBMCs have endogenous peptides bound to them. Therefore, the first step is to remove all endogenous peptides bound to MHC class I molecules on the APC without causing their.degradation before exogenous peptides can be added to them.

Two possible ways to free up MHC class I molecules of bound peptides include lowering the culture temperature from 37°C to 26°C overnight to destablize β₂microglobulin and stripping the endogenous peptides from the cell using a mild acid treatement. The methods release previously bound peptides into the extracellular environment allowing new exogenous peptides to bind to the empty class I molecules. The cold-temperature incubation method enables exogenous peptides to bind efficiently to the MHC complex, but requires an overnight incubation at 26°C which may slow the cell's metabolic rate. It is also likely that cells not actively synthesizing MHC molecules (e.g., resting PBMC) would not produce high amounts of empty surface MHC molecules by the cold temperature procedure.

Harsh acid stripping involves extraction of the peptides with trifluoroacetic acid, pH 2, or acid denaturation of the immunoaffinity purified class I-peptide complexes. These methods are not feasible for CTL induction, since it is important to remove the endogenous peptides while preserving APC viability and an optimal metabolic state which is critical for antigen presentation. Mild acid solutions of pH 3 such as glycine or citrate-phosphate buffers have been used to identify endogenous peptides and to identify tumor associated T cell epitopes. The treatment is especially effective, in that only the MHC class I molecules are destabilized (and associated peptides released), while other surface antigens remain intact, including MHC class II molecules. Most importantly, treatment of cells with the mild acid solutions do not affect the cell's viability or metabolic state. The mild acid treatment is rapid since the stripping of the endogenous peptides occurs in two minutes at 4°C and the APC is ready to perform its function after the appropriate peptides are loaded. The technique is utilized herein to make peptide-specific APCs for the generation of primary antigen-specific CTL. The resulting APC are efficient in inducing peptide-specific CD8+ CTL.

Activated CD8+ cells may be effectively separated from the stimulator cells using one of a variety of known methods. For example, monoclonal antibodies specific for the stimulator cells, for the peptides loaded onto the stimulator cells, or for the CD8+ cells (or a segment thereof) may be utilized to bind their appropriate complementary ligand. Antibody-tagged molecules may then be extracted from the stimulator-effector cell admixture via appropriate means, e.g., via well-known immunoprecipitation or immunoassay methods.

Effective, cytotoxic amounts of the activated CD8+ cells can vary between *in vitro* and *in vivo* uses, as well as with the amount and type of cells that are the ultimate target of these killer cells. The amount will also vary depending on the condition of the patient and should be determined via consideration of all appropriate factors by the practitioner. Preferably, however, about 1 X 10⁶ to about 1 X 10¹², more preferably about 1 X 10⁸ to about 1 X 10¹¹, and even more preferably, about 1 X 10⁹ to about 1 X 10¹⁰ activated CD8+ cells are utilized for adult humans, compared to about 5 X 10⁶ - 5 X 10⁷ cells used in mice.

Preferably, as discussed above, the activated CD8+ cells are harvested from the cell culture prior to administration of the CD8+ cells to the individual being treated. It is important to note, however, that unlike other present and proposed treatment modalities, the present method uses a cell culture system that is not tumorigenic. Therefore, if complete separation of stimulator cells and activated CD8+ cells is not achieved, there is no inherent danger known to be associated with the administration of a small number of stimulator cells, whereas administration of mammalian tumor-promoting cells may be extremely hazardous.

Methods of re-introducing cellular components are known in the art and include procedures such as those exemplified in U.S. Patent No. 4,844,893 to Honsik, et al. and U.S. Patent No. 4,690,915 to Rosenberg. For example, administration of activated CD8+ cells via intravenous infusion is appropriate.

The immunogenic peptides of this invention may also be used to make monoclonal antibodies. Such antibodies may be useful as potential diagnostic or therapeutic agents.

The peptides may also find use as diagnostic reagents. For example, a peptide of the invention may be used to determine the susceptibility of a particular individual to a treatment regimen which employs the peptide or related peptides, and thus may be helpful in modifying an existing treatment protocol or in determining a prognosis for an affected individual. In addition, the peptides may also be used to predict which individuals will be at substantial risk for developing chronic infection.

The following examples are offered by way of illustration, not by way of limitation.

### Example 1

### Class I antigen isolation

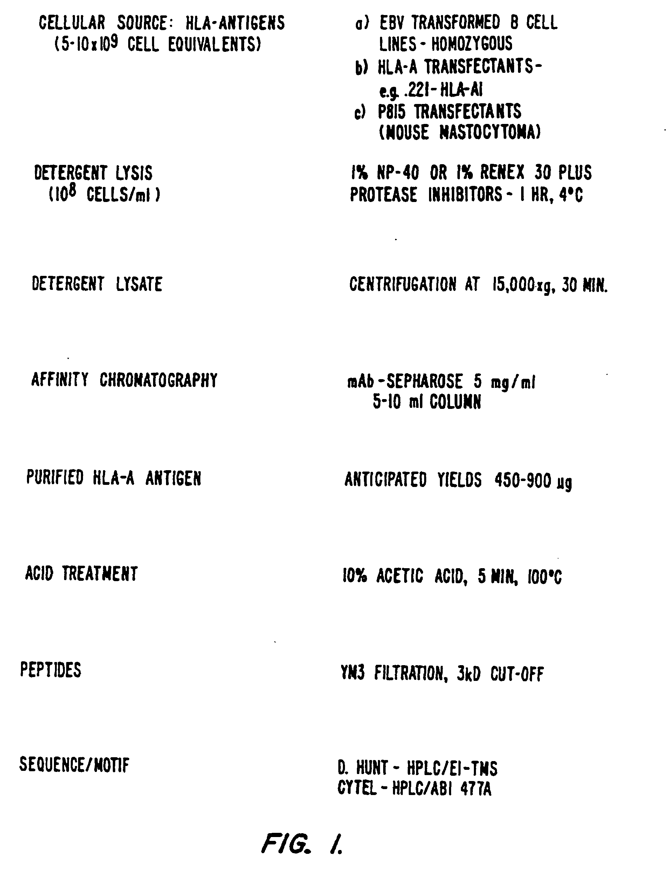

A flow diagram of an HLA-A antigen purification scheme is presented in Figure 1. Briefly, the cells bearing the appropriate allele were grown in large batches (6-8 liters yielding ^{∼}5 x 10⁹ cells), harvested by centrifugation and washed. All cell lines were maintained in RPMI 1640 media (Sigma) supplemented with 10% fetal bovine serum (FBS) and antibiotics. For large-scale cultures, cells were grown in roller bottle culture in RPMI 1640 with 10% FBS or with 10% horse serum and antibiotics. Cells were harvested by centrifugation at 1500 RPM IEC-CRU5000 centrifuge with 259 rotor and washed three times with phosphate-buffered saline (PBS)(0.01 M PO₄, 0.154 M NaCl, pH 7.2).

Cells were pelleted and stored at -70°C or treated with detergent lysing solution to prepare detergent lysates. Cell lysates were prepared by the addition of stock detergent solution [1% NP-40 (Sigma) or Renex 30 (Accurate Chem. Sci. Corp., Westbury, NY 11590), 150 mM NaCl, 50 mM Tris, pH 8.0] to the cell pellets (previously counted) at a ratio of 50-100 x 10⁶ cells per ml detergent solution. A cocktail of protease inhibitors was added to the premeasured volume of stock detergent solution immediately prior to the addition to the cell pellet. Addition of the protease inhibitor cocktail produced final concentrations of the following: phenylmethylsulfonyl fluoride (PMSF), 2 mM; aprotinin, 5 µg/ml; leupeptin, 10 µg/ml; pepstatin, 10 µg/ml; iodoacetamide, 100 µM; and EDTA, 3 ng/ml. Cell lysis was allowed to proceed at 4°C for 1 hour with periodic mixing. Routinely 5-10 x 10⁹ cells were lysed in 50-100 ml of detergent solution. The lysate was clarified by centrifugation at 15,000 x g for 30 minutes at 4°C and subsequent passage of the supernatant fraction through a 0.2 filter unit (Nalgene).

The HLA-A antigen purification was achieved using affinity columns prepared with mab-conjugated Sepharose beads. For antibody production, cells were grown in RPMI with 10% FBS in large tissue culture flasks (Corning 25160-225). Antibodies were purified from clarified tissue culture medium by ammonium sulfate fractionation followed by affinity chromatography on protein-A-Sepharose (Sigma). Briefly, saturated ammonium sulfate was added slowly with stirring to the tissue culture supernatant to 45% (volume to volume) overnight at 4°C to precipitate the immunoglobulins. The precipitated proteins were harvested by centrifugation at 10,000 x g for 30 minutes. The precipitate was then dissolved in a minimum volume of PBS and transferred to dialysis tubing (Spectro/Por 2, Mol. wt. cutoff 12,000-14,000, Spectum Medical Ind.). Dialysis was against PBS (≥20 times the protein solution volume) with 4-6 changes of dialysis buffer over a 24-48 hour period at 4°C. The dialyzed protein solution was clarified by centrifugation (10,000 x g for 30 minutes) and the pH of the solution adjusted to pH 8.0 with 1N NaOH. Protein-A-Sepharose (Sigma) was hydrated according to the manufacturer's instructions, and a protein-A-Sepharose column was prepared. A column of 10 ml bed volume typically binds 50-100 mg of mouse IgG.

The protein sample was loaded onto the protein-A-Sepharose column using a peristaltic pump for large loading volumes or by gravity for smaller volumes (<100 ml). The column was washed with several volumes of PBS, and the eluate was monitored at A280 in a spectrophotometer until base line was reached. The bound antibody was eluted using 0.1 M citric acid at suitable pH (adjusted to the appropriate pH with IN' NaOH). For mouse IgG-1 pH 6.5 was used for IgG2a pH 4.5 was used and for IgG2b and IgG3 pH 3.0 was used. 2 M Tris base was used to neutralize the eluate. Fractions containing the antibody (monitored by A280) were pooled, dialyzed against PBS and further concentrated using an Amicon Stirred Cell system (Amicon Model 8050 with YM30 membrane). The anti-A2 mAb, BB7.2, and the anti-A3 mAb, GAPA3, are particularly useful for affinity purification.

The HLA-A antigen was purified using affinity columns prepared with mAb-conjugated Sepharose beads. The affinity columns were prepared by incubating protein-A-Sepharose beads (Sigma) with affinity-purified mAb as described above. Five to 10 mg of mAb per ml of bead is the preferred ratio. The mAb bound beads were washed with borate buffer (borate buffer: 100 mM sodium tetraborate, 154 mM NaCl, pH 8.2) until the washes show A280 at based line. Dimethyl pimelimidate (20 mM) in 200 mM triethanolamine was added to covalently crosslink the bound mAb to the protein-A-Sepharose (Schneider et al., J. Biol. Chem, 257:10766 (1982). After incubation for 45 minutes at room temperature on a rotator, the excess crosslinking reagent was removed by washing the beads twice with 10-20 ml of 20 mM ethanolamine, pH 8.2. Between each wash the slurry was placed on a rotator for 5 minutes at room temperature. The beads were washed with borate buffer and with PBS plus 0.02% sodium azide.

The cell lysate (5-10 x 10⁹ cell equivalents) was then slowly passed over a 5-10 ml affinity column (flow rate of 0.1-0.25 ml per minute) to allow the binding of the antigen to the immobilized antibody. After the lysate was allowed to pass through the column, the column was washed sequentially with 20 column volumes of detergent stock solution plus 0.1% sodium dodecyl sulfate, 20 column volumes of 0.5 M NaCl, 20 mM Tris, pH 8.0, and 10 column volumes of 20 mM Tris, pH 8.0. The HLA-A antigen bound to the mAb was eluated with a basic buffer solution (50 mM diethylamine in water). As an alternative, acid solutions such as 0.15-0.25 M acetic acid were also used to elute the bound antigen. An aliquot of the eluate (1/50) was removed for protein quantification using either a colorimetric assay (BCA assay, Pierce) or by SDS-PAGE, or both. SDS-PAGE analysis was performed as described by Laemmli (Laemmli, U.K., Nature 227:680 (1970)) using known amounts of bovine serum albumin (Sigma) as a protein standard.

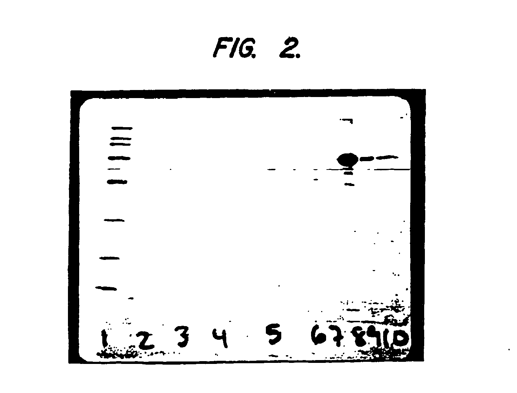

Allele specific antibodies were used to purify the specific MHC molecule. In the case of HLA-A2 and HLA-A3 mAbs BB7.2 and GAPA3 were used respectively. An example of SDS PAGE analysis of purified HLA-A3.2 molecules is shown in Figure 2.

Figure 2 shows SDS-PAGE (12.5%) analysis of affinity purified HLA-A3.2 from the cell line EHM. An affinity column (10 ml) was prepared with protein A-sepharose beads coupled to the monoclonal antibody GAPA3 which is specific for HLA-A3. A detergent lysate of 5x10⁹ cells was passaged over the column and the column was washed extensively. The bound HLA-A3.2 molecules were eluted from the column with 0.15M acetic acid, 50 ml. One ml of the eluate was removed and lyophilized to concentrate the sample. The sample was taken up to 50 *µ*l with Leammli sample buffer and 20 µl were loaded in lane 2. Lane 1 contained molecular weight standards: Myosin, 230 kD; β-galactosidase, 116kD; phosphorylase B, 97.4kD; bovine serum albumin, 66.2kD; ovalbumin, 45kD; carbonic anhydrase, 31kD; soybean trypsin inhibitor, 21.5kD; and lysozyme, 14.4kD. Standard concentrations of bovine serum albumin were run in lanes 8, 10 µg, 9, 3 µg, and 10, 1µg to aid in the estimation of protein yield. For this particular HLA-A3.2 preparation, the estimated yield was approximately 112 µg.

For HLA-A11, A24.1 and A1, an alternative protocol was used whereby anti-HLA-B and C monoclonal antibodies were used to deplete HLA-B and C molecules. The remaining HLA-A molecules were subsequently purified using the W6/32 mAb as described below.

Based on the density of class I expression as indicated by the results of immunofluorescent staining analysis, it is anticipated that average yields of class I antigen isolated from the EBV B cell lines will range from 800-1200 *µ*g per 10¹⁰ cell equivalents.

### Example 2

### An alternative class I purification protocol

HLA-A2.1 molecules were isolated using the mAb B1.23.2 which detects an epitope expressed by HLA-B and C allele molecules, but not by HLA-A antigens. The mAb, W6/32, detects all human class I molecules, including HLA-A, B and C. As mentioned above, these mAbs react well with the B cell lines serving as sources of HLA-A antigens. The B1.23.2 mAb reacts with the various human B cell lines, but fails to react with a mouse cell line that expresses a transfected HLA-A2.1 protein or a chimeric A2.1 mouse K^{b} molecule. It does react with the human cell line, CIR (Alexander, J., et al., Immunogenetics, 29, 380 [1989]), that lacks expression of HLA-A and B molecules, but expresses low levels of HLA-C molecules. This pattern of reactivity illustrates how the B1.23.2 mAb can be used to deplete the B cell lysates of HLA-B and C molecules.

Affinity columns were prepared using the affinity-purified B1.23.2 and W6/32 mAbs, respectively, as described above. The procedures for the preparation of the affinity columns are essentially identical to the procedures described for the preparation of the allele-specific mAb columns described above. The B1.23.2 mAb affinity column was used to deplete the detergent lysates of HLA-B and C molecules using the protocol as described above. The cell lysate depleted of HLA-B and C was then passed over a W6/32 mAb affinity column. The MHC molecule that was eluted from this second passage was the A allele product.

This alternative affinity purification is useful for the purification of any HLA-A allele product, and does not rely on the need for allele-specific mAbs. In addition, it could also be used to isolate any class I molecule type from transfected cell lines.

### Example 3

### Isolation and sequencing of naturally processed peptides

For the HLA-A preparations derived from the base (50 mM diethylamine) elution protocol, the eluate was immediately neutralized with 1 N acetic acid to pH 7.0-7.5. The neutralized eluate was concentrated to a volume of 1-2 ml in an Amicon stirred cell [Model 8050, with YM3 membranes (Amicon)]. Ten ml of ammonium acetate (0.01 M, pH 8.0) was added to the concentrator to remove the non-volatile salts, and the sample was concentrated to approximately 1 ml. A small sample (1/50) was removed for protein quantitation as described above. The remainder was recovered into a 15 ml polypropylene conical centrifuge tube (Falcon, 2097) (Becton Dickinson). Glacial acetic acid was added to obtain a final concentration of 10% acetic acid. The acidified sample was placed in a boiling water bath for 5 minutes to allow for the dissociation of the bound peptides. The sample was cooled on ice, returned to the concentrator and the filtrate was collected. Additional aliquots of 10% acetic acid (1-2 ml) were added to the concentrator, and this filtrate was pooled with the original filtrate. Finally, 1-2 ml of distilled water was added to the concentrator, and this filtrate was pooled as well.

The retentate contains the bulk of the HLA-A heavy chain and β₂-microglobulin, while the filtrate contains the naturally processed bound peptides and other components with molecular weights less than about 3000. The pooled filtrate material was lyophilized in order to concentrate the peptide fraction. The sample was then ready for further analysis.

For HPLC (high performance liquid chromatography) separation of the peptide fractions, the lyophilized sample was dissolved in 50 µl of distilled water, or into 0.1% trifluoracetic acid (TFA) (Applied Biosystems) in water and injected to a C18 reverse-phase narrow bore column (Beckman C18 Ultrasphere, 10 x 250 mm), using a gradient system described by Stone and Williams (Stone, K.L. and Williams K.R., in, Macromolecular Sequencing and Synthesis; Selected Methods and Applications, A.R. Liss, New York, 1988, pp. 7-24. Buffer A was 0.06% TFA in water (Burdick-Jackson) and buffer B was 0.052% TFA in 80% acetonitrile (Burdick-Jackson). The flow rate was 0.250 ml/minute with the following gradient: 0-60 min., 2-37.5% B; 60-95 min., 37.5-75% B; 95-105 min., 75-98% B. The Gilson narrow bore HPLC configuration is particularly useful for this purpose, although other configurations work equally well.

A large number of peaks were detected by absorbance at 214 nm, many of which appear to be of low abundance (Fig. 3). Whether a given peak represents a single peptide or a peptide mixture was not determined. Pooled fractions were then sequenced to determine motifs specific for each allele as described below.

Pooled peptide fractions, prepared as described above were analyzed by automated Edman sequencing using the Applied Biosystems Model 477A automated sequencer. The sequencing method is based on the technique developed by Pehr Edman in the 1950s for the sequential degradation of proteins and peptides to determine the sequence of the constituent amino acids.

The protein or peptide to be sequenced was held by a 12-mm diameter porous glass fiber filter disk in a heated, argon-purged reaction chamber. The filter was generally pre-treated with BioBrene Plus™ and then cycled through one or more repetitions of the Edman reaction to reduce contaminants and improve the efficiency of subsequent sample sequencing. Following the pre-treatment of the filter, a solution of the sample protein or peptide (10 pmol-5 nmol range) was loaded onto the glass filter and dried. Thus, the sample was left embedded in the film of the pre-treated disk. Covalent attachment of the sample to the filter was usually not necessary because the Edman chemistry utilized relatively apolar solvents, in which proteins and peptides are poorly soluble.

Briefly, the Edman degradation reaction has three steps: coupling, cleavage, and conversion. In coupling step, phenylisothiocyanate (PITC) is added. The PITC reacts quantitatively with the free amino-terminal amino acid of the protein to form the phenylthiocarbamyl-protein in a basic environment. After a period of time for the coupling step, the excess chemicals are extracted and the highly volatile organic acid, trifluoroacetic acid, TFA, is used to cleave the PITC-coupled amino acid residue from the amino terminus of the protein yielding the anilinothiazolinone (ATZ) derivative of the amino acid. The remaining protein/peptide is left with a new amino terminus and is ready for the next Edman cycle. The ATZ amino acid is extracted and transferred to a conversion flask, where upon addition of 25% TFA in water, the ATZ amino acid is converted to the more stable phenylthiohydantoin (PTH) amino acid that can be identified and quantified following automatic injection into the Model 120 PTH Analyzer which uses a microbore C-18 reverse-phase HPLC column for the analysis.

In the present procedures, peptide mixtures were loaded onto the glass filters. Thus, a single amino acid sequence usually does not result. Rather, mixtures of amino acids in different yield are found. When the particular residue is conserved among the peptides being sequenced, increased yield for that amino acid is observed.

### Example 4

### Definition of an A3.2 specific motif

There is some ambiguity in the international nomenclature of A3 alleles. The A3.2 allele herein is expressed by cell lines EHM, HO301, and GM3107. This particular subtype is currently referred to as the 3.2 allele (Yang, in Immunobiology of HLA, Vol. 1, Dupont ed., Springer-Verlag, New York pp. 43-44 and 54-55, 1989), or the product of the A*0301 gene (its sequence corresponds to the one published by Strachan, et al., EMBO J., 3:887 (1984), and has been verified by direct cloning and sequencing of the A3 gene found in EHM cell line. The HLA-A3.2 encoded by the A*0301 gene referred to in this document is the commonly expressed HLA-A3 allelic form.

In one case using MAT cells, pooled peptide fractions prepared as described in Example 3 above were obtained from HLA-A3.2 homozygous cell lines, for example, CM3107. The pooled fractions were HPLC fractions corresponding to 7% to 19% CH₃CN. For this class I molecule, this region of the chromatogram was most abundant in peptides. Data from independent experiments were averaged as described below.

The amino acid sequence analyses from four independent experiments were analyzed and the results are shown in Table 5. For each position except the first, the data were analyzed by modifying the method described by Falk et al. to allow for comparison of experiments from different HLA types. This modified procedure yielded quantitative yet standardized values while allowing the averaging of data from different experiments involving the same HLA type.

The raw sequenator data was converted to a simple matrix of 10 rows (each representing one Edman degradation cycle) and 16 columns (each representing one of the twenty amino acids; W, C, R and H were eliminated for technical reasons. The data corresponding to the first row (first cycle) was not considered further because, this cycle is usually heavily contaminated by free amino acids.). The values of each row were summed to yield a total pmoles value for that particular cycle. For each row, values for each amino acid were then divided by the corresponding total yield value, to determine what fraction of the total signal is attributable to each amino acid at each cycle. By doing so, an "Absolute Frequency" table was generated. This absolute frequency table allows correction for the declining yields of each cycle.

Starting from the absolute frequency table, a "relative frequency" table was then generated to allow comparisons among different amino acids. To do so the data from each column was summed, and then averaged. Then, each value was divided next by the average column value to obtain relative frequency values. These values quantitate, in a standardized manner, increases and decreases per cycle, for each of the different sixteen amino acid types. Tables generated from data from different experiments can thus be added together to generate average relative frequency values (and their standard deviations). All standard deviations can then be averaged, to estimate a standard deviation value applicable to the samples from each table. Any particular value exceeding 1.00 by more than two standard deviations is considered to correspond to a significant increase.

The results of the foregoing analysis for HLA-A3.2 were as follows: at position 2, a 2.2-fold increase in valine (V) with lesser increases (1.5-1.7) for structurally similar residues leucine (L) and methionine (M). At position 3, tyrosine (Y) and aspartic acid (D) showed increases in frequency. At position 7 isoleucine (I) was increased, and at position 8 asparagine (N) and glutamine (Q) were increased. At positions 9 and 10, lysine (K) was increased more than 2-fold over the expected random yield.

Cysteine was not modified and thus not detected. PTH-tryptophan coeluted with diphenylurea, and in some experiments, PTH-arginine coeluted with the major derivative of PTH-threonine. Therefore, cysteine and tryptophan are not detectable and arginine is detected only in the absence of threonine.

Previously described MHC structures showed instances of critically conserved residues at position 2 (or 3) and at the C terminus (either position 9 or 10). These residues are referred to as "conserved" residues. The modified data analysis of this invention considered the conserved positions at the N and C terminals.

Thus, the HLA-A3.2 motif should have position two occupied by V, L or M, a length of 9 or 10 amino acids, and a C-terminal position occupied by K.

**TABLE 5) Summary HLA-A3.2 Allele-Specific Motif**

| Position | Conserved Residues |
|---|---|
| 1 | - |
| 2 | V,L,M |
| 3 | Y,D |
| 4 | - |
| 5 | - |
| 6 | - |
| 7 | I |
| 8 | Q,N |
| 9 | K |
| 10 | K |

### Example 5

### Definition of HLA-A1-specific peptide motifs

HLA-A1 molecules were isolated and their naturally processed peptides characterized, as described in Example 3 above. In one case using MAT cells, pooled fractions corresponding to 19% to 50% CH₃CN were used. As in the preceding example, residues showing at any given position except the first position, at least a two standard deviation increase over the random expected yield were identified and shown in Table 6. On the basis of these data, only Serine (S) and Threonine (T) were increased at position two. At position. 3, aspartic acid (D) and glutamic acid (E) were elevated and at position 9 and 10 tyrosine (Y) showed a marked increase. Other increases noted were proline (P) at position 4 and leucine (L) at position 7. Therefore, the motifs for HLA-A1 based on these data would have residues at position 2 occupied by S or T, a peptide length of 9 or 10 amino acids and a C-terminal residue of Y. Alternatively, another motif would comprise a D or E at position 3 together with a C terminal residue of Y.

**TABLE 6 Summary HLA-A1 Allele-Specific Motif**

| Position | Conserved Residues |
|---|---|
| 1 | - |
| 2 | S, T |
| 3 | D,E |
| 4 | P |
| 5 | - |
| 6 | - |
| 7 | L |
| 8 | - |
| 9 | Y |

### Example 6

### Definition of HLA-A11 allele-specific peptide motifs

HLA-A11 motifs were defined by amino acid sequence analysis of pooled HPLC fractions, in one case corresponding to 7% to 45% CH₃CN of fractionated peptides eluted from HLA-A11 molecules purified from the cell line BVR. On the basis of the data presented in Table 7, a motif for A11 consists of a conserved residue at position 2 of threonine (T) or valine (V), a peptide length of 9 or 10 amino acids, and a C-terminal conserved residue of lysine (K). At position 3 increases in methionine (M) and phenylalanine (F) were also seen and at position 8 glutamine (Q) was increased.

**TABLE 7 Summary HLA-A11 Allele-Specific Motif**

| Position | Conserved. Residues |
|---|---|
| 1 | - |
| 2 | T,V |
| 3 | M,F |
| 4 | - |
| 5 | - |
| 6 | - |
| 7 | - |
| 8 | Q |
| 9 | K |
| 10 | K |

### Example 7

### Definition of HLA-A24.1 Specific Peptide Motifs

ELA-A24.1 allele-specific motifs were defined by amino acid sequence analysis of pooled fractions in one case corresponding to 7% to 19% CH₃CN of HPLC fractionated peptides eluted from HLA-A24.1 molecules purified from the cell line KT3. On the basis of the data presented in Table 8 a motif for HLA-A24.1 consists of a conserved residue at position 2 occupied by tyrosine (Y), a peptide length of 9 or 10 amino acids, and a C-terminal conserved residue of phenylalanine (F) or leucine (L). Increases were also observed at several other positions: isoleucine (I) and methonine (M) at position 3; aspartic acid (D), glutamic acid (E), glycine (G), lysine (K) and proline (P) at position 4; lysine (K), methonine (M) and asparagine (N) at position 5; valine (V) at position 6; asparagine (N) and valine (V) at position 7; and, alanine (A), glutamic acid (E), lysine (K), glutamine (Q) and serine (S) at position 8.

**Table 8 Summary HLA-A24.1 Allele-Specific Motif**

| Position | Conserved Residues |
|---|---|
| 1 | - |
| 2 | Y |
| 3 | I,M |
| 4 | D,E,G,K,P |
| 5 | L,M,N |
| 6 | V |
| 7 | N,V |
| 8 | A,E,K,Q,S |
| 9 | F,L |
| 10 | F,A |

### Example 8

### Identification of immunogenic peptides

Using the motifs identified above for various MHC class I allele amino acid sequences from various viral and tumor-related proteins were analyzed for the presence of these motifs. Sequences for all of the target antigens were obtained from the GenBank data base (Release No. 71.0; 3/92). The identification of motifs was done using the "FINDPATTERNS" program (Devereux, Haeberli and Smithes (1984). Nucleic Acids Research 12 (1); 387-395).

The amino acid sequence or the nucleotide sequence encoding products was obtained from the GenBank database. In the cases of Human Papilloma Virus (HPV), Prostate Specific antigen (PSA), p53 oncogene, Epstein Barr Nuclear Antigen-1 (EBRA-1), and c-erb2 oncogene (also called HER-2/neu), and Melanoma Antigen-1 (MAGE-1), a single sequence exists.

In the cases of Hepatitis B Virus (HBV), Hepatitis C Virus (HCV), and Human Immunodeficiency Virus (HIV) several strains/isolates exist and many sequences have been placed in GenBank.

For HBV, binding motifs were identified for the adr, adw and ayw types. In order to avoid replication or identical sequences, all of the adr motifs and only those motifs from adw and ayw that are not present in adr were added to the list of peptides.

In the case of HCV, a consensus sequence from residue 1 to residue 782 was derived from 9 viral isolates. Motifs were identified on those regions that had no or very little (one residue) variation between the 9 isolates. The sequences of residues 783 to 3010 from 5 viral isolates were also analyzed. Motifs common to all the isolates were identified and added to the peptide list.

Finally, a consensus sequence for HIV type 1 for North American viral isolates (10-12 viruses) was obtained from the Los Alamos National Laboratory database (May 1991 release) and analyzed in order to identify motifs that are constant throughout most viral isolates. Motifs that bear a small degree of variation (one residue, in 2 forms) were also added to the peptide list.

Several motifs for each allele shown below were used to screen several antigens. Protein E6 of human papilloma virus (HPV) type 16 using motifs from all of the alleles disclosed above are shown (Table 9). Protein E7 of HPV type 18 was also searched for motifs from all alleles (Table 9) Melanoma antigens MAGE 1, 2 and 3 were searched for motifs from all alleles (Table 10). The antigen PSA was searched for motifs from all alleles (Table 11). Finally, core and envelope proteins from hepatitis C virus were also searched (Table 12). In the tables and the description of the motifs, the conventional symbol letter for each amino acid was used. The letter "X" represents a wild card character (any amino acid).

The following motifs were screened in the present search:

**For HLA-A1 (A*0101) :**

| | |
|---|---|
| 1 | XSXXXXXXY |
| 2 | XSXXXXXXXY |
| 3 | XTXXXXXXY |
| 4 | XTXXXXXXXY |
| 5 | XXDXXXXXY |
| 6 | XXDXXXXXXY |
| 7 | XXEXXXXXY |
| 8 | XXEXXXXXXY |

**For HLA-A3.2 (A*0301)**

| | |
|---|---|
| 1 | XVXXXXXXK |
| 2 | XVXXXXXXXK |
| 3 | XLXXXXXXK |
| 4 | XLXXXXXXXK |
| 5 | XMXXXXXXK |
| 6 | XMXXXXXXXK |

**For HLA-A11 (A*1101):**

| | |
|---|---|
| 1 | XTXXXXXXK |
| 2 | XTXXXXXXXK |
| 3 | XVXXXXXXK |
| 4 | XVXXXXXXXK |

**For HLA-A24.1 (A*2401):**

| | |
|---|---|
| 1 | XYXXXXXXF |
| 2 | XYXXXXXXXF |
| 3 | XYXXXXXXL |
| 4 | XYXXXXXXXL |

**Peptides with MHC Class I Binding Motifs Table 9**

| AA Position | Sequence | Antigen | HLA molecule |
|---|---|---|---|
| | 30IHDIILECVY | HPV16.E6 | Al |
| | 69 VCDKCLKFY | HPV16.E6 | A1 |
| | 77 YSKISBYRHY | HPV16.E6 | A1 |
| | 80 ISEYRHYCY | HPV16.E6 | A1 |
| | 92 GTTLEQQYNK | HPV16.E6 | A11 |
| | 93 TTLEQQYNK | HPV16.E6 | A11 |
| | 106 LLIRCINCQK | HPV16.E6 | A3 |
| | | | |
| | 2 HGDTPTLHEY | HPV16.E7 | A1 |
| | 16 QPETTDLYCY | HPV16.E7 | A1 |
| | 44 QAEPDRAHY | HPV16.E7 | A1 |
| | 89 IVCPICSQK | HPV16.E7 | A3, A11 |
| | | | |
| | 3 RFEDPTRRPY | HPV18.E6 | A1 |
| | 4 FEDPTRRPY | HPV18.E6 | A1 |
| | 25 LQDIEITCVY | HPV18.E6 | A1 |
| | 41 LTEVFEFAFK | HPV18.E6 | A11 |
| | 72 YSRIRELRHY | HPV18.E6 | A1 |
| | 84 SVYGDTLEK | HPV18.E6 | A3, A11 |
| | 101 LLIRCLRCQK | HPV18.E6 | A3 |
| | | | |
| | 59 HTMLCMCCK | HPV18.E7 | A11 |

Human Papilloma Virus 16 and 18 (E6 and E7 Proteins)

**Peptides with MHC Class I Binding Motifs Table 10**

| AA Position | Sequence | Antigen | HLA molecule |
|---|---|---|---|
| | 2 SLEQRSLHCK | MAGE 1 | A3 |
| | 96SLFRAVITK | MAGE 1 | A3 |
| | 96SLFRAVITKK | MAGE 1 | A3 |
| | 108 DLVGFLLLK | MAGE 1 | A3 |
| | 128 MLESVIKNYK | MAGE 1 | A3 |
| | 128MLESVIKNY | MAGE 1 | A1 |
| | 152 QLVFGIDVK | MAGE 1 | A3 |
| | 161 EADPTGHSY | MAGE 1 | A1 |
| | 182 LLGDNQIMPK | MAGE 1 | A3 |
| | 215 WEELSVMEVY | MAGE 1 | A1 |
| | 223 VYDGREHSAY | MAGE 1 | A1 |
| | 238 LLTQDLVQER | MAGE 1 | A3 |
| | 239 LTQDLVQEK | MAGE 1 | A11 |
| | 239 LTQDLVQEKY | MAGE 1 | A1 |
| | 240 TQDLVQEKY | MAGE 1 | A1 |

Melanoma Antigen MAGE 1

**Peptides with MHC Class I Binding Motifs Table 11**

| AA Position | Sequence | Antigen | HLA molecule |
|---|---|---|---|
| | 21 IVGGWECEK | PSA | A3, A11 |
| | S7 LTAAHCIRNK | PSA | A11 |
| | 88 VSHSFPHPLY | PSA | A1 |
| | 95 PLYDMSLLK | PSA | A3 |
| | 178 DVCAQVHPQK | PSA | A3, A11 |
| | 182 QVHPQKVTK | PSA | A3 , A11 |
| | 236 PSLYTKVVHY | PSA | A1 |
| | 239 YTKVVHYRK | PSA | A11 |
| | 241 KVVHYRKWIK | PSA | A3, A11 |
| | 242 VVHYRKWIK | PSA | A3, A11 |

Prostate Specific Antigen (PSA)

**Peptides with MHC Class I Binding Motifs Table 12**

| AA Position | Sequence | Antigen | HLA molecule |
|---|---|---|---|
| | 2 STNPKPQRK | HCV | A11 |
| | 14 NTNRRPQDVK | HCV | A11 |
| | 43 RLGVRATRR | HCV | A3 |
| | 302 VQDCNCSIY | HCV | A1 |
| | 556 WMNSTGFTK | HCV | A3 |
| | 605 LTPRCMVDY | HCV | A1 |
| | 626 FTIFKIRMY | HCV | A1 |

Hepatitis C Virus (Consensus Sequence)

### Example 9

### Quantitative HLA class I binding assay

To verify that motif-containing peptide sequences are indeed capable of binding to the appropriate class I molecules, specific binding assays were established. HLA-A3.2 molecules were purified from GM3107 EBV cells by affinity chromatography using the GAPA3 mAb (anti-A3) to isolate A3.2. Prior to the step, the lysate was depleted of HLA B and C molecules by repeated passages over a B1.23.2 column (this antibody is B,C specific) generally as described in Example 2, above.

As a radiolabeled probe, the peptide 941.12 (KVFPYALINK), containing an A3.2 motif, was used. This peptide contains the anchor residues V₂ and K₁₀, associated with A3.2-specific binders, described above. A Y residue was inserted at position 5 to allow for radiolodination. Peptides were labeled by the use of the Chloramine T method Buus et al., Science 235:1352 (1987), which is incorporated herein by reference.

A dose range of purified A3.2 was incubated with 10 nM of 941.12 at pH 7.0 and 23°C, in presence of a protease inhibitor cocktail (1 mM PMSF, 1.3 mM 1.10 phenanthroline, 73 µM pepstatin A, 8 mM EDTA, and 200 µM N aₚ-tosyl-L-lysine chloromethyl ketone (TLCK)), in presence of 1 µM purified human β2 microglobulin. After two days, the % bound radioactivity was measured by gel filtration over TSK 2000 columns as previously described for class II peptide binding assays in Sette et al., in Seminars in Immunology, Vol. 3, Gefter, ed. (W.B. Saunders, Philadelphia, 1991), pp 195-202, which is incorporated herein by reference. (see, Fig. 4). Good binding (in the 60 to 100% range) was observed for A3.2 concentrations ranging between 35 and 300 nM. 30% binding was observed at 15 nM A3.2.

To minimize A3.2 usage and to increase the sensitivity of the assay, a concentration of 5-10 nM A3.2 was selected for further assays. In the experiment shown in Fig. 5, 7nM A3.2 and an equivalent concentration of radiolabeled 941.12 were incubated using the conditions described above and in the presence of a dose range of three peptides (HBc 18-27 (924.07), a Prostate Specific Antigen peptide (939.01), and HIV nef 73-82 (940.03)). It was found that peptide 940.03 inhibited strongly, with a 50% inhibitory concentration (IC50%) of 22 nM, while a weaker inhibition was observed with peptide 939.01 (IC50% 940 nM). Finally, peptide 924.07 did not show any inhibition up to the 30 µM level. Thus, it is concluded that peptides 940.03 and 939.01 are high and intermediate affinity binders, respectively, while peptide 924.07 is classified as a low affinity or negative binder.

Throughout this disclosure, results have been expressed in terms of IC50's. Given the conditions in which the assays are run (i.e., limiting MHC and labeled peptide concentrations), these values approximate K_{D} values. It should be noted that IC50 values can change, often dramatically, if the assay conditions are varied, and depending on the particular reagents used (e.g., Class I preparation, etc.). For example, excessive concentrations of MHC will increase the apparent measured IC50 of a given ligand.

An alternative way of expressing the binding data, to avoid these uncertainties, is as a relative value to a reference peptide. The reference peptide is included in every assay. As a particular assay becomes more, or less, sensitive, the IC50's of the peptides tested may change somewhat. However, the binding relative to the reference peptide will not change. For example, in an assay run under conditions such that the IC50 of the reference peptide increases 10-fold, all IC50 values will also shift approximately 10-fold. Therefore, to avoid ambiguities, the assessment of whether a peptide is a good, intermediate, weak, or negative binder should be based on it's IC50, relative to the IC50 of the standard peptide.

If the IC50 of the standard peptide measured in a particular assay is different from that reported in the table, then it should be understood that the threshold values used to determine good, intermediate, weak, and negative binders should be modified by a corresponding factor. For example, if in an A2.1 binding assay, the IC50 of the A2.1 standard (941.01) were to be measured as 8 nM instead of 5 nM, then a peptide ligand would be called a good binder only if it had an IC50 of less than 80 nM (i.e., 8nM x 0.1), instead of the usual cut-off value of 50 nM.

The experimental system herein described can be used to test binding of large numbers of synthetic peptides to a variety of different class I specificities. Specific binding assays can be performed as follows.

### HLA-A11-specific assay

The cell line BVR was used as a source of HLA. The dependency of the binding on MHC concentration in presence or absence of β₂M are shown in Fig. 6, while Fig. 7 depicts the dose dependency of the inhibition by excess unlabeled ligand. Finally, Fig. 8 shows a Scatchard analysis experiment. Values of apparent kD of -6 nM and of 10% active receptor were obtained, and were remarkable for their similarity to the values obtained for A2.1 and A3.2. The sequence of the peptide used as a radiolabeled probe (940-06) is AVDLYHFLK.

### HLA-A1-specific assay

In this case, the EBV cell line Steinlin was used as a source of purified HLA. The same protocol previously applied to purification of other HLA alleles (i.e., depletion of B, C molecules by a B1.23.2 mAb column, followed by purification of A molecules by means of a W632 mAb column) was utilized. On the basis of the pool sequencing data, consensus peptides were synthesized, directly radiolabeled, and tested for HLA binding using the standard protocol (1 mM β₂M, 2 days RT incubation in presence of protease inhibitors). A graph illustrating the relationship between % binding and µM input HLA A1 is shown in Fig. 9. From the data, it was concluded that in analogy with what was observed for HLA A2, 3, and 11, as little as 30 nM are sufficient to obtain ^{∼}10% binding. The sequence of the peptide used as a radiolabeled probe (944.02) is YLEPAIAKY. In the next set of experiments, the specificity of the assay established was verified by its inhabitability by excess unlabeled peptide. The IC50% was measured (Fig. 10) as 20 nM. Further Scatchard analysis (Fig. 11) verified that the apparent K_{D} of the interaction corresponded to 21 nM, with a % of active receptor corresponding to 5.1%.

### HLA-A24 specific assay

HLA A24 molecules were purified from the KT3 EBV cell line. In this case, two consensus peptides whose sequences were based on the pool sequencing data have been synthesized. Their sequences are: 979-01, AYIDNVYKF and 979.02, AYIDNYNKF. The results of experiments in which the % bound of these two peptides as a function of input MHC was measured are shown in Fig. 12. In both cases, 10-15% binding was obtained with as little as 20-50 nM MHC. Cold inhibition experiments (Fig. 13), limiting MHC concentrations, revealed that the binding was readily inhibitable by excess unlabeled peptide, with an apparent K_{D} of 30 and 60 nM, respectively. Further Scatchard experiments verified values of 136 nM and 28 nM, respectively. The apparent % of available receptor (active MHC) were 8.3% and 7.4%, respectively (Fig. 9a and b). On the basis of these data, peptide 979.02 was arbitrarily selected as standard label indicator for A24 assays. Furthermore, on the basis of the data herein described, we also conclude that the goal of establishing an A24-specific binding assay has been accomplished. In conclusion, specific assays for the five major HLA alleles have been described.

### Example 10

### Expansion of HLA A Motifs

Establishing *in* vitro binding assays allows one to readily quantitate *in vitro* the binding capacity of various synthetic peptides to the various alleles of interest (HLA A1, A2, A3, A11, and A24). This allows verification of the correctness of the motifs by means of peptides carrying the various HLA A motifs for their capacity to bind purified HLA molecules. Typically, peptides were synthesized with specific HLA motifs embedded in a neutral backbone composed of only alanine residues. In some cases, a K residue was also introduced within the sequence, with the purpose of increasing solubility. The use of such "neutral" poly A backbones, as applied to the case of class II molecules, has been described in detail, for example, by Jardetzky et al. (Jardetzky et al., EMBO J. 9(6):1797,1990).

For example, in the case of A3.2, a motif has been defined with a hydrophobic residue in position 2 and a positive charge (K) in position 9. Thus, to verify that the presence of these two anchor residues would allow, in the context of a poly A backbone, for A3.2 binding, the poly A analog with the sequence AMAAAAAAK was synthesized (Table 13).

Similarly, other peptides carrying other HLA motifs were also synthesized and tested for HLA binding. It was found that in all cases, the presence of the specific HLA motifs was conducive to binding to the relevant HLA allele, with estimated K_{D} comprised of between 125 and 2.8 nM. In most cases, the binding was also absolutely specific, in that no binding was detected to irrelevant alleles. Only two exceptions to this general rule were observed. Firstly, A3. and A11 peptides crossreacted extensively with each other, perhaps as could have been expected by the fact that the motifs for these two alleles are remarkably similar. Second, some A1 peptides crossreacted, albeit with much lower affinities, on A11 and A3.2.

To further define the structural requirements for the interaction between peptide epitopes and various class I alleles of interest, analogs of 10 residues in length of some of the 9 residue peptides shown in Table 13 were synthesized (Table 14). These analogs were generated by inserting an additional Ala residue within the poly A backbone, so that the anchor residues are not located in positions 2 and 10 (as opposed to 2 and 9 in the previous table). The results obtained illustrate that motifs of 10 residues are also capable of specifically binding to the relevant class I alleles, albeit with a slightly lower efficiency.

In summary, these data confirm that both 9-mer and 10-mer peptides which contain the appropriate motifs can bind HLA. On the basis of these data, 8-mer or 11-mer peptides should also be capable of binding, even if perhaps with lower affinities.

The data described above show that the presence of certain residues in the anchor positions does allow (at least in a "neutral" poly A backbone) for HLA binding. To investigate to what degree other amino acids (for example, chemically related amino acids) might be tolerated in these crucial anchor positions, analogs of some of the poly A peptides from Table 13 were synthesized, in which the residue present in position 2 (or 3) or 9 was varied. The results of this analysis are shown in Tables 15-19.

In the case of A3.2 (Table 15), in position 2, L, M, I, V, S, A, T, and F were found to be preferred (binding ≥ 0.1 relative to previously defined anchor residues), while C, G, and D were permitted (binding ≥ 0.01 to 0.1 relative to previously defined anchor residues). The substiution of E, because of its similarity to D, in this position should also be tolerated. In position 9, K, R, and Y were preferred. Because of a similarity in nature, that H and F should also be preferred. No other residue was tolerated in position 9 for A3 binding.

In the case of A11 (Table 16), the preferred residues in position 2 were L, M, I, V, A, S, T, G, N (L and Q by similarity). Tolerated were C, F, D (and E by similarity). In position 9, K was preferred and R was tolerated. H should also be tolerated by similarity.

In the case of A24 (Table 17), Y and F were preferred in position 2 (and W by similarity); no other residue was tolerated. In position 9, F, I, and L were preferred (and W and M by extension). No other residue was tolerated.

In the case of A1, three different anchor residues had previously been defined. The results shown in the preceding section show that they act independently of each other (i.e., that two out of three anchors would be sufficient for binding). This is indeed the case. For this reason, analogs containing two anchors were synthesized to define what residues might be preferred or tolerated in each position. The data shown in Table 18 show that in position 2, T, S, and M are preferred, and no other residue is tolerated. In position 3 (Table 19), D and E are preferred, and A,S (and T by similarity) are tolerated. Finally, in position 9, only Y is preferred, and no other residue appears to be tolerated (Table 19).

Thus, on the basis of the data, it is concluded that peptides carrying any combination of two preferred residues can bind. Peptides containing "imperfect" motifs, i.e., carrying a preferred residue at one position and a tolerated one at the other anchor position, should also be capable of binding, even if with somewhat lower affinity. Using the motifs of this invention for various MHC class I alleles amino acid sequences from various viral and tumor-related proteins were analyzed for the presence of motifs. The results of this motif analysis is shown in Table 23 a - k.

### Example 11

### Validation of HLA Peptide Binding Motifs with an Unbiased Set of HPV 16 Peptides.

Human Papillomaviruses (HPVs) are implicated in the etiology of cervical cancer (Pfister, H. (1974) Biology and biochemistry of papillomaviruses, Rev. Physiol. Biochem. 99:111; zur Hausen, H. (1991). Human papillomaviruses in the pathogenesis of anogenital cancer. Virology. 184:9) and in up to 10% of total mortality due to cancer worldwide (zur Hausen, H. (1991). Viruses in Human Cancers. Science, 254:1167). Cervical cancer is the second most common cause of cancer-related death in females worldwide (Parkin, D. M., Laara, E., and Muir, C. S. (1988), Estimates of the worldwide frequency of sixteen major cancers in (1980). Int. J. Cancer. 41:184). HPV DNA is present in more than 90% of the cervical carcinomas and predominantly of the HPV 16 genotype (Resnick, R. M., Cornelissen, M. T., Wright, D, K., Eichinger, G. H., Fox, H. S., ter Schegget, J., and Manos, M. M. (1990). Detection and typing of human papillomavirus in archival cervical cancer specimens by DNA amplification with consensus primers. J. Natl. Cancer Inst*;* Van den Brule, A. J. C., Walboomers, J. M. M., du Maine, M., Kenemans, P., and Meijer, C. J. L. M. (1991). Difference in prevalence of human papillomavirus genotypes in cytomorphologically normal smears is associated with a history of cervical intraepithetal neoplasia. Int. J. Cancer. 48:404). The ability of HPV 16 early region 6 and 7 (E6, E7) open reading frames to in vitro immortalize rodent cells (Yasumoto, S., Burkhardt, A.L., Doniger, J., and DiPaolo, J.A. (1986). Human Papillomaviruses type 16 DNA induced malignant transformation of NIH3T3 cells. J. Virol. 57:572) and human keratinocytes (Pirisi. L., Yasumoto, S., Feller, M., Doniger, J., and DiPaolo, J.A. (1987). Trans-formation of human fibroblasts and keratinocytes with human papillomavirus type 16 DNA. J. Virol, 61:1061) and to transform human fibroblasts (Smits, H. L., Raadsheer, E., Rood, I., Mehendale, S., Slater, R. M., van der Noordaa, J., and ter Schegget, J. (1988). Induction of anchorage-independent growth of human embryonic fibroblasts with a deletion in the short arm of chromosome 11. J. Virol. 62:4538) suggests direct involvement of HPV 16 in the multi-step process of cervical carcinogenesis,

In general T cell immunity, in particular mediated by cytotoxic T lymphocytes (CTL) is important in the defense against virus-induced tumors (Melief, C. J. (1992). Tumor eradication by adoptive transfer of cytotoxic T lymphocytes. Adv. Cancer Res. 58:143; Melief, C. J., and Kast, W. M. (1992) Lessons from T cell responses to virus induced tumors for cancer eradication in general. Cancer Surv. 13:81). Recently in a mouse model, it was reported that some degree of protection against HPV 16 E7 expressing tumors can be obtained with CTL after immunization with HPV 16 E7 expressing cells (Chen. L., Thomas, E, K., Hu, S. L., Hellström, I., and Hellstrom, K. E. (1991). Human papillomavirus type 16 nucleoprotein E7 is a tumor rejection antigen. Proc. Natl. Acad. Sci. 88:110; Chen, L., Ashe, S., Brady, W. A., Hellstrom, I., Hellström, K. E., Ledbetter, J. A,, McGowan, P., and Linsley, P. S. (1992). Costimulation of Antitumor immunity by the B7 counterreceptor for the T lymphocyte molecules CD28 and CTLA-4. Cell. 71:1093). In vivo protection by CTL was recently shown in mouse models in which synthetic peptides containing CTL epitopes were used for efficient priming of mice against virus infections (Schulz, M., Zinkernagel, R. M., and Hengarter, H. (1991). Peptide-induced antiviral protection by cytotoxic T cells. Proc. Natl. Acad. Sci. USA 88:991; Kast, W. M., Roux, L., Curren, J., Blom, H. J. J., Voordouw, A. C., Meleon, R. H., Kolakofski, D., and Melief, C. J. M. (1991). Protection against lethal Sendai virus infection by in vivo priming of virus-specific cytotoxic T lymphocytes with an unbound peptide. Proc. Natl. Acad. Sci. USA. 88:2283). Moreover in a mouse model it has now been shown that complete protection against HPV 16 induced tumors can be achieved by peptide vaccination with a CTL epitope derived from the viral oncogene E7().

The HPV 16 E6 and E7 gene products are the most desirable target antigens for vaccination against HPV 16 induced tumors. Both are retained and highly expressed in HPV 16-transformed cancer cells in vivo (Baker, C. J., Phelps, W. C., Lindgren, V., Braun, M. J., Gonda, M. A., and Howley, P. M. [1987]. Structural and transcriptional analysis of human papillomavirus type 16 sequences in cervical carcinoma cell lines. J. Virol. 61:962; Smotkin, D., and Wettstein, F. O. [1986]. Transcription of human papillomavirus type 16 early genes in a cervical cancer and cancer-derived cell line and identification of the E7 protein. Proc. Natl. Acad. Sci. USA. 83:4680) and involved in the induction and maintenance of cellular transformation in vitro (Crook, T., Morgenstern, J. P., Crawford, L., and Banks, L. [1989]. Continued expression of HPV-16 E7 protein is required for maintenance of the transformed phenotype of cells co-transformed by HPV-16 plus. EJ ras. EMBO J. 8:513; Hawley-Nelson, P., Vousden, K. H., Hubbert, N. L., Lowy, D. R., and Schiller, J. T. [1989]. HPV 16 E6 and E7 proteins cooperate to immortalize human foreskin keratinocytes. EMBO J. 8:3905). Dependence of in vitro growth of cell lines derived from cervical cancers on the expression of E6 and E7 emphasizes involvement of these oncogenes in maintenance of the phenotype of cervical carcinoma cell lines (Von Knebel Doeberitz, M,, Bauknect, T., Bartch, D., and zur Hausen, H. [1991]. Influence of chromosomal integration on glucocorticoid-regulated transcription of growth-stimulation papillomavirus genes E6 and E7 in cervical carcinoma cells. Proc. Natl. Acad. Sci. USA. 88:1411). To determine the CTL epitopes and potential vaccine candidates of HPV 16 for humans, we screened peptides spanning the HPV 16 E6 and E7 protein sequences for their ability to bind to the most frequent human MHC molecules, namely HLA-A1, A3.2, A11.2 and A24. Combined these five alleles will cover about 90% of the world population (Dupont, B., ed. (1987). Immunology of HLA Vol. I -- Histocompatibility Testing. Springer-Verlag, New York).

A complete set of 240 overlapping synthetic peptides of 9 aa length and 8 aa overlap covering the entire HPV 16 E6 and E7 oncogene sequences were synthesized. The peptides were tested for their ability to bind the aforementioned HLA molecules in the binding assay described above. The results of this analysis show the relative affinity of all peptides for the respective HLA alleles and reveal the possible candidate CTL epitopes for use in peptide based vaccines for humans in tables 20(a)-(d).

The results confirm that peptide binding motif described in this invention for the aforementioned HLA alleles predict which peptide of a protein is likely to bind into the groove of a specified HLA molecule. Since we used a large and unbiased set of peptides, the results of the peptide binding analyses were used to evaluate the value of these motifs both for their predictive capacities and the necessity to have particular anchor aa residues on positions 2, (3) and 9 in a peptide.

*Peptides.* Peptides were generated by solid phase strategies on a multiple peptide synthesizer (Abimed AMS 422) by repeated cycles in which addition of Fmoc protected amino acids to a resin of polystyrene was alternated with a Fmoc-deprotection procedure (Gausepohl, H., Kraft, M., Boulin, Ch., and Frank, R. W. [1990]. Automated multiple peptide synthesis with BOP activation. in Proc. of the 11th American peptide symposium. J. E. Rivier and G. R. Marshall, Ed.. ESCOM, Leiden. 1003-1004). The peptides all carrying a COOH group at the C-terminal end, were cleaved from the resin and side chain protective groups were removed by treatment with aqueous TFA. Peptides were analyzed by reversed phase HPLC lyophilized and dissolved at a concentration of 1 mg/ml in phosphate-buffered saline with 3% DMSO (Sigma, St. Louis, MO 63175) before use. Once dissolved, the peptides were stored at -70° C. Since cysteine containing peptides are susceptible to (air) oxidation during synthesis and handling, these peptides were synthesized with an alanine instead of a cysteine.

*Identification* of *peptides from HPV* 16 *E6* and E7 proteins that bind to different *HLA-A* alleles. A complete set of 240 peptides of 9 aa in length and overlapping by 8 aa, covering the sequences of the entire HPV 16 E6 and E7] proteins, was tested for binding to 5 different HLA-A molecules.

The results of this analysis are depicted in tables. 20(a)-(d). Table 20(a) describes the peptides of HPV 16 that bound to HLA-AL molecules. All peptides were tested. Listed are only peptides yielding ratio values of ≥ 0.001. It can be seen that 2 peptides bound with high affinity to this molecule (>0.1), 6 with intermediate affinity (0.1-0.01) and 1 with low affinity (0.01-0.001). Peptides were ranked by ratio value to allow comparison of data obtained in different experiments. To calculate the concentration of a peptide necessary to yield a 50% inhibition dose (IC₅₀) one has to divide the value of the standard IC₅₀ by the ratio. For example, peptide E6-80 has an IC₅₀ of 23 nM (81/3.5).

Table 20(b) describes the peptides that bound to HLA-A3.2 molecules. Seven peptides were identified as high affinity binders, 6 as intermediate affinity binders and 13 as low affinity binders. Table 20(c) describes the peptides that bound to HLA-A11.2 molecules. Six high affinity peptides were identified, 4 intermediate affinity binders and 10 low affinity binders. Two high affinity binding peptides (E6-59 IVYRDGNPY and E6-80 ISEYRHYAY) and two weak affinity binding peptides with a Y at the 9th position (E6-42 QQLLRREVY, E6-69 VADKALKFY) were identified for HLA-All.2 Considering the high binding strength of the first two peptides and the similarity between the HLA-A11.2 motif and the HLA-A3.2 motif in which Y's are preferred at the 9th aa position, tyrosines should be included at the 9th position in the HLA-A11.2 motif. Comparing Tables 21(b) and (c) it is clear that there is a large overlap of peptides that bound to both A3.2 and A11.2 molecules. Eighteen out of 28 E6 and E7 peptides binding to these two HLA molecules overlapped and only 8 peptides were unique for HLA-A3.2 and 2 peptides unique for HLA-A11.2.

Finally, table 20(d) describes the peptides that bound to HLA-A24 molecules. Here 2 peptides were identified as high affinity binding peptides, 5 as intermediate affinity binding peptides and 5 as low binding peptides. One high affinity peptide (E6-72 KALKFYSKI) and one intermediate affinity peptide (E7-49 RAHYNIVTF) were identified, indicating that an A at the second position should be allowed in the HLA-A24 motif. All these inclusions are indicated in Table 20-e. In analyzing tables 1-5 it can be concluded that between 2 and 7 high affinity binding peptides were identified for all of the tested HLA-A molecules. Occasionally some peptides were binding to more alleles. Three peptides (E6-7, E6-37 and E6-79), bound to HLA-A2.1, A3.2 and A11.2. One peptide (E6-38) bound to HLA-A3.2, A11.2 and A24 and two peptides (E6-69 and E6-80) bound to HLA-A1, A3.2 and A11.2. But these crossreactive peptides bound only weakly to one or more of the different HLA molecules. In general, however, it can be concluded that, except for HLA-A3.2 and HLA-A11.2 molecules, almost all HLA molecules bind unique peptides.

### Validation of HLA-A peptide binding motifs with an unbiased set of HPV 16 E6 and E7 peptides.

We analyzed how well the motifs for anchor positions described in this invention predicted the binding of a peptide, and also the reverse: how well binding peptides' followed the identified motifs. For this, peptides were ranked as high binders, intermediate binders, weak binders, and negative binders and for each peptide the motif prediction based on the anchor motif rules of Table 6 were analyzed. The overall efficiency of the 2, (3), and 9 anchor motifs was then calculated and this is summarized in table 20(e). it can be concluded that the motif described above for the different HLA-A molecules are quite accurate. One hundred percent of the HLA-A1, A3.2, and A24 high binders would be predicted as well as 67% of the HLA-11.2. Even for the intermediate binders between 40 and 100% would be predicted depending on the HLA-A molecule analyzed. Furthermore, the percent of weak binding peptides that would be predicted is low and the percent of those peptides that were predicted to bind but actually did not bind is very low for all these alleles.

Analyzed differently, of the 12 peptides predicted to bind to HLA-A1 actually 5 bound with high or intermediate affinity. This indicates that only a few peptides would have to be made to find these potential CTL epitopes. The figures for HLA-A3.2, A11.2, and A24 were 10/32, 7/26, and 4/7, respectively. This implies that the predictive value for all of these alleles is good. Besides a small number of peptides that had not been predicted by the recently described motifs, the (-) in tables 21(a) - (d), a number of peptides that were predicted by the 2, (3) and 9 anchor motifs did not bind, indicating that having the right anchor residues is not always sufficient for binding and implicating that non-anchor residues can make negative contributions to the binding of a peptide.

### Example 12

### Presence of a Motif is Necessary But Not Sufficient for High Affinity Class I Binding

To investigate further how the presence of different motifs might influence the capacity of different peptides to bind to the relevant HLA alleles, the sequences of various potential target molecules were scanned for the presence of motif-containing peptides. The peptides thus identified were synthesized and tested for binding. It was found (Table 20) that in the case of A3.2, only 39 (19%) of the 205 peptides bound with high affinity in the 1 to 50 nM range. 22.4% of them bound with intermediate affinities (in the 50 to 500 nM range), while 34.6% bound weakly (in the 500 nM to 50 *µ*M range). Finally, 23.9% of them did not bind at all, at least up to the 50 µM level. In the case of A11, 33 (33%) of the 100 peptides bound with high affinity in the 1 to 50 nM range. 35% of them bound with intermediate affinities (in the 50 nM range), while 24% bound weakly (in the 500 nM to 50 *µ*M range). Finally, 8% of them did not bind at all, at least up to the 50 µM level.

Similar results were also obtained (data not shown) in the case of A1 and A24.

The same type of analysis were also performed in the case of 10-mer peptides carrying either the A3.2, and A11 motifs (Tables 22(a) and (b)). It was found that in these cases, the frequency of good binders was even lower (17.5%, and 29.8%, respectively). These data confirm the fact that motif-containing 10-mer peptides can indeed bind, albeit with, in general, reduced affinity.

In summary, the data shown in this section clearly show that the presence of the correct anchor residues is not sufficient *per se* to allow for good HLA binding. It is thus apparent that the nature of the residues contained in positions other than 2(3) and 9 (or 10) can influence binding. The most likely explanation of this observation is that the presence of certain residues (in positions other than 2 and 9) can negate or increase the binding potential of a peptide determinant.

The data shown in the preceding sections describe how specific binding assays can be used to identify, within motif-containing peptides, peptides that are immunogenic. We also wanted to devise an alternative strategy, namely to derive procedures that would be able to predict, within motif-containing peptides, which peptides might be good or intermediate binders and thereby might be immunogenic. In other experiments not shown intermediate or good binders have been shown to be immunogenic. In particular, to identify residues that have a negative impact on binding an analysis of all positions for A3.2, A11, and all motif-containing peptides, both 9-mers and 10-mers is carried out. In the case of A11, because of the small occurrence of nonbinding peptides, a different cutoff was used such that the analysis compares good and intermediate binders on the one hand to weak and nonbinders on the other.

### Example 13

### Algorithms to Identify Immunogenic Peptides

In light of results presented in Example 13 above, algorithms are developed to provide a more exact predictor of binding based upon the effects of different residues at each position of a peptide sequence, in addition to the anchor or conserved residues. More specifically, we utilize the data bank obtained during the screening of our collection of A1, 3,11 or 24 motif containing peptides to develop an algorithm for each particular allele which assigns a score for each amino acid at each position along a peptide. The score for each residue is taken as the ratio of the frequency of that residue in good and intermediate binders to the frequency of occurrence of that residue in non-binders.

In the present algorithm residues have been grouped by similarity. This avoids the problem encountered with some rare residues, such as tryptophan, where there are too few occurrences to obtain a statistically significant ratio. A listing is made of scores obtained by grouping for each of the twenty amino acids by position for 9-mer peptides containing conserved residues that define their motif (2/9 motifs). A peptide is scored in the algorithm as a product of the scores of each of its residues.

The power of an algorithm to correlate with binding is further underlined by its ability to predict a population of peptides with the highest occurrence of good binders. If one were to rely, for example, solely on the 2/9 motif for predicting 9-mer peptides which bind to a specific MHC allele the large number of peptides containing the motif would be predicted to be good binders. In fact only a relatively small percentage of these peptides are good binders and a somewhat larger percentage are intermediate binders, while a still larger percentage of the peptides predicted by the motif are either weak or non-binding peptides. In contrast, using the grouped algorithm of this invention a population of peptides are created with a greater percentage of good binders, a still greater percentage of intermediate binders, and a smaller percentage, relative to that predicted by motif-containing peptides, are weak and non-binders.

The present example of an algorithm uses the ratio of the frequency of occurrence of an amino acid in binders and non-binders to measure the impact of a particular residue at each position of a peptide. It is immediately apparent to one of ordinary skill in the art that there are alternative ways of creating a similar algorithm. For example, one could use average binding affinity values, or relative binding of single amino acid substitutions in a motif containing peptide with a poly-alanine backbone to generate an algorithm table.

An algorithm using average binding affinity has the advantage of including all of the peptides in the analysis, and not just good/intermediate binders and non-binders. Moreover, it gives a more quantitative measure of affinity than the simpler group ratio algorithm. We create such an algorithm by calculating for each amino acid, by position, the average log of binding when that particular residue occurs in our set of motif containing peptides. The algorithm score for a peptide is then taken as the sum of the scores by position for each of its residues.

### Example 14

### Preparation of effective HLA allele-specific antigen presenting cells.

This example demonstrates the use of cold temperature incubation or acid stripping/peptide loading method to prepare effective HLA-allele-specific antigen presenting cells (APC). The APC were used to sensitize precursor cytotoxic T lymphocytes which led to the development of antigen-specific cytotoxic cells. This was accomplished using either phytohemaglutinin (PHA) T-cell blasts or peripheral blood mononuclear cells (PBMC) or staphylococcus aureus Cowan I (SAC-I) activated PBMC as APC. The results are applicable to other APC and to the other MHC alleles.

The following describes sources for materials used in the following examples:
L-Ascorbic acid, Cat #B582, J.T. Baker, Phillipsburg, NJ.
Anti-HLA A2 (BB7.2), Cat #HB82, ATCC, Rockville, MD.
Anti-HLA DR (LB3.1), from J. Gorga, Children's Hospital, Pittsburgh, PA.
Anti-HLA Alpha chain pan ABC (9.12.1), from R.
DeMars, University of Wisconsin, Madison, Wl.
Anti-mouse IgG FITC conjugate, Cat #F2883, Sigma, St.
Louis, MO.
β₂microglobulin, Cat #MO114, Scripps Labs, San Diego, CA.
BSA Fraction V, Cat #A9418, Sigma, St. Louis, MO.
50cc conical centrifuge tubes, Cat #2070, Falcon, Lincoln, Park, NJ.
Cryo 1°C freezing container, Cat #5100-0001, Nalge, Rochester, NY.
Cryovial, Cat #5000-0012, Nalge, Rochester, NY.
Dimethyl sulfoxide (DMSO), Cat #D2650, Sigma, St. Louis, MO.
DNAse, Cat #260912, Calbiochem, San Diego, CA.
Dynabeads M-450 goat anti-mouse IgG, Cat #110.06,
Dynal, Great Neck, NY.
EDTA tetrasodium salt, Cat #ED4SS, Sigma, St. Louis, MO.
FACScan, Becton Dickinson, San Jose, CA.
Fetal calf serum (FCS), Cat #3000, Irvine Scientific, Irvine, CA.
Ficoll-Paque, Cat #17-0840-03, Pharmacia, Piscataway, NJ.
Gentamicin, Cat #600-5750AD, Gibco, Grand Island, NY.
L-Glutamine, Cat #9317, Irvine Scientific, Irvine, CA.
GS-6KR centrifuge, Beckman Instruments, Palo Alto, CA.
Human AB serum (HS), Cat #100-112, Gemini
Bioproducts, Calabasas, CA.
Human rIL-2, Sandoz, Basel, Switzerland.
Human rIL-7, Cat #F1-1587-1, Genzyme, Cambridge, MA.
Isopropanol, Cat #A464-4, Fisher Scientific,
Pittsburgh, PA.
MicroCELLector T-150 culture flask for selection of
CD4+ cells, Cat #8030, Applied Immune Sciences, Menlo Park, CA.
Micromedic automatic gamma counter, ICN Micromedics Systems, Huntsville, AL.
OKT4 hybridoma supernatant, Cat #CRL 8002, ATCC, Rockville, MD.
Paraformaldehyde, Cat #T-353, Fisher, Pittsburgh, PA.
PBS calcium and magnesium free (CMF), Cat #17-516B,
BioWhittaker, Walkersville, MD.
Peptides used in this study were synthesized at Cytel and described in Table 24 a.
Phytohemagglutinin (PHA), Cat #HA-16, Wellcome,
Dartford, England.
RPMI 1640 + Hepes + glutamine, Cat #12-115B,
BioWhittaker, Walkersville, MD.
RPMI 1640 + Hepes + glutamine, Cat #380-2400AJ,
Gibco, Grand Island, NY.
Sodium chloride (NaCl), Cat #3624-05, J.T. Baker,
Phillipsburg, NJ.
Sodium (⁵¹Cr) chromate, Cat #NEZ 030, NEN,
Wilmington, DE.
Sodium phosphate monobasic, Cat #S9638, Sigma, St. Louis, MO.
Triton X-100, Cat #X-100, Sigma, St. Louis, MO. 24 well tissue culture plate, Cat #3047, Falcon, Becton Dickinson, San Jose, CA.
96 well U-bottomed cluster plate, Cat #3799, Costar, Cambridge, MA.
*Culture Medium.* PHA blasts and CTL inductions were done in RPMI 1640 + Hepes + glutamine (Gibco) supplemented with 2 mM L-glutamine (Irvine Scientific), 50 µg/ml gentamicin (Gibco), and 5% heat inactivated pooled human Type AB serum (Gemini Bioproducts) [RPMI/5% HS]. EBV transformed lymphoblastoid cell lines (LCL) were maintained in RPMI 1640 + Hepes + glutamine (BioWhittaker) supplemented with L-glutamine and gentamicin as above and 10% heat inactivated fetal calf serum (Irvine Scientific) [RPMI/10% FCS]. Chromium release assays were performed in RPMI/10% FCS.
*Cytokines.* Recombinant human interleukin-2 (rIL-2) (Sandoz) was used at a final concentration of 10 U/ml. Recombinant human interleukin-7 (rIL-7) (Genzyme) was used at a final concentration of 10 ng/ml.
*Isolation of Peripheral Blood Mononuclear Cells (PBMC).* Whole blood was collected in heparin (10 U/ml) containing syringes and spun in 50cc conical centrifuge tubes (Falcon) at 1600 rpm (Beckman GS-6KR) 15 min. The plasma layer was then removed and 10 ml of the buffy coat collected with a 10 ml pipette using a circular motion. The buffy coat was mixed thoroughly and diluted with an equal volume of serum free RPMI 1640. The diluted buffy coat was then layered over 20 ml Ficoll-Paque (Pharmacia) in a 50cc conical tube and centrifuged 400 x g for 20 min at room temperature with the brake off. The Ficoll-plasma interface containing the PBMCs was collected using a transfer pipet (two interfaces per 50cc tube) and washed three times with 50 ml RPMI (1700, 1500, and 1300 rpm for 10 min.
*Freezing* and *Thawing PBMC.* PBMC were frozen at 30 x 10⁶ cells/ml of 90% FCS + 10% DMSO (Sigma), in 1 ml aliquots using cyrovials (Nalge). Cryovials were placed in Cryo 1°C freezing containers (Nalge) containing isopropanol (Fisher) and placed at -70°C from 4 hr (minimum) to overnight (maximum). Isopropanol was changed after every 5 uses. Cryovials were transferred to liquid nitrogen for long term storage. PBMC were thawed by continuous shaking in a 37°C water bath until the last crystal was nearly thawed. Cells were immediately diluted into serum free RPMI medium containing DNAse 30 µg/ml (to avoid clumping) (Calbiochem), and washed twice.
Depletion *of Lymphocyte Subpopulations.* CD4 lymphocyte depletion was performed using antibody-coated flasks: MicroCELLector T-150 flasks for the selection of CD4+ cells (Applied Immune Sciences) were washed according to the manufacturer's instructions with 25 ml PBS CMF + 1 mM EDTA (Sigma) by swirling flasks for 30 sec followed by incubation for 1 hr at room temperature on a flat surface. Buffer was aspirated and flasks were washed 2 additional times by shaking the flasks for 30 sec and maintaining coverage of the binding surface. To each washed flask, 25 ml culture medium + 5% HS were added and incubated for 20 min at room temperature on a flat surface. Media was left in the flask until it was ready to receive the cells. PBMC were thawed in RPMI/5% HS containing 30 µg/ml DNAse, and washed twice. HS in the wash blocks Fc receptors on PBMCS. For one flask a maximum of 12 x 10⁷ cells were resuspended in 25 ml culture medium. Culture medium was aspirated from the flask and then the cell suspension was gently added to the MicroCELLector. Flasks containing the cells were incubated for 1 hr at room temperature on a flat surface. At the end of the incubation, the flask was gently rocked from side to side for 10 sec to resuspend the nonadherent cells. Nonadherent CD4 depleted cells were harvested, and then flasks were washed twice with PBS CMF to collect the nonadherent cells. Harvested CD4-depleted cells were pelleted by centrifugation and resuspended in complete culture medium (RPMI/5%/HS).
*Generation of PHA Blasts.* PBMC were isolated using the standard Ficoll-Paque protocol. Frozen cells were washed twice before use. Cells were cultured at 2 x 10⁶/ml in RPMI/5t HS containing 1µg/ml PHA (Wellcome) and 10 U/ml rIL-2. PHA blasts were maintained in culture medium containing 10 U/ml r IL-2 with feeding and splitting as needed. PHA blasts were used as APC on day 6 of culture. Generation of empty class I molecules and peptide loading were only performed by the acid strip method when using these APC. *Acid Stripping*/*Peptide* Loading of PBMC and *PHA Blasts.* PBMC were isolated using the Ficoll-Paque protocol. When using frozen cells, PBMC were washed twice before using. PHA blasts were prepared as previously described and washed twice before using. Once cells were prepared, they were washed once in cold sterile 0.9% NaCl (J.T. Baker) + 1% BSA. In a 50cc conical centrifuge tube, the cells were resuspended at 10⁷/ml in cold sterile citrate-phosphate buffer [0-13 M L-ascorbic acid (J.T. Baker), 0.06 M-sodium phosphate monobasic (Sigma) pH 3, 1% BSA, 3 *µ*g/ml β₂microglobulin (Scripps Labs)] and incubated for 2 min on ice. Immediately, 5 volumes of cold sterile neutralizing buffer #1 [0.15 M sodium phosphate monobasic pH 7.5, 1% BSA, 3 µg/ml β₂microglobulin, 10 *µ*g/ml peptide] were added, and the cells were pelleted at 1500 rpm, 5 min at 4°C. Cells were resuspended in 1 volume cold sterile neutralizing buffer #2 [PBS CMF, 1% BSA, 30 lig/ml DNAse, 3 µg/ml β₂microglobulin, 40 µg/ml peptide] and incubated for 4 hrs at 20°C. Cells were diluted with culture medium to approximately 5 x 10⁶/ml and irradiated with 6000 rads. Cells were then centrifuged at 1500 rpm for 5 min at room temperature and resuspended in culture medium. The acid stripped/peptide loaded cells were used immediately in the CTL induction cultures (below).
*Induction of Primary CTL* using *Acid Stripped*/*Peptide* Loaded Autologous *PBMCs* or *PHA Blasts* as Stimulators. Acid stripping/peptide loading of PBMC and PHA blasts are described above. During the last 4 hr incubation of stimulator cells with peptide, the responder cell population was prepared: Responders were PBMC that were depleted of CD4+ cells (described above). Responder cells were resuspended in culture medium at 3 x 10⁶/ml. 1 ml of the responder cell suspension was dispensed into each well of a 24-well tissue culture plate (Falcon, Becton Dickinson). The plates were placed in the incubator at 37°C, 5% CO₂ until the stimulator population was ready. Once irradiated, stimulator APC were resuspended in culture medium containing 20 ng/ml rIL-7 at 10⁶/ml for the PBMC, or at 3 x 10⁵/ml for the PHA blasts. 1 ml of stimulator cell suspension was added per well to the plates containing the responders. On day 7 after induction, a 100 µl culture medium containing 200 ng/ml riL-7 was added to each well (20 ng/well rIL-7 final). On day 10 after induction, 100 µl of culture medium containing 200 U/ml rIL-2 was added to each well (20 U/well rIL-2 final).
*Antigen Restimulation of CTL.* On day 12-14 after the induction, the primary CTL were restimulated with peptide using adherent APC. Autologous PBMC were thawed and washed as described above. Cells were irradiated at 6000 rads. Cells were pelleted and resuspended in culture medium at 4 x 10⁶/ml. 1 ml of cell suspension was added to each well of a 24-well tissue culture plate, and incubated for 2 hrs at 37°C, 5% CO₂. Non-adherent cells were removed by washing each well three times with serum free RPMI. After this step, a 0.5 ml culture medium containing 3 µg/ml β₂microglobulin and 20 µg/ml total peptide was added to each well. APC were incubated for 2 hrs at 37°C, under 5% CO₂ with the peptide and β₂microglobulin. Wells were aspirated and 1 ml of responder cells at 1.5 x 10⁶/ml in culture medium was added to each well. After 2 days, 1 ml of culture medium containing 20 U/ml rIL-2 was added to each well.
*Cytotoxicity Chromium Release Assay.* Seven days following restimulation of primary induction, the cytotoxic activity of the cultures was assessed.
a. Effector Cell Preparation: the responders, which at this stage are renamed "effectors", were centrifuged and resuspended at 10⁷/ml in RPMI/10% FCS. Three-fold serial dilutions of effectors were performed to yield effector to target ratios of 100:1, 33:1, 11:1, and 3:1. Effector cells were aliquoted at 100 µl/well on 96 well U-bottomed cluster plates (Costar), in duplicate.
b. Target Cell Preparation: Approximately 16-20 hrs prior to the assay, target cells were resuspended at 3 x 10⁵/mi in RPMI/10% FCS in the presence or absence of 3 µg/ml β₂microglobulin and 10 µg/ml total peptide. After preincubation, target cells were centrifuged and pellets were resuspended in 200 µl (300µCi) sodium (⁵¹Cr) chromate (NEN). Cells were incubated at 37°C for 1 hr with agitation. Labelled target cells were washed 3 times with RPMI/10% FCS.
c. Setting-Up the Assays: Target cell concentration was adjusted to 10⁵/ml in RPMI/10% FCS and 100 *µ*l aliquots were added to each well containing responders. K562 cells (cold targets, to block NK, and LAK activity) were washed and resuspended in RPMI/10% FCS at 10⁷/ml. Aliquots of 20 µl were added per well, yielding a 20:1 of cold K562 target:labelled target. For the determination of the spontaneous ⁵¹Cr release, 100 µl/well of RPMI/10% FCS were added to 100 µl/well of labelled target cells, and 20 µl/well of K562. For maximum ⁵¹Cr release, 100 µl 1% Triton X-100 (Sigma) in PBS CMF, was added to the 100 µl/well labelled target cells, and 20 µl/well K562. Plates were centrifuged for 2 min at 1200 rpm to accelerate cell conjugate formation. Assays were incubated for 5 hr at 37°C, 5% CO₂. Assays were harvested by centrifuging plates for 5 min at 1200 rpm and collecting 100 µl/well of supernatant. Standard gamma counting techniques were used to determine percent specific lysis (Micromedic automatic gamma counter, 0.5 min per tube).
*Cultured Cell Lines.* JY, a HLA A2.1 expressing human EBV-transformed B-cell line, was grown in RPMI/10% FCS. K562, a NK cell sensitive erythroblastoma line was grown in RPMI/10% FCS. K562 was used to reduce background killing by NK and LAK cells in the chromium release assays.
Peptides. The peptides used in these studies were synthesized at Cytel and their sequences are described in Table 24 a. Peptides were routinely diluted in 100% DMSO at 20 mg/ml, aliquoted, and stored at -20°C.
FACS Analysis. Approximately 10⁶ cells were used for each antibody that was to be tested. Cells were washed twice with PBS CNU + 0.1% BSA. To each sample, 100 *µ*l PBS CMF + 0.1% BSA + primary antibody at 2 µg/ml (BB7.2, ATCC) or (9.12.1, Inserm-CNRS, Marseille, France) or (LB3.1, Children's Hospital Pittsburgh) were added. A negative control was always included. Cells were incubated on ice for 2U min and washed twice with PBS CMF + 0.1% BSA. Cells were resuspended in 100 . µl anti-mouse IgG FITC conjugate (Sigma), diluted 1:50 in PBS CMF + 0.1% BSA, and incubated 20 min on ice. Cells were washed twice with PBS CMF + 0.1% BSA, and resuspended in PBS for FACScan (Becton Dickinson) analysis. When it was necessary to postpone analysis to the subsequent days, the cells were fixed with PBS/1% paraformaldehyde (Fisher) and analyzed within one week.
*Binding Assays Using Intact Cells and Radiolabelled Peptide.* JY cells were treated with citrate-phosphate buffer and neutralizing buffer #1 as described above. JY control cells were left untreated in tissue culture media. After treatment both cell populations were washed twice with serum free RPMI and loaded with ¹²⁵I-radiolabelled 941.01 (HBc15-27) peptide (standard chloramine T iodination). To determine binding specificity, 2 x 10⁶ cells were resuspended in 200 µl neutralizing buffer #2 (described above) containing ¹²⁵1-941.01 (10⁵ cpms) +/- 100 µg unlabelled 941.01. Cells were incubated for 4 hrs at 20°C and washed twice with serum free RPMI to remove free peptide. Cells were resuspended in 200 *µ*l of serum free RPMI. In a microfuge tube the cell suspension was layered over an 800 *µ*l FCS and pelleted by centrifugation for 5 sec. Supernatants were aspirated and the radioactivity remaining in the pellet was measured (Micromedic automatic gamma counter, 1 min per tube).

### Example 15

### Class I MHC molecule peptide stripping/loading by mild acid treatment.

Mild acid solutions of pH 3 such as glycine or citrate-phosphate buffers have been used by various groups to identify endogenous peptides and to identify tumor associated T cell epitopes. The treatment is unique in that only the MHC class I molecules are destabilized (and peptides released), while all other surface antigens remain intact including MHC class II molecules. Most importantly, treatment of cells with the mild acid solutions of this example do not affect the cell's viability or metabolic state. The mild acid treatment is rapid since the stripping of endogenous peptides occurs in two minutes at 4°C and the APC is ready to perform its function after the appropriate peptides are loaded. In this example we utilized the technique to make peptide specific APCs for the generation of primary antigen-specific CTL. The resulting APC were efficient in inducing peptide-specific CD8+ CTL.
*Measurements by FACS Analysis.* PHA-induced T-cell blasts were acid stripped/peptide loaded according to the methods described in Example 15. The resulting cells were stained for FACS analysis using anti-HLA-A2 (BB7.2) and anti-HLA alpha chain-specific (9.12.1) monoclonal antibodies. Controls for this experiment included the same cell population which was not treated at pH 3 (but treated with PBS buffer at pH 7.2), and with cells treated with citrate-phosphate buffer (to strip the MHC) but neutralized in the absence of β₂microglobulin and peptide. The results presented in Figure 15, indicate that treatment of these cells with the citrate-phosphate (pH3) buffer significantly reduced (10-fold) the reactivity of the cells toward both anti-HLA class I antibodies alone (anti-HLA-A2 and the alpha chain specific), but not towards a monoclonal antibody specific for class II MHC molecules (anti-HLA-DR). Most importantly, neutralization of the acid-stripped cells in the presence of β₂microglobulin and peptide resulted in preservation of a significant amount of class I MHC antibody-reactive sites, with only a 2.5-fold decrease in fluorescence intensity. Importantly, the acid-treated cells remained viable, as measured by trypan blue exclusion and forward/lateral FACS scatter analysis. Similar results were obtained using EBV-transformed B cell lines, fresh (or frozen) PBMC and other peptides (which bind to either HLA-A2.1 or HLA-A1) (data not shown).
*Binding of Radiolabeled Peptides to Empty MHC Molecules.* To determine the efficiency of peptide loading using the cold temperature incubation or acid stripping/peptide loading protocol, JY cells (an.HLA-A2.1 EBV-transformed B cell line) were preincubated at 26°C overnight or acid-stripped to remove the endogenous MHC-associated peptides and the loading of exogenous peptide was determined using a ¹²⁵I-radiolabelled HLA-A2.1 binding peptide. The specificity of this reaction was determined by measuring the inhibition of labelled peptide binding using a cold peptide of the same sequence. Results presented in Table 24 b demonstrate that acid-treatment of the cells increased significantly (approximately 10-fold) the amount of labelled peptide binding to the JY cells. Furthermore, the binding of labelled peptide was completely blocked by the addition of the cold peptide, demonstrating specific binding (data not shown).
*In Vitro Induction of Primary Antigen -Specific CTL Using Acid Stripped*/*Peptide Loaded APCS.* Additional critical parameters for the induction of primary CTL using both the cold temperature incubation and acid strip protocol are: 1) enrichment of CD8+ T-cells in the responder cell population (or depletion of CD4+ T-cells), 2) addition of rIL-7 to the CTL induction cultures from day 0, and 3) restimulation of the cultures with antigen on day 12-14 using autologous adherent cells pulsed with peptide.

Results presented in Figs. 16 and 17 show experiments performed using PBMC and PHA-induced T-cell blasts as APC. Figure 18 shows experiments using PHA-induced T-cell blasts as APC while Figure 19 shows the use of PBMC as APC.

### Example 16

### Screening peptides to identify CTL epitopes

In order to identify CTL epitopes, CTL was stimulated by SAC-I activated PBMCs as APC. Cold temperature expression of the MHC in which class 1 β-2-microglobulin complex is unstable was utilized in addition to acid stripping to generate PBMC APC.
*Complete Culture Medium*. The tissue culture medium used in this study consisted of RPMI 1640 with Hepes and L-glutamine (Gibco) supplemented with 2 mM L-glutamine (Irvine Scientific), 0.5mM sodium pyruvate (Gibco), 100 U/100 ug/ml penicillin/streptomycin (Irvine), and 5% heat-inactivated Human Serum Type AB (RPMI/5% HS; Gemini Bioproducts). Culture media used in the growth of EBV-transformed lines contained 10% heat-inactivated fetal calf serum (RPMI/10% FCS, Irvine) instead of human serum.
*Cytokines.* Recombinant human Interleukin-2 (rIL-2) and Interleukin-4 (rIL-4) were obtained from Sandoz and used at a final concentration of 10 U/ml and 10 ng/ml, respectively. Human interferon-γ (IFN-γ) and recombinant human Interleukin-7 (rlL-7) were obtained from Genzyme and used at 20 U/ml and 10 ng/ml, respectively.
Peptides. Peptides were synthesized at Cytel and are described in Table 24 a. Peptides were routinely diluted in 100% DMSO at 20 mg/ml, aliquoted, and stored at -70°C until use.
*Cell Lines.* JY, Steinlin, EHM, BVR, and KT3 are homozygous human EBV-transformed B cell lines expressing HLA A_{2.1}, A₁, A₃, A₁₁, and A₂₄, respectively. They are grown in RPMI/10% FCS. K562, an NK cell sensitive, erythoblastoma line grown in RPMI/10% FCS, was used for reduction of background killing in CTL assays. Melanoma cell lines either expressing the MAGE antigen, mel 397 and mel 938, or not expressing the MAGE antigen; mel 888, were also grown in RPMI/10% FCS.
*Isolation of Peripheral Blood Mononuclear Cells (PBMCs).* Whole blood was collected into heparin containing syringes and spun in 50cc tubes at 1600 RPM (Beckman GS-6KR) for 15 minutes. The plasma layer was then removed and 10 ml of buffy coat was collected with a pipette using a circular motion. The buffy coat was mixed well and diluted with an equal volume of RPMI. The buffy coat (30 ml) was then layered on 20 ml of
Ficoll-Paque (Pharmacia) and centrifuged at 1850 RPM (400xg) for 20 minutes, 25°C, with the brake off. The interface between the ficoll and the plasma containing the PBMCs was recovered with a transfer pipet (two interfaces per 50 ml tube) and washed three times with 50 ml of RPMI (1700, 1500, and 1300 RPM for 10 minutes). Cells were resuspended in 10-20 ml of culture medium, counted, and adjusted to the appropriate concentration.
Freezing PBMCs. 30 million cells/tube (90% FCS/10% DMSO; Sigma) were inserted into a Nalgene Cryo 1°C Freezing Container containing isopropanol (Fisher) and placed at -70°C from 4 hrs (minimum) to overnight (maximum). The isopropanol was changed every five times. Tubes were transferred to liquid nitrogen for long term storage. To thaw, PBMCs were continuously shaken in a 37°C water bath until the last crystal was almost thawed (tubes were not allowed to sit in the water bath or at room temperature for any period of time). Cells were diluted into serum-free RPMI containing 30 µg/ml DNase to prevent clumping by dead cell DNA and washed twice.
*Induction of Primary CTL Using SAC-I Activated PBMCs as ApCs*
a. Preparation of APCs: PBMCs were purified using the standard Ficoll-Paque protocol and resuspended at 1 x 10⁶/ml in RPMI/5% FCS containing 0.005% Pansorbin cells (SAC-I cells expressing Protein A; Calbiochem), 20 *µ*g/ml Immunobeads (Rabbit anti-Human IgM; Biorad), and 20 ng/ml of human rIL-4. Two ml of cells per well were plated in a 24-well plate (Falcon, Becton Dickinson) and cultured at 37°C. After 3 days, the medium was removed and the cells were washed three times followed by addition of RPMI/10% HS. The cells were used after culturing for an additional 2 days in RPMI/10% HS.
b. Expression of empty Class I molecules on the surface of APCs and peptide loading of APCs.
   1. Cold temperature incubation:
      a. Expression of empty MHC in APCs: The APCs were adjusted to a concentration of 2 x 10⁶/ml in complete culture medium containing 10 ng/ml rIL-4, 20 U/ml human IFN-γ and 3 µg/ml β2-microglobulin (β₂m; Scripps Lab). The cells were then incubated overnight at 26°C in the presence of 5% CO₂. It should be noted that these cells only express a fraction of Class I molecules in the empty state (^{~}10%).
      b. Peptide loading of APC stimulator cells: Empty Class I expressing APCs were washed 1-2 times with serum free RPMI (+ L-glutamine and Hepes) and resuspended at 1 ×10⁷ in serum-free RPMI containing 50 µg/ml total of the peptide pool (i.e., 16.7 µg/ml of each peptide in a pool of three; 25 µg/ml of each peptide in a pool of two; 50 µg/ml of individual peptide), 30 µg/ml DNAse, and 3 µg/ml β₂m. Following a 4 hour incubation at 20°C, the cells were irradiated at 6100 rads (5 x 10⁶/ ml; 25 million cells/tube), washed and adjusted to the appropriate concentration for addition to the induction culture (see below).
   2. Acid stripping: This was used as an alternative method for generating empty MHC on the surface of the APCs. The SAC-I activated PBMCs were washed once in cold 0.9% sodium chloride (J.T. Baker) containing 1% BSA. The cells were resuspended at 10⁷/ml in cold citrate-phosphate buffer (0.13M L-ascorbic acid [J.T. Baker], 0.06M sodium phosphate monobasic [Sigma], pH3) containing 1% BSA and 3 *µ*g/ml β₂m and incubated on ice. After 2 minutes, 5 volumes of cold 0.15M sodium phosphate monobasic buffer, pH7.5, containing 1% BSA, 3 *µ*g/ml β₂m, and 10 µg/ml peptide [neutralizing buffer #1] was added and the cells centrifuged at 1500 RPM for 5 minutes at 4°C. The cells were resuspended in 1 ml of cold PBS containing 1% BSA, 30 µg/ml DNase, 3 µg/ml β₂microglobulin, and 50 µg/ml peptide [neutralizing buffer #2] and incubated for 4 hours at 20°C. As above, subsequent to the four hour incubation at 20°C, the cells were irradiated at 6100 rads (5 x 10⁶/ ml; 25 million cells/tube), washed, then adjusted to the appropriate concentration for addition to the induction culture (see below).
c. Preparation of the CD4+ depleted PBMC responder cell population (depletion of lymphocyte sub-populations using AIS flasks). AIS MicroCellector T-150 flasks (specific for the depletion of CD4+ T cells; Menlo Park, CA) were primed by adding 25 ml of PBS/1 mM EDTA, swirling for 30 seconds so that all surfaces were moistened, and then incubating with the binding surface down at room temperature for 1 hour. Following this incubation, flasks were shaken vigorously for 30 seconds, washed 1 time with PBS/EDTA, 2 additional times with PBS and then incubated with 25 ml of culture medium for 15 minutes. PBMCs were thawed in serum-free RPMI (+ L-glutamine + Hepes) containing 30 µg/ml DNAse, washed once, and incubated for 15 minutes in culture medium. Following aspiration of culture medium from the flasks, up to 180 million PBMCs were added in 25 ml of culture medium containing 30 µg/ml DNAse. After 1 hour at room temperature, the flasks were rocked gently for 10 seconds to resuspend the nonadherent cells. The nonadherent cell suspension containing the CD8+ T cells was collected and the flasks were' washed 2 times with PBS. The CD4+ T cell depleted PBMCs were centrifuged and counted for addition to the induction culture. The CD4+ and CD8+ phenotype of the CD4+ depleted cell population was determined by FACS analysis (see below). In general, this technique resulted in a two-fold enrichment for CD8+ T cells with an average of approximately 40-50% CD8+ T cells and 15-20% remaining CD4+ T cells following depletion of CD4+ T cells. Depletion of CD4+ T cells can also be accomplished by antibody and complement or antibody coated magnetic beads (Dynabeads). Depletion of CD4+ T cells served the purpose of enriching CTLp and removing cells which would complete for cell nutrients and may interfere with CTLp expansion.
d. Induction of primary CTL. During the 4 hour peptide loading of the stimulator APCs, CD4+ depleted PBMC to be used as the responder population were prepared utilizing AIS flasks for selection of CD8+ T cells through the depletion of CD4+ T cells (above). The responder cells were plated at 3 x 10⁶/ml in a 1 ml volume (24 well plate) and placed at 37°C until the peptide loaded stimulator APCs were prepared. The irradiated, peptide loaded APCs were washed 1 time in serum-free RPMI (+ L-glutamine and Hepes), adjusted to 1x10⁶/ml in complete medium, and plated into a 24 well plate at 1 ml/plate: For PBMC, 1 x 10⁶ stimulator cells (1 ml volume) were plated into the wells containing the responder cells; For SAC-I activated PBMC and PHA blasts, 1 ml of 3 x 10⁵/ml stimulator cells were plated in each well. A final concentration of 10 µg/ml of additional peptide was added in addition to 10 ng/ml final concentration of rIL-7 (2 ml total volume). On day 7 an additional 10 *µ*g/ml rIL-7 was added to the culture and 10 U/ml rIL-2 was added every 3 days thereafter. On day 12, the cultures were restimulated with peptide pulsed adherent cells and tested for cytolytic activity 7 days later (below).
*Protocol for Restimulation of Primary CTL Using Adherent APC.* PBMCs were thawed into serum-free RPMI (+ L-glutamine and Repes) containing 30µg/ml DNAse, washed 2 times, and adjusted to 5 x 10⁶ /ml in culture medium containing DNAse. PBMCs (25 million cells/tube in 5 ml) were irradiated at 6100R. After 1 wash, the PBMCs were resuspended in culture medium and adjusted to 4 x 10⁶/ml. 1 ml of irradiated PBMCs was added per well of a 24-well plate. The PBMC were incubated for 2 hours at 37°c, washed 3 times to remove non-adherent cells, and cultured in medium containing 20 µg/ml total peptide and 3 *µ*g/ml β₂microglobulin added in a 0.5 ml volume and again incubated for 2 hours at 37°C. The peptide was aspirated and 1.5 x 10⁶ responder cells resuspended in culture medium were added in a 1 ml volume. After 2 days, 1 ml of culture medium containing 20 U/ml rIL-2 was added.
*FACS Analysis.* One million cells/tube were centrifuged, resuspended in 100 ul/tube PBS/0.1% BSA/0.02% sodium azide (Sigma) plus 10 ul/tube directly conjugated antibody (Becton Dickinson), and incubated on ice 15-20 minutes. Cells were then washed 2 times with PBS/0.1% BSA/0.02% sodium azide and resuspended in PBS to analyze on FACScan (Beckton Dickinson). When it was not possible to analyze samples within 1-2 days, cells were fixed with PBS containing 1% paraformaldehyde (Fisher) and analyzed within one week.
*Cytotoxicity Assay*
a. Target cell preparation. Approximately 16-20 hours prior to the CTL assay, target cells (Class I matched EBV-transformed lines) were washed once and resuspended in a 10 ml volume at 3 x 10⁵/ml in RPMI/5% FCS in the presence or absence of 10 µg/ml total peptide.
b. Labeling of target cells: Target cells were centrifuged and resuspended in 200 µl/tube sodium ⁵¹Cr chromate (NEN), then incubated at 37°C for 1 hour on a shaker. Targets were washed 3 times (10 ml/wash) with RPMI/10% FCS and resuspended in 10 ml (to determine the efficiency of labelling, 50 µl/target was counted on the Micromedic automatic gamma counter).
c. CTL assay. Target cells were adjusted to 2 x 10⁵/ml and 50 *µ*l of the cell culture was added to each well of a U-bottomed 96-well plate (Costar Corp.) for a final concentration of 1 X 10⁴/well. K562 cells were washed once, resuspended at 4 x 10⁶/ml, and 50 *µ*l/well was added for a final concentration of 2 x 10⁵/well (ratio of cold K562 to target was 20:1). Responder cells were washed once, resuspended at 9 x 10⁶/ml, and three fold serial dilutions were performed for effector to target ratios of 90:1, 30:1, 10:1, and 3:1. Responder cells were added in a volume of 100 *µ*l in duplicate wells. For spontaneous release, 50 µl/well of labelled target cells, 50 µl/well K562, and 100 µl/well of medium was added. For maximum release, 50 µl/well target, 50 µl/well K562, and 100 µl/well of 0.1% Triton-X100 (Sigma) was added. Plates were centrifuged for 5 minutes at 1200 RPM. Following a 5 hour incubation at 37°C, plates were centrifuged again for 5 minutes at 1200 RPM, and 100 µl/well of supernatant was collected. Standard gamma counting techniques (Micromedic automatic gamma counter; 0.5 minutes/tube) were used to determine the percent specific lysis according to the formula: % specific lysis - cpm experimental - cpm spontaneous release/cpm maximum release - cpm spontaneous release X 100. A cytotoxicity assay (CTL assay) was considered positive if the lysis by CTL of targets sentized with a specific peptide at the two highest effector to target (E:T) ratios was 15% greater than lysis of control targets (i.e., target cells without peptide). A cytotoxicity assay (CTL assay) was considered borderline if the lysis by CTL of targets sensitized with a specific peptide at the two highest effector to target (E:T ratios was 6% greater than lysis of control targets (i.e., target cells without peptide).
d. Results. Of the peptides that bind to the indicated alleles, 9 of the 49 MAGE peptides, 10 of the 45 HIV peptides, 3 of the 25 HCV peptides, and 2 of the 20 HBV peptides tested to date induced primary CTL *in vitro*. Representative graphs illustrating CTL responses to various immunogenic peptides are shown for MAGE (Figure 22), HIV (Figure 23), HCV (Figure 24), and HBV (Figure 2). The CTL induction data are summarized in Table 24 which lists the immunogenic peptides which bind to the appropriate MHC and induce primary CTL in vitro. Indicated is the peptide's sequence, corresponding antigen and HLA allele to which it binds. Results shown in Figure 20 illustrate lysis of peptide sensitized targets and endogenous targets following stiumlation with SAC-I activated PBMCs loaded with a MAGE 3 peptide, 1044.07 by the cold temperature and incubation technique. Figure 21 shows a comparison of the acid strip loading technique (Panel a) with the cold temperature incubation technique (panel b).

Although the present invention has been described in some detail by way of illustration and example for purposes of clarity and understanding, it will be apparent that certain changes and modifications may be practiced within the scope of the appended claims.

**TABLE 13 VALIDATION OF CYTEL'S HLA MOTIFS**

| Binding Capacity (IC50 nM) | | | | | | |
|---|---|---|---|---|---|---|
| SEQUENCE | MOTIF | A1 | A2.1 | A3.2 | A11 | A24 |
| AADKAAAAY | A1 | 50 | - -* | - - | - - | - - |
| ATAKAAAAY | A1 | 15 | - - | 329 | 77 | - - |
| ATDKAAAAY | A1 | 2.8 | - - | 9250 | 840 | ND |
| ALAKAAAAV | A2.1 | - - | 125 | - - | - - | - - |
| AMAAAAAAK | A3.2 | - - | - - | 48 | 8.4 | - - |
| ATAAAAAAK | A11 | - - | - - | 59 | 40 | - - |
| AYAKAAAAF | A24 | - - | - - | - - | - - | 115 |

| | | | | | | |
|---|---|---|---|---|---|---|
| *A dash indicates an IC50 greater than 20,000 nM. | | | | | | |

**TABLE 14 VALIDATION OF CYTEL'S HLA MOTIFS**

| Binding Capacity (IC50 nM) | | | | | |
|---|---|---|---|---|---|
| SEQUENCE | MOTIF | A1 | A2.1 | A3.2 | A11 |
| AADKAAAAAY | A1 | 45 | - -* | - - | - - |
| ATAKAAAAAY | A1 | 58 | -- | 1100 | 1030 |
| ATDKAAAAAY | A1 | 4.0 | -- | 10000 | 4533 |
| ALAKAAAAAV | A2.1 | ND | 1400 | - - | - - |
| AMAAAAAAAK | A3.2 | ND | - - | 85 | 24.0 |
| ATAAAAAAAK | A11 | - - | - - | 216 | 88 |

| | | | | | |
|---|---|---|---|---|---|
| *A dash indicates an IC50 greater than 20,000 nM. | | | | | |

**TABLE 15 HLA-A3.2 2**

| PEPTIDE | SEQUENCE | AVERAGE RATIO TO 952.25 | SUBSTITUTION |
|---|---|---|---|
| 952.25 | ALAAAAAAK | 1 | |
| 952.26 | AMAAAAAAK | 1.2 | position 2 |
| 952.23 | AVAAAAAAK | 0.95 | |
| 981.04 | ASAAAAAAK | 0.89 | |
| 952.24 | AIAAAAAAK | 0.57 | |
| 952.27 | AAAAAAAAK | 0.57 | |
| 981.06 | ATAAAAAAK | 0.49 | |
| 981.08 | AFAAAAAAK | 0.13 | |
| 981.09 | AGAAAAAAK | 0.077 | |
| 981.13 | ACAAAAAAK | 0.031 | |
| 981.12 | ADAAAAAAK | 0.014 | |
| 981.11 | ANAAAAAAK | 0.0010 | |
| 981.05 | AKAAAAAAK | <0.0016 | |
| 981.07 | AYAAAAAAK | <0.0005 | |
| 981.10 | APAAAAAAK | <0.0006 | |
| 952.35 | ALAAAAAAR | 0.46 | position 9 |
| 981.36 | ALAAAAAAY | 0.15 | |
| 981.33 | ALAAAAAAA | 0.0034 | |
| 981.35 | ALAAAAAAQ | <0.0006 | |
| 981.37 | ALAAAAAAS | <0.0005 | |
| 981.38 | ALAAAAAAT | <0.0005 | |
| 981.34 | ALAAAAAAN | <0.0005 | |
| 981.39 | ALAAAAAAE | <0.0003 | |

**TABLE 16 HLA-A11**

| PEPTIDE | SEQUENCE | AVERAGE RATIO TO 952.25 | SUBSTITUTION |
|---|---|---|---|
| 952.25 | ALAAAAAAK | 1 | - |
| 952.26 | AMAAAAAAK | 2.5 | position 2 |
| 952.27 | AAAAAAAAK | 1.1 | |
| 952.24 | AIAAAAAAK | 0.72 | |
| 981.06 | ATAAAAAAK | 0.55 | |
| 981.04 | ASAAAAAAK | 0.46 | |
| 981.09 | AGAAAAAAK | 0.38 | |
| 952.23 | AVAAAAAAK | 0.23 | |
| 981.11 | ANAAAAAAK | 0.23 | |
| 981.13 | ACAAAAAAK | 0.019 | |
| 981.08 | AFAAAAAAK | 0.020 | |
| 981.12 | ADAAAAAAK | 0.012 | |
| 981.05 | AKAAAAAAK | 0.0065 | |
| 981.07 | AYAAAAAAK | <0.0065 | |
| 981.10 | APAAAAAAK | <0.0051 | |
| 952.35 | ALAAAAAAR | 0.015 | position 9 |
| 981.33 | ALAAAAAAA | <0.0059 | |
| 981.34 | ALAAAAAAN | <0.0071 | |
| 981.35 | ALAAAAAAQ | <0.0051 | |
| 981.36 | ALAAAAAAY | <0.0071 | |
| 981.37 | ALAAAAAAS | <0.0051 | |
| 981.38 | ALAAAAAAT | <0.0051 | |
| 981.39 | ALAAAAAAE | <0.0071 | |

**TABLE 17 HLA-A24**

| PEPTIDE | SEQUENCE | AVERAGE RATIO TO 983.01 | SUBSTITUTION |
|---|---|---|---|
| 983.01 | AYAKAAAAF | 1 | |
| 983.08 | APAKAAAAF | 0.24 | position 2 |
| 983.09 | APAKAAAAF | 0.0058 | |
| 983.10 | AAAKAAAAF | 0.0023 | |
| 983.11 | AKAKAAAAF | <0.0012 | |
| 983.05 | AYAKAAAAI | 0.20 | position 9 |
| 963.04 | AYAKAAAAL | 0.11 | |
| 983.06 | AYAKAAAAV | 0.0023 | |
| 983.02 | AYAKAAAAA | <0.0012 | |
| 983.03 | AYAKAAAAY | <0.0012 | |
| 983.07 | AYAKAAAAK | <0.0012 | |

**TABLE 18 HLA-A1**

| PEPTIDE | SEQUENCE | AVERAGE RATIO TO 982.07 | SUBSTITUTION |
|---|---|---|---|
| 982.011 | ATDKAAAAY | Motif | |
| 982.07 | ATAKAAAAY | 1 | |
| 982.09 | ASAKAAAAY | 0.17 | position 2 |
| 982.13 | AMAKAAAAY | 0.095 | no D in pos 3 |
| 982.08 | AAAKAAAAY | 0.0064 | |
| 954.09 | ALAKAAAAY | 0.0045 | |
| 954.11 | AIAKAAAAY | 0.0045 | |
| 954.13 | AVAKAAAAY | 0.0020 | |
| 982.10 | AKAKAAAAY | 0.0011 | |
| 982.11 | ANAKAAAAY | <0.0001 | |
| 982.12 | ADAKAAAAY | <0.0001 | |
| 982.14 | AGAKAAAAY | <0.0001 | |
| 982.15 | APAKAAAAY | <0.0001 | |
| 982.16 | AYAKAAAAY | <0.0001 | |
| 982.17 | AHAKAAAAY | <0.0001 | |
| 982.24 | ATAKAAAAA | 0.0040 | position 9 |
| 982.23 | ATAKAAAAF | 0.0019 | no D in pos 3 |
| 982.28 | ATAKAAAAH | 0.0010 | |
| 982.32 | ATAKAAAAV | 0.0005 | |
| 982.25 | ATAKAAAAN | <0.0001 | |
| 982.26 | ATAKAAAAD | <0.0001 | |
| 982.27 | ATAKAAAAW | <0.0001 | |
| 982.30 | ATAKAAAAK | <0.0001 | |
| 982.31 | ATAKAAAAI | <0.0001 | |
| 982.29 | ATAKAAAAP | <0.0001 | |

**TABLE 19 HLA-A1**

| PEPTIDE | SEQUENCE | AVERAGE RATIO TO 982.07 | SUBSTITUTION |
|---|---|---|---|
| 982.01 | ATDKAAAAY | Motif | - |
| 982.07 | ATAKAAAAY | 1 | - |
| 982.01 | AADKAAAAY | 0.14 | position 3 |
| 954.03 | AAEKAAAAY | 0.038 | no T in pos 2 |
| 982.02 | AAAKAAAAY | 0.0055 | |
| 982.06 | AASKAAAAY | 0.0024 | |
| 982.04 | AANKAAAAY | 0.0011 | |
| 982.03 | AAQKAAAAY | 0.0008 | |
| 982.05 | AAKKAAAAY | <0.0001 | |
| 982.20 | AADKAAAAA | 0.0016 | position 9 |
| 982.21 | AADKAAAAW | 0.0005 | no T in pos 2 |
| 982.19 | AADKAAAAF | <0.0001 | |
| 982.22 | AADKAAAAK | <0.0001 | |

**TABLE 20(A) HPV16 E6 AND E7 PEPTIDES BINDING TO HLA-A1**

| Orgin | First aa Position | Sequence^{*} | Binding Ratio to Standard^{‡} | Motif Prediction |
|---|---|---|---|---|
| E6 | 80 | ISEYRHYAY | 3.500 | + |
| E6 | 69 | VADKALKFY | 0.240 | + |
| E7 | 44 | QABPDRAHY | 0.029 | + |
| E7 | 37 | EIDOPARPOA | 0.025 | - |
| E7 | 19 | TTDLYAYEQ | 0.023 | + |
| E6 | 144 | MSAARSSRT | 0.019 | +/- |
| E7 | 73 | HVDIRTLED | 0.014 | - |
| E6 | 139 | WTGRMISAR. | 0.010 | - |
| E6 | 61 | YRDGNPYAV | 0.008 | - |

| | | | | |
|---|---|---|---|---|
| ^{*} Bold A's indicate residues in which cysteine was replaced by alanine. ⁺⁺ The average IC₅₀ value ±SE of the standard in the course of the experiments considered in this table was 81±30 nM. Listed in the table are peptides yielding ratio values of ≥0.001. All other peptides yielded ratio values of ≥0.001. | | | | |

**TABLE 20 (B) HPV16 E6 and E7 Peptides Binding to HLA-A3.2**

| Origin | First aa Position | Sequence^{*} | Binding Ratio to Standard^{‡} | Motif Prediction |
|---|---|---|---|---|
| E6 | 107 | LIRAINAQK | 3.7000 | + |
| E6 | 59 | IVYRDGNPY | 3.0000 | + |
| E7 | 89 | IVAPIASQK | 2.2000 | + |
| E6 | 33 | IILRAVYAK | 1.5000 | + |
| E6 | 125 | HLDKKQRFH | 0.4400 | + |
| E6 | 143 | AMSAARSSR | 0.1800 | + |
| E6 | 7 | AMFQDPQER | 0.1000 | + |
| E6 | 93 | TTLEQQYNK | 0.0780 | + |
| E6 | 37 | AVYAKQQLL | 0.0320 | - |
| E7 | 51 | HYNIVTFAA | 0.0210 | - |
| E6 | 145 | SAARSSRTR | 0.0200 | + |
| E6 | 75 | KFYSKISEY | 0.0100 | + |
| E6 | 89 | SLYGTTLEQ | 0.0080 | - |
| E7 | 52 | YNIVTFAAK | 0.0067 | - |
| E6 | 80 | ISEYRHYAY | 0.0064 | + |
| E6 | 42 | QQLLRREVY | 0.0058 | - |
| E6 | 68 | AVADKALKF | 0.0056 | + |
| E6 | 97 | QQYNKPLAD | 0.0045 | - |
| E6 | 79 | KISEYRHYA | 0.0044 | - |
| E6 | 84 | RHYAYSLYG | 0.0036 | - |
| E6 | 69 | VADKALKFY | 0.0025 | + |
| E6 | 146 | AARSSRTRR | 0.0020 | + |
| E7 | 58 | AAKADSTLR | 0.0016 | + |
| E6 | 38 | VYAKQQLLR | 0.0012 | - |
| E6 | 67 | YAVADKALK | 0.0012 | + |
| E7 | 60 | KADSTLRLA | 0.0012 | - |

| | | | | |
|---|---|---|---|---|
| ^{*} Bold A's indicate residues in which cysteine was replaced by alanine. ^{‡} The average IC₅₀ value ±SE of the standard in the course of the experiments considered in this table was 30±3 nM. Listed in the table are peptides yielding ratio value of ≥0.001. All other peptides yielded ratio values of ≤0.001. | | | | |

**Table 20 (C) HPV16 E6 and E7 Peptides Binding to HLA-A11.2**

| Orgin | First aa Position | Sequence^{*} | Binding Ratio to Standard^{‡} | Motif Prediction |
|---|---|---|---|---|
| E6 | 33 | IILEAVYAK | 6.7000 | + |
| E6 | 93 | TTLEQQYNK | 1.8000 | + |
| E7 | 89 | IVAPIASQK | 1.3000 | + |
| E6 | 7 | AMFQDPQER | 0.8400 | +/- |
| E6 | 59 | IVYRDGNPY | 0.4700 | - (+)' |
| E6 | 80 | ISEYRHYAY | 0.4300 | - (+)^{#} |
| E6 | 37 | AVYAKQQLL | 0.0450 | - |
| E6 | 145 | SAARSSRTR | 0.0330 | +/- |
| E6 | 107 | LIRAINAQK | 0.0120 | + |
| E7 | 58 | AAKADSTLR | 0.0110 | +/- |
| E6 | 42 | QQLLRREVY | 0.0084 | +/- (+)^{#} |
| E6 | 143 | AMSAARSSR | 0.0084 | - |
| E6 | 79 | KISEYRHYA | 0.0076 | - |
| E6 | 67 | YAVADKALK | 0.0074 | + |
| E7 | 52 | YNIVTFAAK | 0.0060 | + |
| E6 | 68 | AVADKALKF | 0.0037 | - |
| E6 | 69 | VADKALKFY | 0.0030 | - (+)^{#} |
| E6 | 38 | VYAKQQLLR | 0.0022 | +/- |
| E6 | 140 | TGRAMSAAR | 0.0012 | +/- |
| E7 | 90 | VAPIASQKP | 0.0012 | - |
| E7 | 51 | HXNIVTFAA | 0.0010 | - |

| | | | | |
|---|---|---|---|---|
| ^{*}Bold A's indicate residues in which cysteine was replaced by alanine. ^{‡}The average IC₅₀ value ±SE of the standard in the course of the experiments considered in this table was 10±3 nM. Listed in the table are peptides yielding ratio value of ≥ 0.001. All other peptides yielded ratio values of ≤0.001. ^{#} Brackets indicate score according to adjusted motif. | | | | |

**Table 20(D) HPV16 E6 and E7 Peptides Binding to HLA-A24**

| Orgin | First aa Position | Sequence^{*} | Binding Ratio to Standard^{‡} | Motif Prediction |
|---|---|---|---|---|
| E6 | 87 | AYSLYGTTL | 0.1200 | + |
| E6 | 72 | KALKFYSKI | 0.1100 | - (+)^{#} |
| E6 | 131 | RFHNIRGRW | 0.1000 | + |
| E7 | 49 | MLHYNIVTF | 0.0670 | - (+)^{#} |
| E6 | 49 | VYDFAFRDL | 0.0610 | + |
| E6 | 82 | EYKHYAYSL | 0.0460 | + |
| E6 | 26 | LQTTIHDII | 0.0200 | - |
| E6 | 66 | PYAVADKAL | 0.0055 | - |
| E6 | 1 | MHQKRTAMF | 0.0049 | - |
| E6 | 85 | HYAYSLYGT | 0.0037 | - |
| E6 | 44 | LLRREVYDF | 0.0023 | + |
| E6 | 38 | VYAKQQLLR | 0.0011 | - |

| | | | | |
|---|---|---|---|---|
| ^{*} Bold A's indicate residues in which cysteine was replaced by alanine. ^{‡} The average IC₅₀ value ±SB of the standard in the course of the experiments considered in this table was 22±6 nM. Listed in the table are peptides yielding ratio value of ≥0.001. All other peptides yielded ratio values of ≤0.001. ^{#} Brackets indicate score according to adjusted motif. | | | | |

**TABLE 20(E) SUMMARY OF EFFICACY OF 2, (3), 9 ANCHOR MOTIFS**

| HLA Allele | | | | | | | | |
|---|---|---|---|---|---|---|---|---|
| Percentage Binders with Motifs | A1 Predicted/Found | | A3.2 Predicted/Found | | A11.2 Predicted/Found | | A24 Predicted/Found | |
| High (≥0.1) | 2 | 2 (100%) | 7 | 7(100%) | 6 | 6 (100%) | 2 | 3 (67%) |
| Intermediate (0.1-0.01) | 3 | 6(50%) | 3 | 5 (60%) | 3 | 4 (75%) | 2 | 4 (50%) |
| Weak (0.01-0.001) | 0 | 1 (0%) | 6 | 14 (43%) | 6 | 11 (55%) | 1 | 5 (20%) |
| Negative (≤0.001) | 7 | 231(3%) | 16 | 214 (7%) | 14 | 219 (6%) | 2 | 228 (1%) |
| Totals | 12 | 240 | 32 | 240 | 26 | 240 | 7 | 240 |

**TABLE 21 (A) A3.2 9-MER OPTIMAL MOTIFS**

| 9-mers | GOOD BINDERS | INTERMEDIATE BINDERS | WEAK BINDERS | NON-BINDERS | TOTAL |
|---|---|---|---|---|---|
| IK | 3 (15%) | 4 (20%) | 7 (35%) | 6 (30%) | 20 (100%) |
| IR | 3 (15.8%) | 4 (21.1%) | 7 (36.8%) | 5 (26.3%) | 19 (100%) |
| LK | 13 (48.1%) | 6 (22.2%) | 5 (18.5%) | 3 (11.1%) | 27 (100%) |
| LR | 4 (8.5%) | 9 (19.1%) | 20 (42.6%) | 14 (29.8%) | 47 (100%) |
| SK | 0 | 0 | 0 | 0 | 0 |
| SR | 0 | 0 | 0 | 1 (100%) | 1 (100%) |
| TK | 3 (15%) | 6 (30%) | 7 (35%) | 4 (20%) | 20 (100%) |
| TR | 2 (16.7%) | 1 (6.0%) | 2 (16.7%) | 7 (58.3%) | 12 (100%) |
| VK | 9 (28.1%) | 13 (40.6%) | 9 (28.1%) | 1 (3.1%) | 32 (100%) |
| VR | 2 (7.4%) | 3 (11.1%) | 14 (51.9%) | 8 (29.6%) | 27 (100%) |
| Total | 39 (19%) | 46 (22.4%) | 71 (34.6%) | 49 (23.9%) | 205 (100%) |

**TABLE 21 (B) A11 9-MER OPTIMAL MOTIFS**

| 9-mers | GOOD BINDERS | INTERMEDIATE BINDERS | WEAK BINDERS | NON-BINDERS | TOTAL |
|---|---|---|---|---|---|
| GK | 0 | 1 (100%) | 0 | 0 | 1 (100%) |
| IK | 5 (25%) | 5 (25%) | 7 (35%) | 3 (15%) | 20 (100%) |
| LK | 6 (22.2%) | 10 (37%) | 9 (33.3%) | 2 (7.4%) | 27 (100%) |
| TK | 10 (50%) | 4 (20%) | 4 (20%) | 2 (10%) | 20 (100%) |
| VK | 12 (37.5%) | 15 (46.9%) | 4 (12.5%) | 1 (3.1%) | 32 (100%) |
| Total | 33 (33%) | 35 (35%) | 24 (24%) | 8 (8%) | 100 (100%) |

**TABLE 22(A) A3.2 10-MER OPTIMAL MOTIFS**

| 10-mers | GOOD BINDERS | INTERMEDIATE BINDERS | WEAK BINDERS | NON-BINDERS | TOTAL |
|---|---|---|---|---|---|
| AK | 1 (33.3%) | 1 (33.3%) | 1 (33.3%) | 0 | 3 (100%) |
| AR | 0 | 0 | 1 (100%) | 0 | 1 (100%) |
| FK | 0 | 0 | 0 | 0 | 0 |
| FR | 0 | 0 | 1 (25%) | 3 (75%) | 4 (100%) |
| IK | 0 | 6 (27.3%) | 10 (45.5%) | 6 (27.3%) | 22 (100%) |
| IR | 1 (7.1%) | 1 (7.1%) | 2 (14.2%) | 10 (71.4% ) | 14 (100%) |
| LK | 16 (53.3%) | 7 (23.3%) | 5 (16.7%) | 2 (6.7%) | 30 (100%) |
| LR | 4 (12.5%) | 9 (28.1%) | 11 (34.3%) | 8 (25%) | 32 (100%) |
| MK | 1 (100%) | 0 | 0 | 0 | 1 (100%) |
| MR | 1 (100%) | 0 | 0 | 0 | 1 (100%) |
| TK | 2 (11.8%) | 5 (29.4%) | 8 (47.1%) | 2 (11.8%) | 17 (100%) |
| TR | 1 (4.8%) | 1 (4.8%) | 9 (42.9%) | 10 (47.6%) | 21 (100%) |
| VK | 7 (35%) | 4 (20%) | 5 (25%) | 4 (20%) | 20 (100%) |
| VR | 0 | 6 (21.4%) | 15 (53.6%) | 7 (25%) | 28 (100%) |
| Total | 34 (17.5%) | 40 (20.6%) | 68 (35.1%) | 52 (26.8%) | 194(100%) |

**TABLE 22(B) A11 10-MER OPTIMAL MOTIFS**

| 10-mers | GOOD BINDERS | INTERMEDIATE BINDERS | WEAK BINDERS | NON-BINDERS | TOTAL |
|---|---|---|---|---|---|
| AK | 1 (33.3%) | 1 (33.3%) | 1 (33.3%) | 0 | 3 (100%) |
| CK | 0 | 0 | 1 (100%) | 0 | 1 (100%) |
| GK | 0 | 0 | 0 | 0 | 0 |
| IK | 4 (18.2t) | 5 (22.7t) | 12 (54.Str) | 1 (4.5%) | 22 (100%) |
| LK | 9 (30%) | 12 (40%) | 8 (26.7%) | 1 (3.3%) | 30 (100%) |
| MK | 1 (100%) | 0 | 0 | 0 | 1 (100%) |
| TK | 6 (35.3%) | 5 (29.4%) | 5 (29.4%) | 1 (5.9%) | 17 (100%) |
| VK | 7 (35%) | 8 (40%) | 4 (20%) | 1 (5%) | 20 (100%) |
| Total | 29 (29.8%) | 31 (33%) | 31 (33%) | 4 (4.3%) | 94 (100%) |

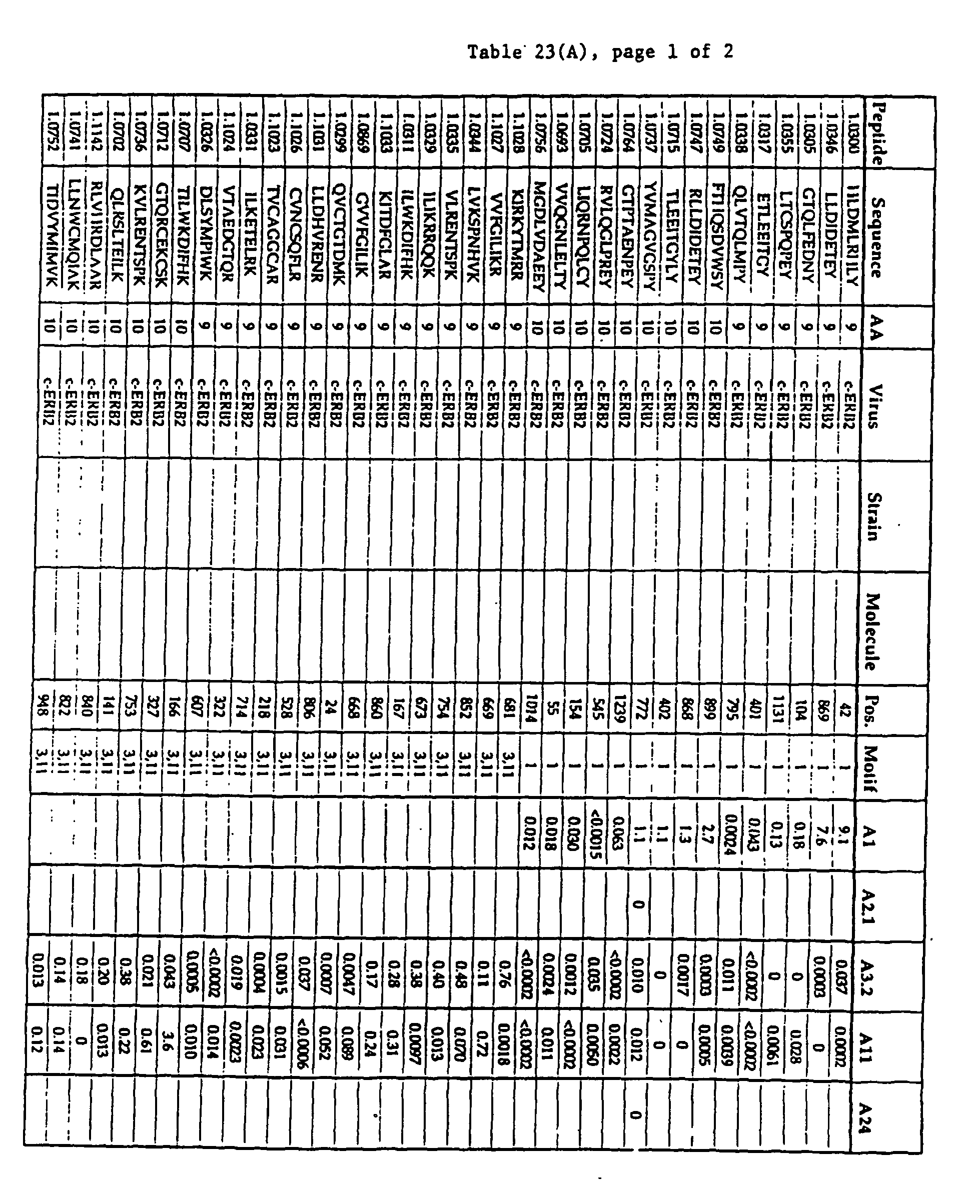

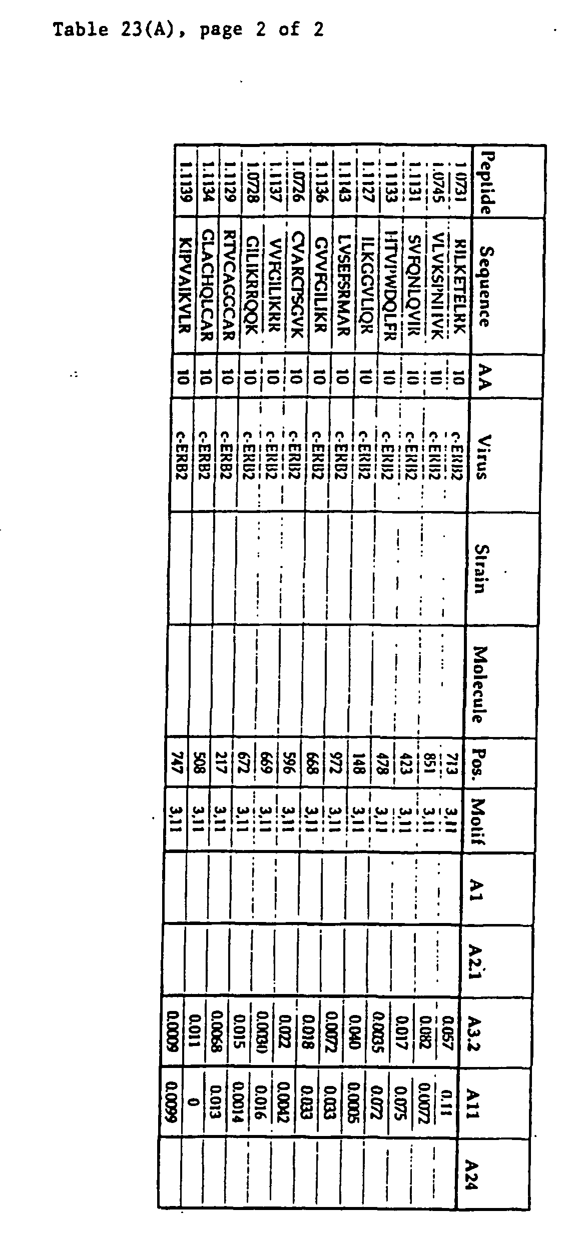

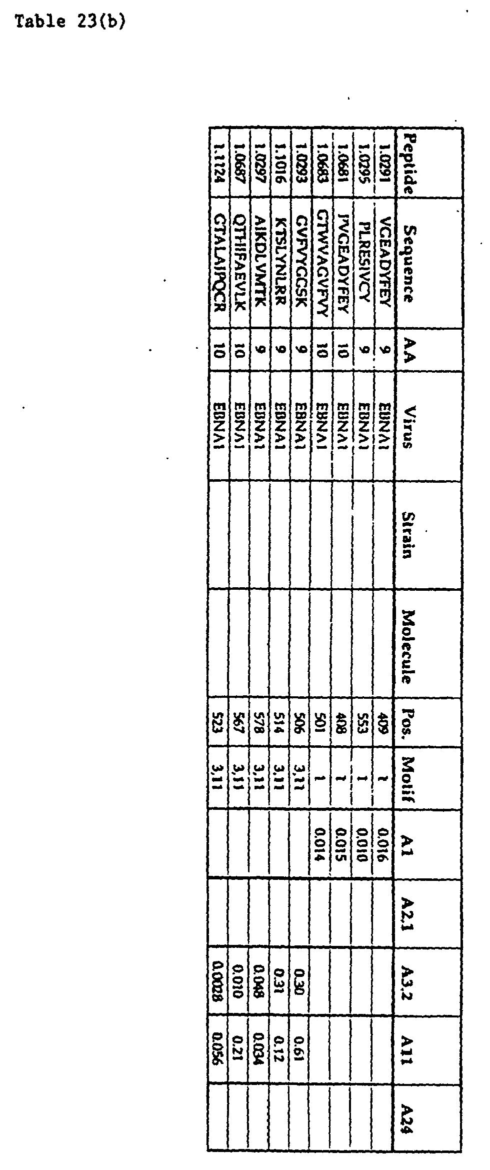

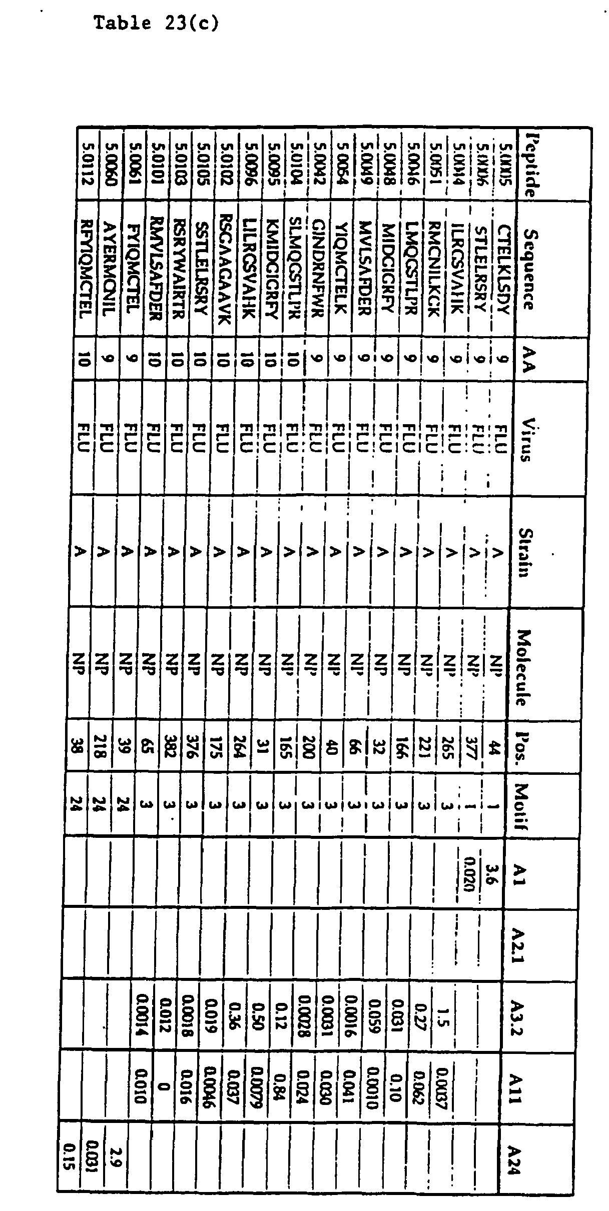

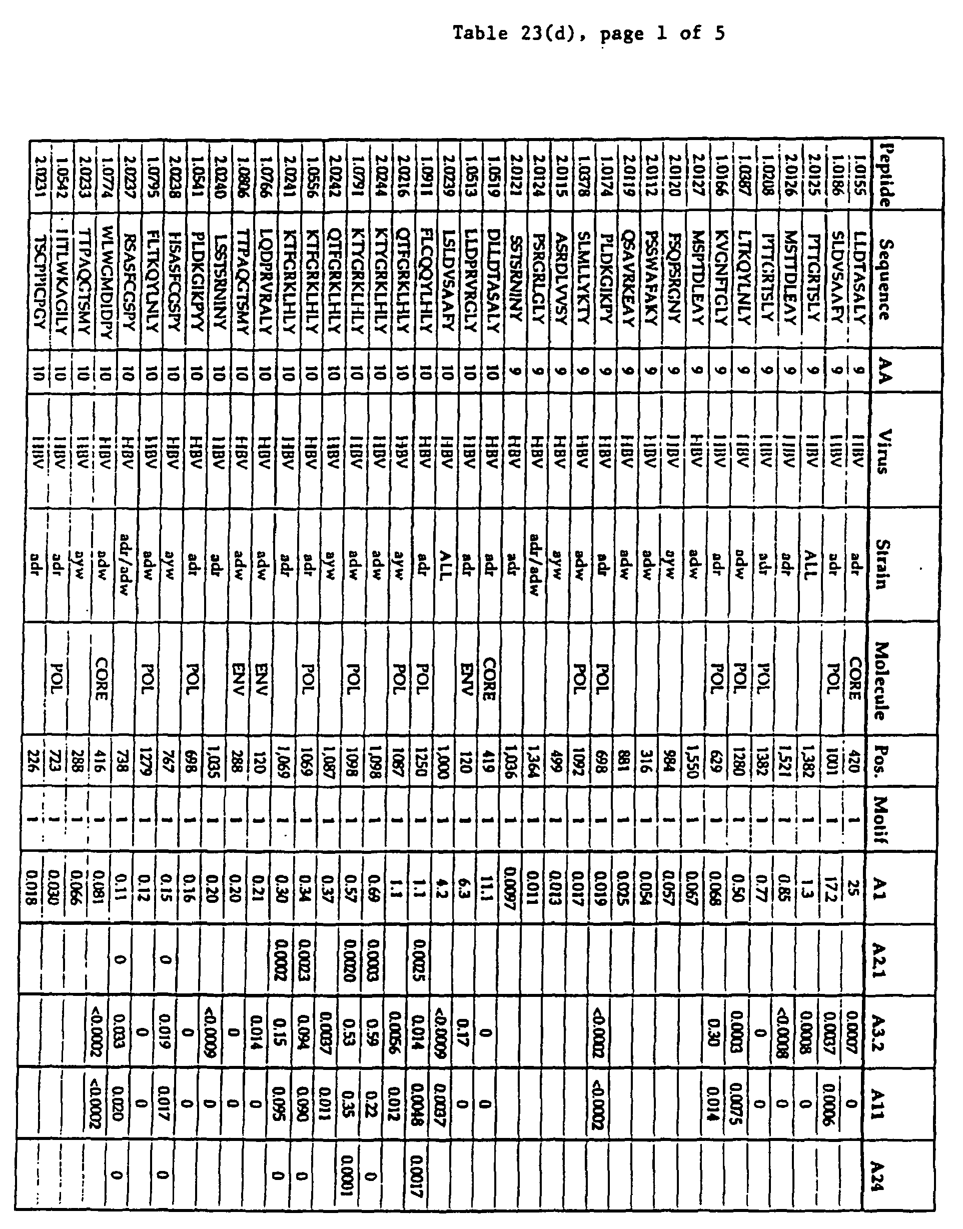

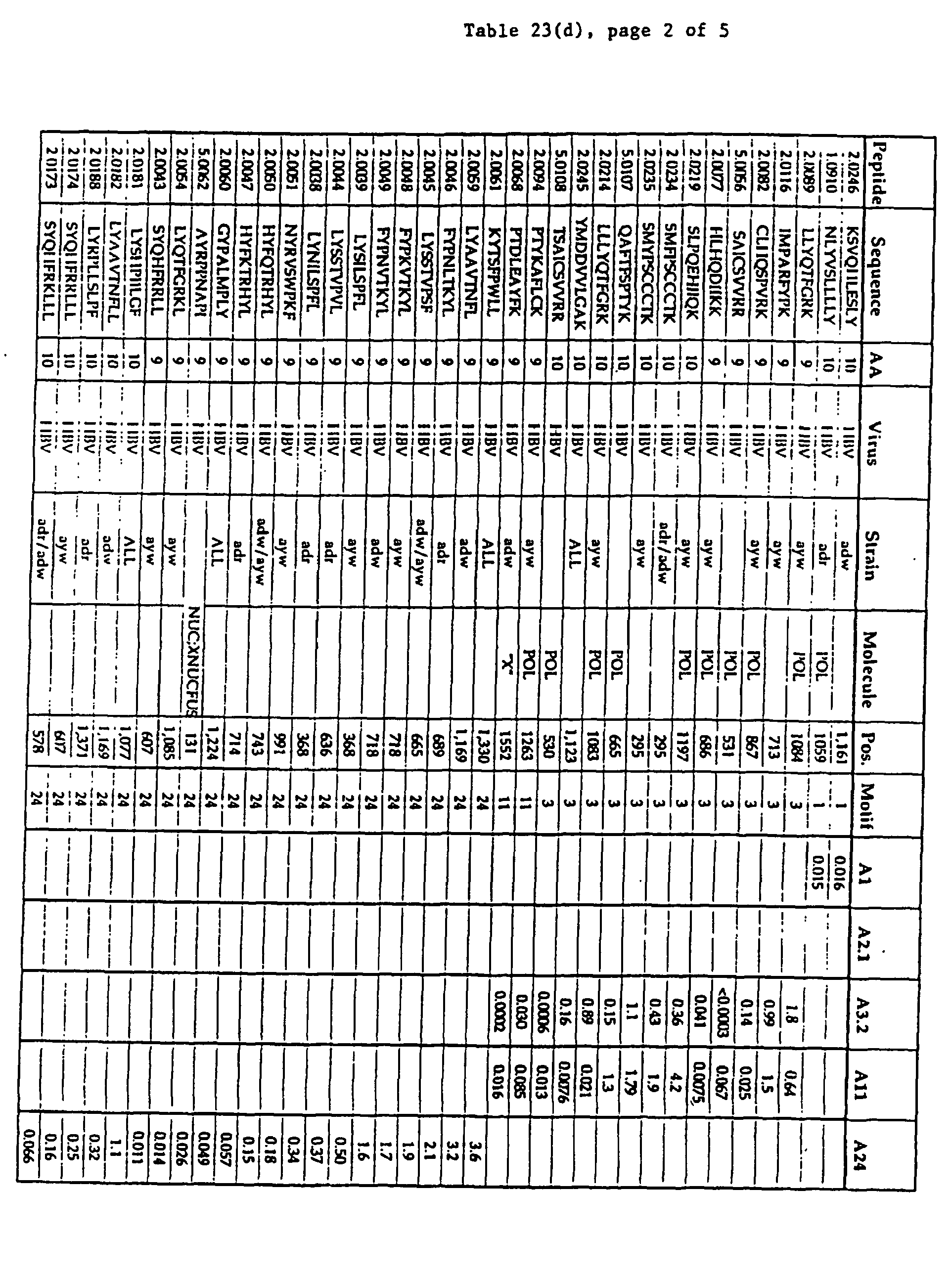

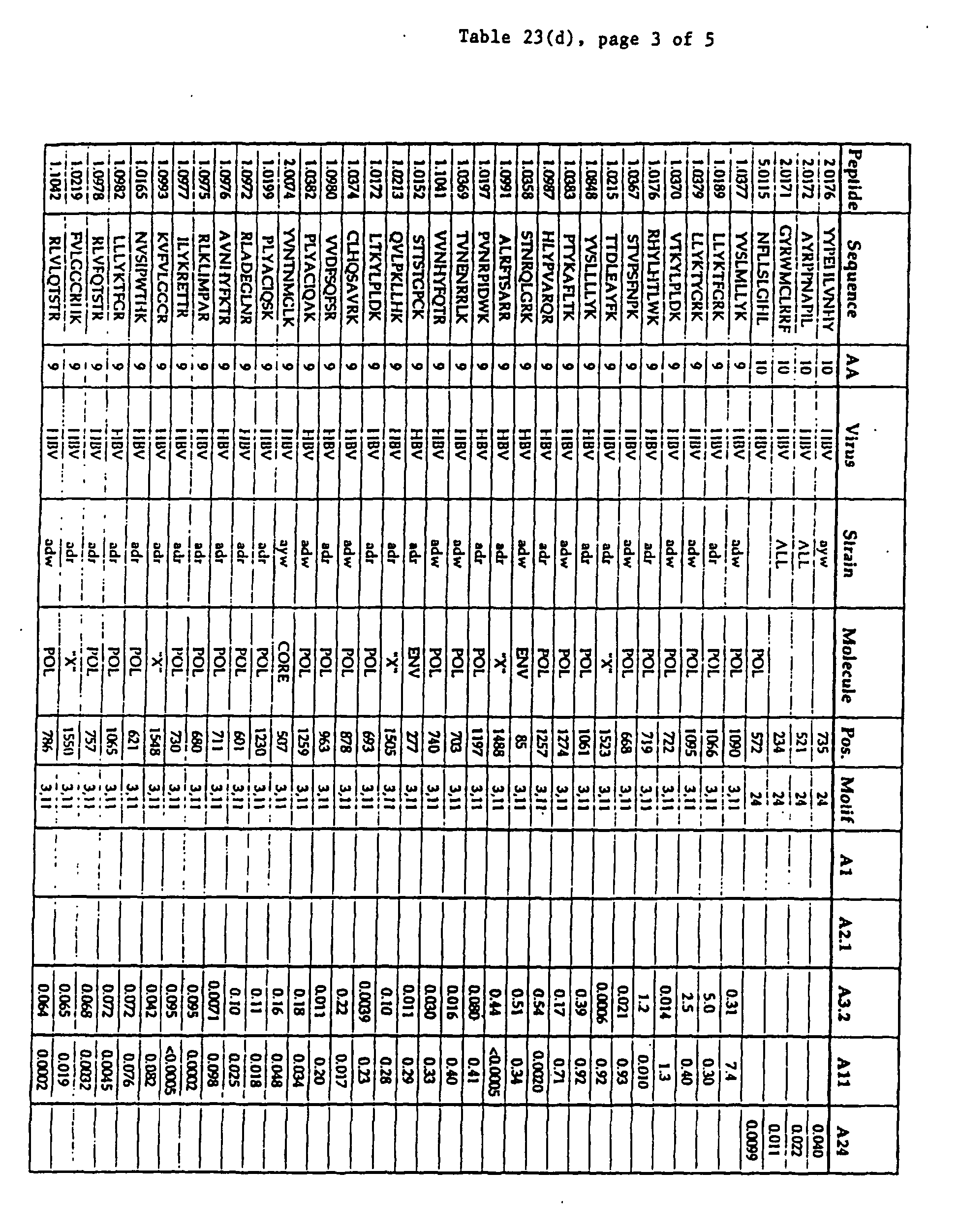

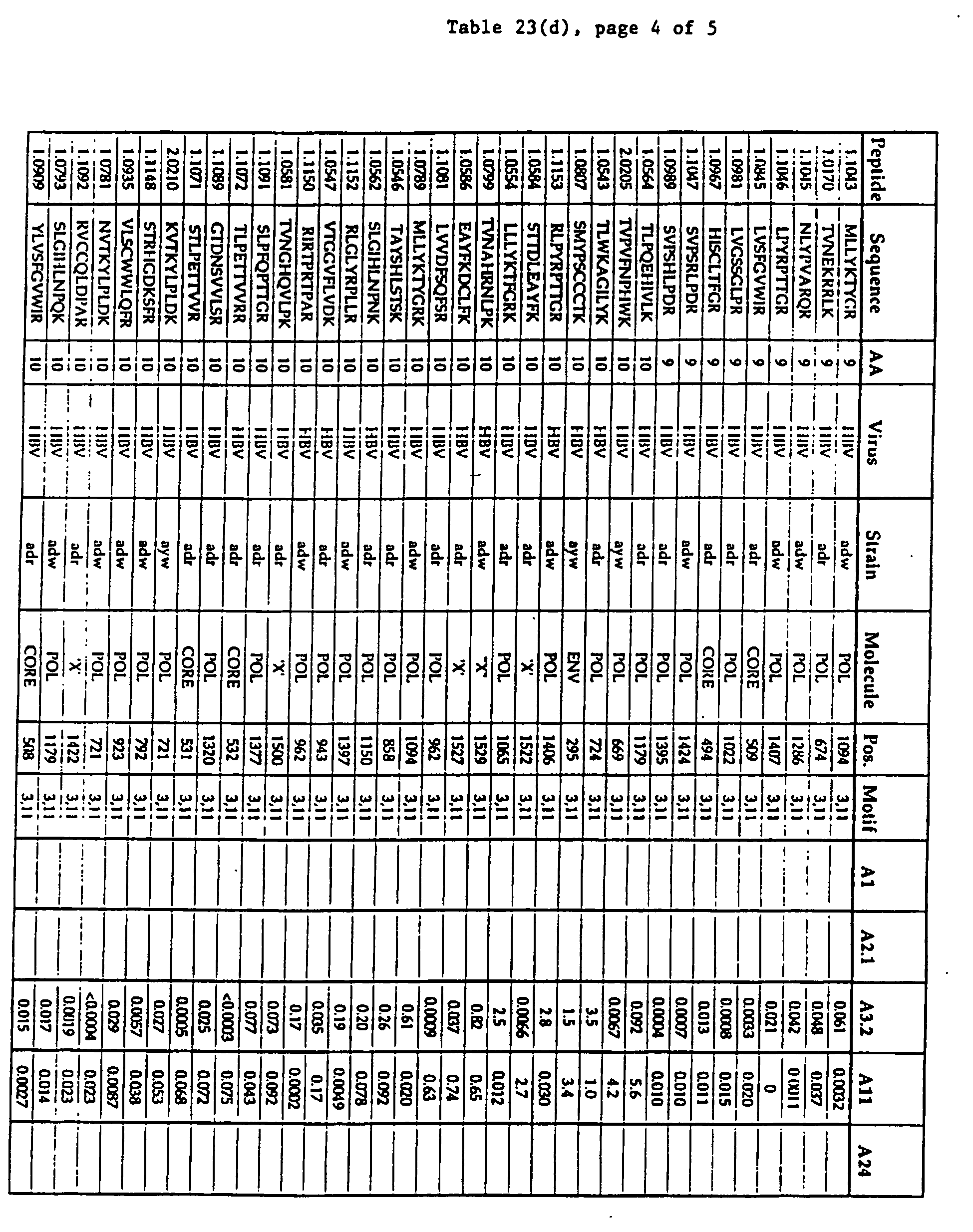

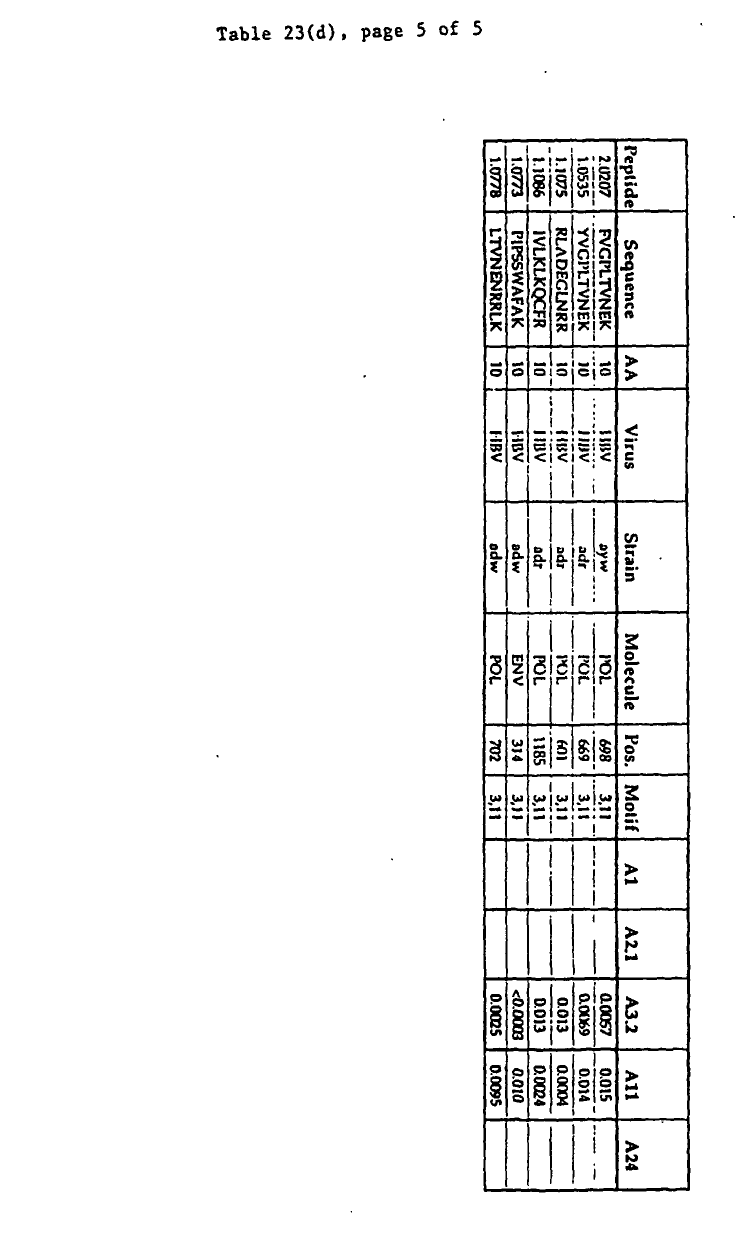

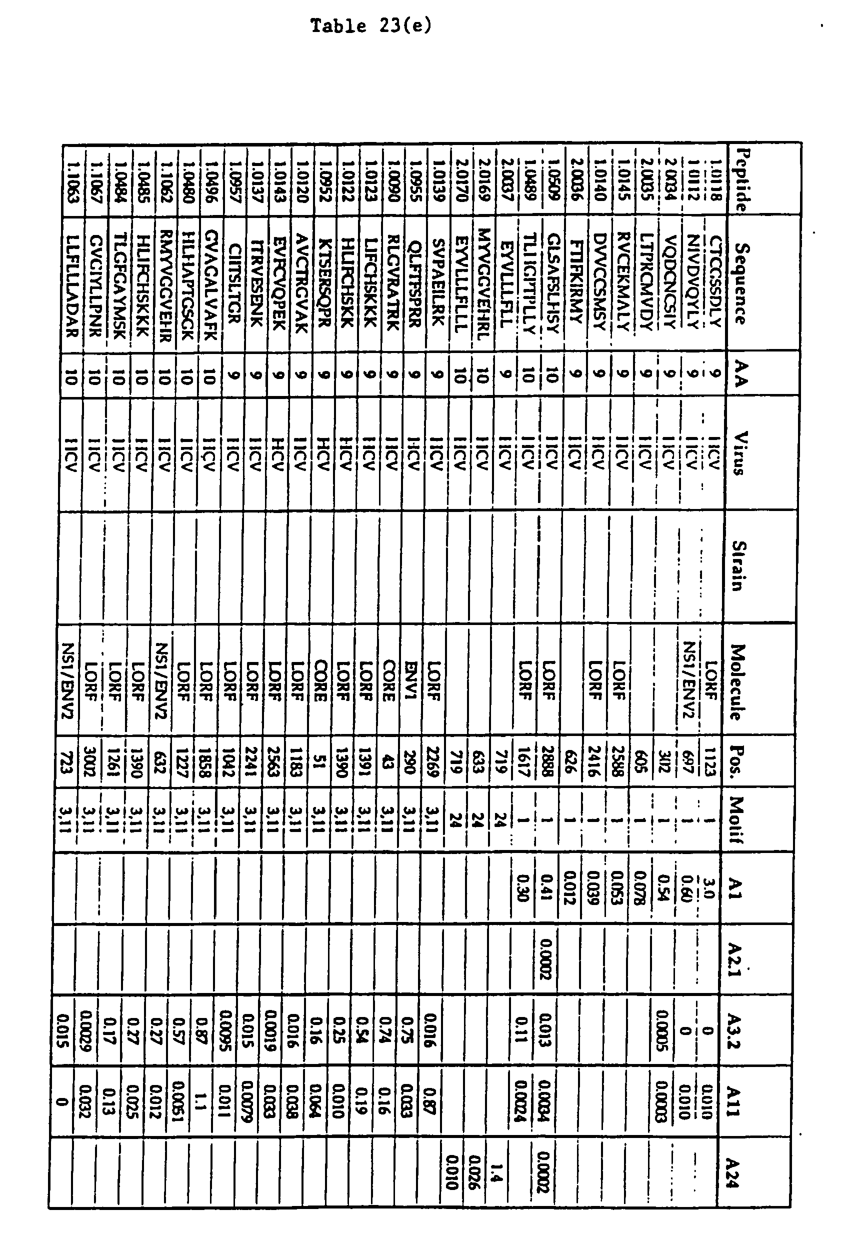

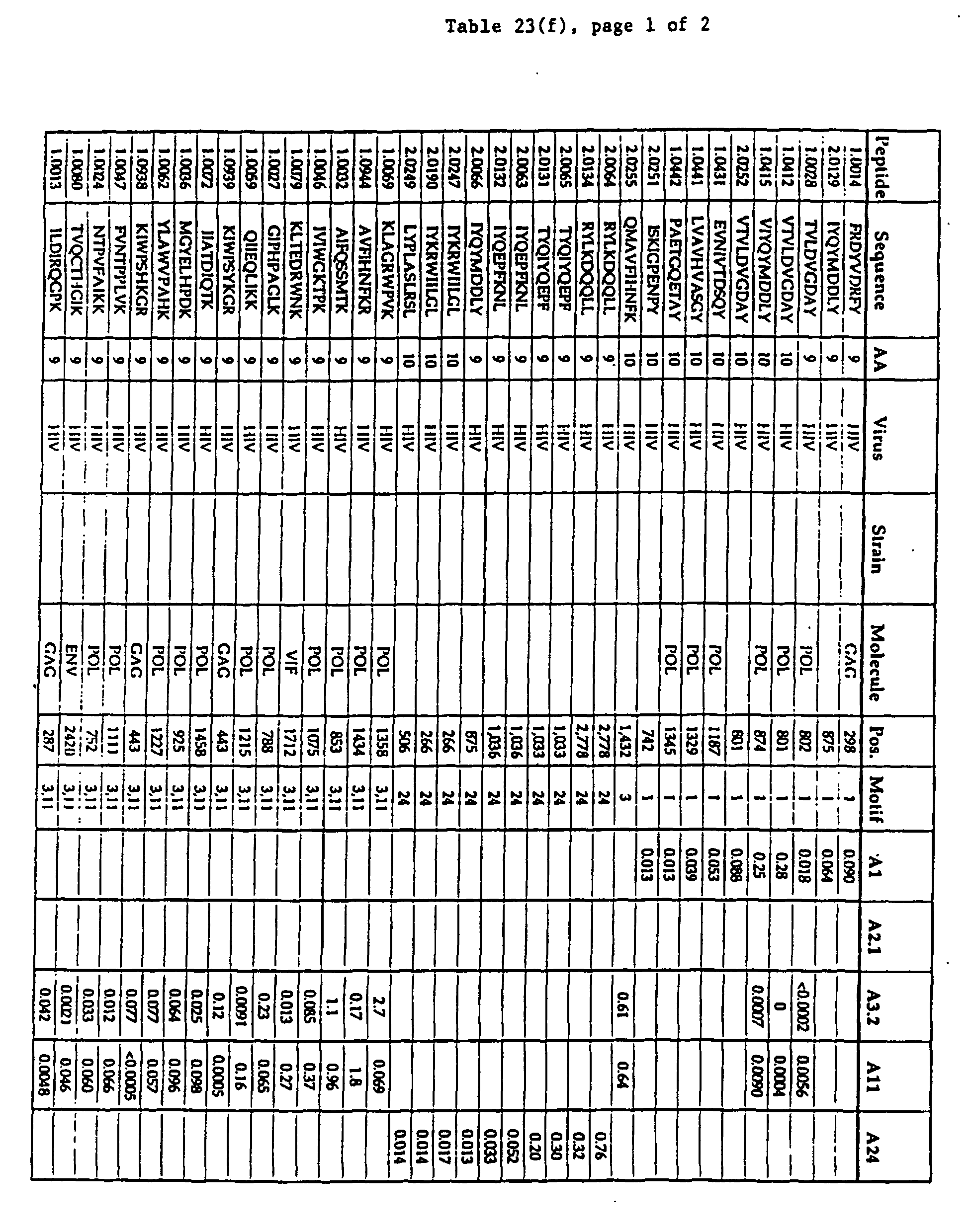

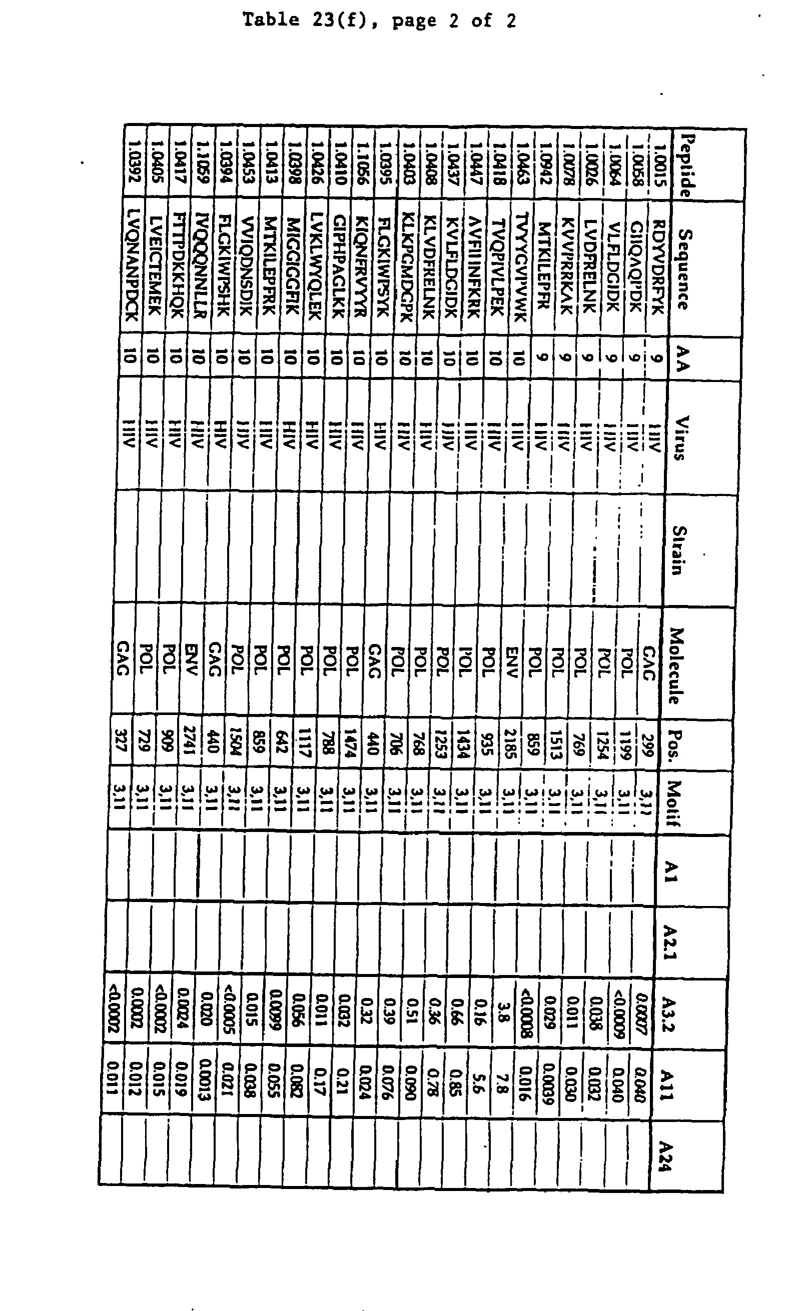

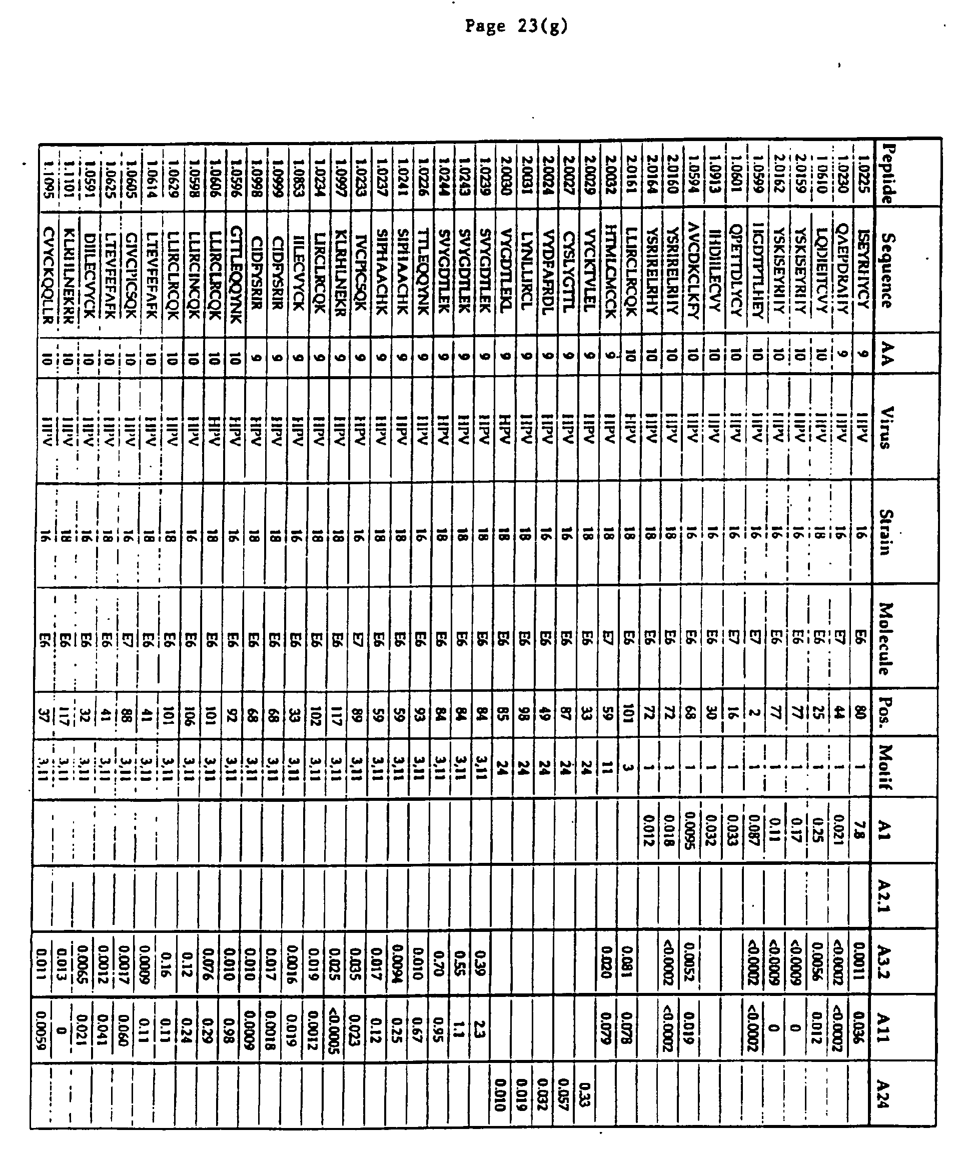

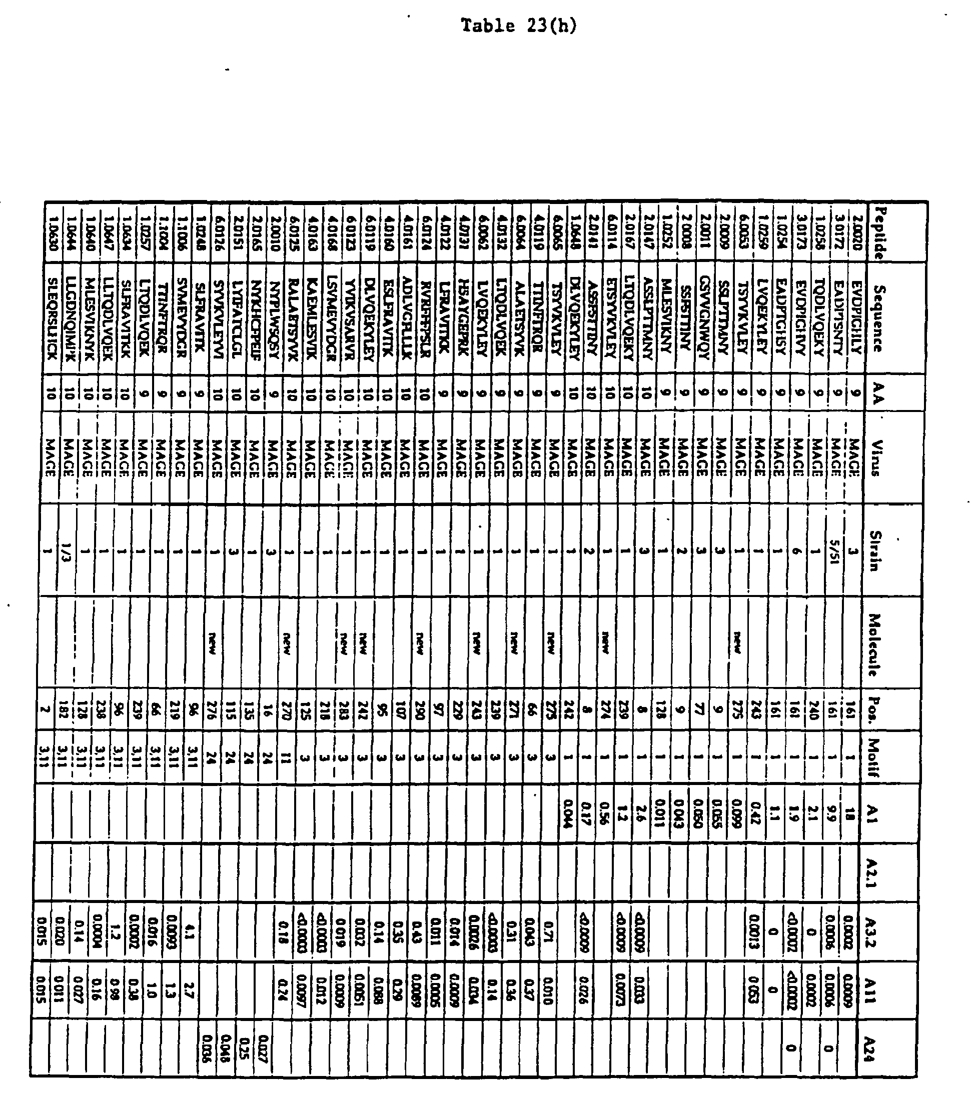

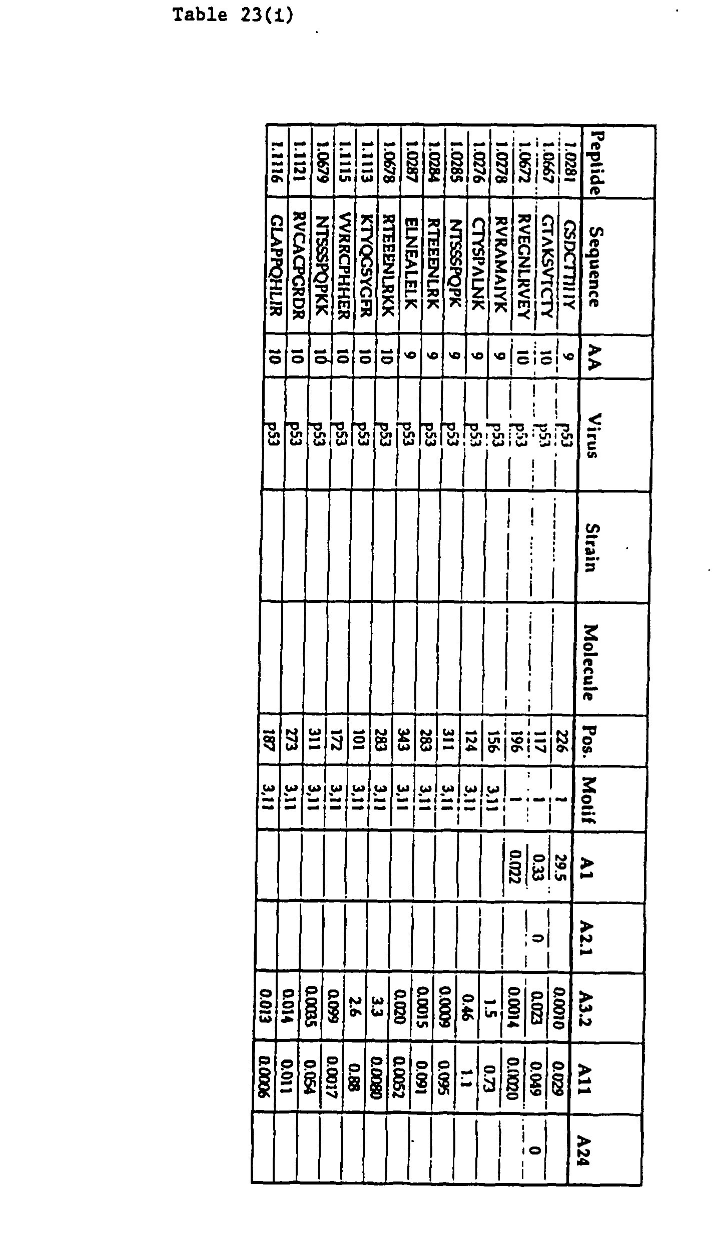

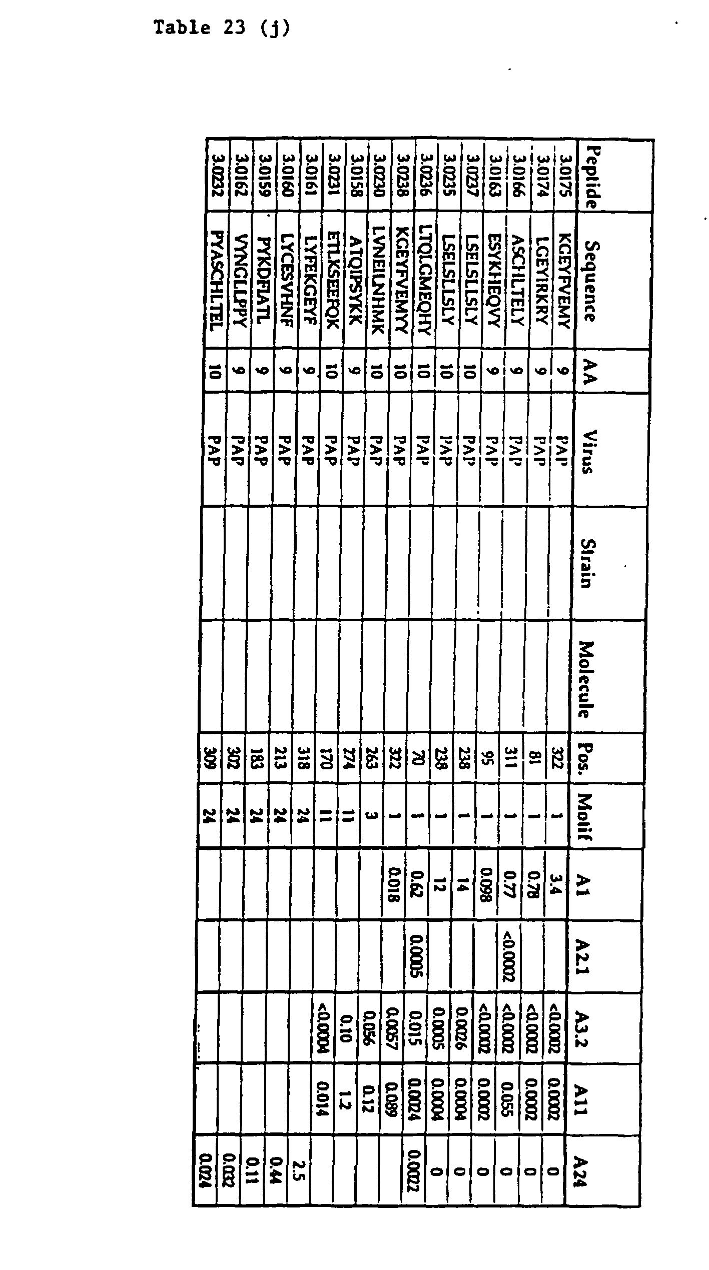

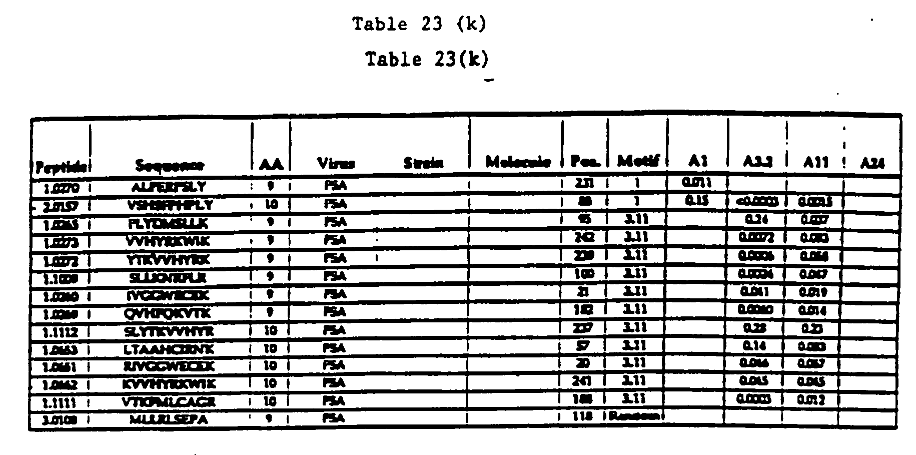

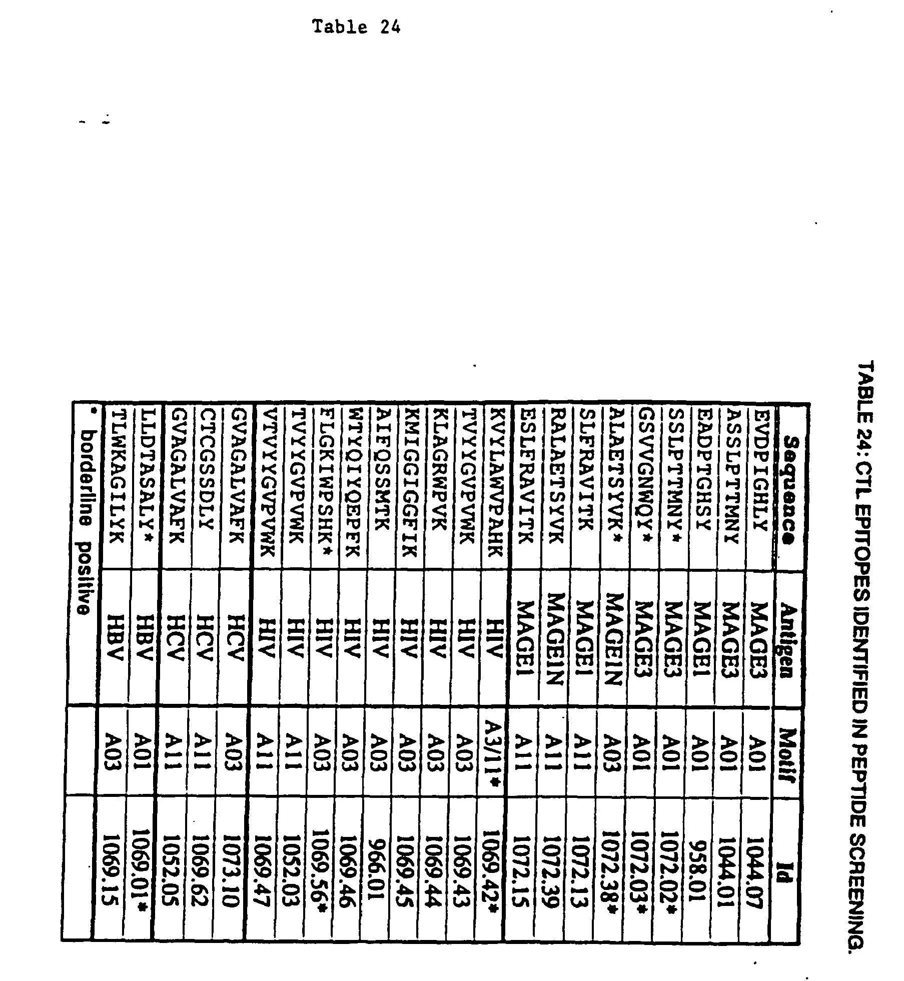

**Table 25 Peptides Synthesized by Cytel For Loading Onto Acid Stripped Autologous PBMCs and PHA Blasts**

| Peptide ID # | Antigen | Sequence |
|---|---|---|
| 777.03 | HBs | FLLTRILTI |
| 924.07 | HBc | FLPSDFFPSV |
| 927.32 | HBp | GLYSSTVPV |
| 938.01 | MAGE 1 | EADPTGHSY |
| 939.03 | PSA | VLVHPQWVL |
| 941.01 | HBc | FLPSDYFPSV |
| 1044.04 | PAP | ILLWDPIPV |
| 1044.05 | PSA | KLQCVDLVHI |
| 1044.06 | PSA | MLLRLSEPAEL |

**Table 25 b**

| Cell Population | ¹²⁵I-Labeled | CPMS | |
|---|---|---|---|
| | Peptide +/- Cold | +/- std. dev. | |
| | Peptide | | |
| JY acid stripped | - cold peptide | 3553 ± 157 | n = 3 |
| JY acid stripped | + cold peptide | 13 | n = 1 |
| JY control | -cold peptide | 370 ± 37 | n = 3 |
| JY control | + cold peptide | 50 | n = 1 |

## Claims

1. A composition comprising an immunogenic peptide having a HLA-A3.2 binding motif, which immunogenic peptide has between about 9 and about 10 residues and the following residues, from the N-terminus to the C-terminus:
a first conserved residue selected from the group consisting of L, M, I, V, S, A, T, F, C, G, D and E;
and a second conserved residue of K, R, Y, H and F;
wherein the first and second conserved residues are separated by 6 to 7 residues.

2. The composition of claim 1, wherein the first conserved residue is at the second position from the N-terminus.

3. A composition comprising an immunogenic peptide having a HLA-A1 binding motif, which immunogenic peptide has between about 9 and about 10 residues and the following residues, from the N-terminus to the C-terminus:
a first conserved residue of T, S and M; and
a second conserved residue of D, E, A, S and T;
a third conserved residue of Y;
wherein the first and second conserved residues are adjacent and the second and third conserved residues are separated by 5 or 6 residues.

4. The composition of claim 3, wherein the first conserved residue is at the second position from the N-terminus.

5. A composition comprising an immunogenic peptide having a HLA-A1 binding motif, which immunogenic peptide has between about 9 and about 10 residues and the following residues, from the N-terminus to the C-terminus:
a first conserved residue of T, S and M; and
a second conserved residue of Y;
wherein the first and second conserved residues are separated by 6 to 7 residues.

6. The composition of claim 5, wherein the first conserved residue is at the second position from the N-terminus and the second conserved residue is at the ninth or tenth position from the N-terminus.

7. A composition comprising an immunogenic peptide having an HLA-A1 binding motif, which immunogenic peptide has between 9 and about 10 residues and the following residues, from the N-terminus to the C-terminus.
a first conserved residue of D, E, A, S and T; and
a second conserved residue of Y;
wherein the first and second conserved residues are separated by 5 to 6 residues.

8. The composition of claim 5, wherein the first conserved residue is at the third position from the N-terminus and the second conserved residue is at the ninth or tenth position from the N-terminus.

9. A composition comprising an immunogenic peptide having a HLA-A11 binding motif, which peptide has between about 9 and about 10 residues and the following residues, from the Nterminus to the C-terminus:
a first conserved residue of L, M, I, V, A, S, T, G, N, Q, C, F, D, E; and
a second conserved residue of K, R, H;
wherein the first and second conserved residues are separated by 6 to 7 residues.

10. The composition of claim 9, wherein the first conserved residue is at the second position from the N-terminus.

11. A composition comprising an immunogenic peptide having a HLA-A24.1 binding motif, which immunogenic peptide has between about 9 and about 10 residues and the following residues, from the N-terminus to the C-terminus:
a first conserved residue of Y, F, W; and
a second conserved residue of F, I, L, W, M;
wherein the first and second conserved residues are separated by 6 to 7 residues.

12. The composition of claim 11, wherein the first conserved residue is at the second position from the N-terminus.

13. A composition comprising an immunogenic peptide having an HLA-A3.2 binding motif, which immunogenic peptide has 9 or 10 residues:
a first conserved residue at the second position selected from the group consisting of A, I, L, M, T, and V; and a second conserved residue at the C terminal postion selected from the group consisting of K and R.
wherein the first and second conserved residues are separated by 6 to 7 residues.

14. A composition comprising an immunogenic peptide having an HLA-A11 binding motif, which immunogenic peptide has 9 or 10 residues and the following residues, from the N-terminus to the Cterminus:
a first conserved residue at the second position from the N terminus selected from the group consisting of A, I, L, M, T and V; and
a second conserved residue at the C terminal position selected from the group consisting of K;
wherein the first and second conserved residues are separated by 6 to 7 residues.

15. A pharmaceutical composition comprising a pharmaceutically acceptable carrier and an immunogenic peptide having a HLA-A3.2 binding motif, which immunogenic peptide has between about 9 and about 10 residues and the following residues, from the N-terminus to the C-terminus:
a first conserved residue selected from the group consisting of L, M, I, V, S, A, T, F, C, G, D and E; and
a second conserved residue of K, R and Y;
wherein the first and second conserved residues are separated by 6 to 7 residues.

16. A pharmaceutical composition comprising a pharmaceutically acceptable carrier and an immunogenic peptide having a HLA-A1 binding motif, which immunogenic peptide has between about 9 and about 10 residues and the following residues, from the N-terminus to the C-terminus:
a first conserved residue of T, S and M; and
a second conserved residue of D, E, A, S and T;
a third conserved residue of Y;
wherein the first and second conserved residues are separated by 1 residue and the second and third conserved residues are separated by 5 or 6 residues.

17. A pharmaceutical composition comprising a pharmaceutically acceptable carrier and an immunogenic peptide having a HLA-A1 binding motif, which immunogenic peptide has between about 9 and about 10 residues and the following residues, from the N-terminus to the C-terminus:
a first conserved residue of T, S or M; and
a second conserved residue of Tyr;
wherein the first and second conserved residues are separated by 6 to 7 residues.

18. A pharmaceutical composition comprising a pharmaceutically acceptable carrier and an immunogenic peptide having a HLA-A1 binding motif, which peptide has between about 9 and about 10 residues and the following residues, from the N-terminus to the C-terminus:
a first conserved residue of D, E, S, T; and
a second conserved residue of Y;
wherein the first and second conserved residues are separated by 5 to 6 residues.

19. A pharmaceutical composition comprising a pharmaceutically acceptable carrier and an immunogenic peptide having a HLA-A24.1 binding motif, which peptide has
a first conserved residue of Y, F, W; and
a second conserved residue of F, I, L, W, or m;
wherein the first and second conserved residues are separated by 6 to 7 residues.

20. A method of identifying an immunogenic peptide comprising the following steps:
determining a binding motif for an MHC molecule encoded by a preselected MHC Class I allele;
screening an amino acid sequence of an antigenic protein for the presence of the binding motif;
selecting a sequence in the antigenic protein having the binding motif;
preparing a test peptide of about 8 and about 11 residues comprising the selected subsequences;
determining the ability of the test peptide to bind to the preselected MHC allele and induce a CTL response, thereby identifying immunogenic peptides.
